(19) Europäisches Patentamt
European Patent Office
Office européen des brevets

(11) EP 3 795 643 A1

(12) EUROPEAN PATENT APPLICATION

(43) Date of publication:
24.03.2021 Bulletin 2021/12

(21) Application number: 19306175.1

(22) Date of filing: 23.09.2019

(51) Int Cl.:
C09B 57/00 (2006.01)
G01N 33/58 (2006.01)
A61K 49/00 (2006.01)
G01N 21/64 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Centre National de la Recherche Scientifique 75016 Paris (FR)
- Université Claude Bernard Lyon 1 69100 Villeurbanne (FR)
- Institut National de la Santé Et de la Recherche Médicale 75654 Paris Cedex 13 (FR)
- Institut National des Sciences Appliquées de Lyon 69100 Villeurbanne (FR)
- CPE Lyon Formation Continue et Recherche 69100 Villeurbanne (FR)
- Centre Léon-Bérard 69373 Lyon Cedex 08 (FR)
- Hospices Civils De Lyon 69229 Lyon Cedex 02 (FR)

(72) Inventors:
- GRANJON, Thierry 38110 La Tour du Pin (FR)
- MANITI, Ofelia 38110 La Tour du Pin (FR)
- CHENIOUR, Mouhedine 69580 Sathonay-Camp (FR)
- MARCILLAT, Olivier 69006 Lyon (FR)
- GOEKJIAN, Peter 69100 Villeurbanne (FR)
- VIGNERON, Arnaud 38300 Eclose-Badinières (FR)
- GUEYRARD, David 01480 Villeneuve (FR)
- IBANEZ, Sébastien 69960 Corbas (FR)

(74) Representative: Regimbeau
20, rue de Chazelles
75847 Paris Cedex 17 (FR)

# (54) FLUORESCENT PROBE AND APPLICATIONS THEREOF

(57) The invention relates a fluorescent compound of formula I:

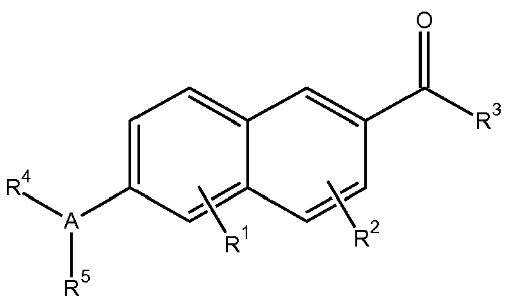

(I)

wherein A is selected from P, P=O and N;
$R^1$ is a residue comprising an oxygen atom
$R^2$ is a residue comprising an oxygen atom, or a halogen,
$R^3$, $R^4$ and $R^5$ are alkyls, possibly substituted
or a salt or a solvate thereof.
Those compounds are useful as organic fluorescent probes sensitive to the viscosity of their environment for the study of cell membrane fluidity.

EP 3 795 643 A1

**Description**

Membranes, as selective barriers between the inner and the outer compartments, regulate a plethora of phenomena related to signalling, molecule entry into cells, metabolite exchange or membrane trafficking. Therefore, investigations in the field of membranes are exponentially increasing today. The complexity of biological membranes, as major components of the living cell, and their numerous interactions with intra- and extracellular networks, make their direct investigation difficult.

Membrane fluidity modulates the binding of proteins to the membrane, transmembrane protein function, formation of multi-protein or protein-lipid complexes and more generally, signaling or metabolic pathways. Therefore, quantifying membrane fluidity is a challenge in the field of biological membranes. To respond to this challenge, there is a need for tools to accurately measure physical membrane state in living cells or tissue. In this respect, organic fluorescent probes sensitive to the viscosity of their environment are widely used for the study of both membrane fluidity and organization. Among them, Laurdan, which belongs to the family of 2-(dimethylamino)-6-acylnaphthalene, is one of the most popular.

During the transition from gel to crystal-liquid phase (with membranes getting more disordered) Laurdan's emission spectrum is shifted from 440 nm to 490 nm. This shift was shown to be primarily linked to the mobility of water molecules in the vicinity of the excited naphthalene moiety (Parasassi et al., Biophysical Society Volume 60 July 1991, 179-189) which is reported to be located at the hydrophilic-hydrophobic interface of the bilayer. Thus the 440 nm/490 nm fluorescence emission intensity ratio mirrors the polarity of Laurdan environment which in turn gives insight into the packing of lipid molecules and membrane fluidity state.

Studies of Laurdan in biomimetic assemblies are numerous both by fluorescence spectroscopy and fluorescence microscopy, but living cell membranes studies with Laurdan are more scarce. Laurdan is a rather hydrophobic molecule, with low solubility in aqueous buffers. It was shown to accumulate in apolar compartments such as lipid droplets. Plasma membrane labelling is therefore rather ineffective. Moreover, the distribution of Laurdan between ordered and disordered regions of the membranes is not homogeneous (Kim et al., ChemBioChem 2007, 8, 553-559). Therefore, a series of Laurdan-derived fluorophores were synthesized in the field (Kim et al., ChemBioChem 2007, 8, 553-559; Cheniour et al., RSCAdv., 2016, 6, 5547-5557). C-Laurdan, 6-dodecanoyl-2-[N-methyl-N-(carboxymethyl)-amino] naphthalene, for instance, has a supplementary carboxyl group at the aminonaphtalene moiety, which induces favorable hydrophilic interactions with water molecules near the lipid head-group (Kim et al., ChemBioChem 2007, 8, 553-559). Yet, since C-Laurdan possesses a carboxylic group which dissociates with a pKa 6.6, its fluorescence properties will be affected not only by membrane packing/fluidity but also by the presence of charged phospholipids and by pH variations in various cell compartments.

Moreover, UV-excitable probes such as Laurdan and derivatives are very sensitive to changes in membrane fluidity, but they are all subject to photo-bleaching. Therefore, microscopy visualization of these fluorophores requires biphoton microscopy settings to limit excitation energy. Other derivatives with spectral properties shifted towards the red edge of the spectrum were designed, such as Di-4-ANEPPDHQ, which has emission maxima of 560 nm and 610 nm in gel and liquid crystalline phase membranes, respectively (Dinic et al., Biochimica et Biophysica Acta 1808 (2011) 298-306; Sezgin et al., ChemPhysChem 2015, 16, 1387-1394). Molecular mechanisms underlining spectral changes of Di-4-ANEPPDHQ were shown to be different from those of Laurdan, with Laurdan being more sensitive to lipid packing and Di-4-ANEPPDHQ more sensitive to cholesterol content in membranes. Yet, cholesterol sensitivity of Di-4-ANEPPDHQ was proposed to be related to cholesterol-fluorophore interactions or to an electrochromic effect, since this probe is sensitive to membrane potential, rather than to a direct measure of lipid packing in membranes (Amaro et al., J. Phys. D: Appl. Phys. 50 (2017) 134004 (9pp)).

Therefore, up-to-date, no polarity-sensitive derivative was able to replace Laurdan, despite its imperfections, for membrane fluidity studies.

The aim of the invention is to obviate these drawbacks.

One of the purposes of the invention is to provide a fluorescent probe sensitive to membrane fluidity.

Another purpose of the invention is to provide a method for labelling a lipid membrane.

An additional purpose of the invention is to provide a method for determining the degree of fluidity of a lipid membrane

Another purpose of the invention is to provide a method for diagnosing cancer in an individual.

An ultimate purpose of the invention is to provide a method for tumour follow-up in a patient.

Thus, the invention relates to a fluorescent compound of formula I:

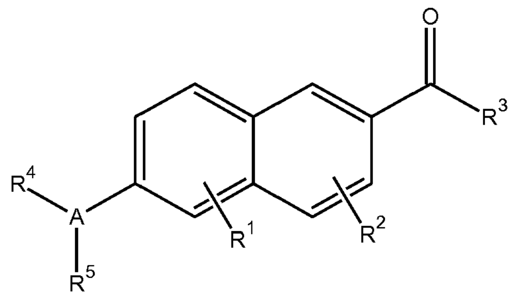

(I)

wherein A is selected from P, P=O and N;

$R^1$ is selected from the group comprising hydrogen atom, $NO_2$, $NR^6_2$, $CH_3$, $OR^6$, $SO_3H$; Cl, F, $CO_2H$, $CONR^6_2$, $O_2CR$ $^6$, $NHCOR^6$; $NHCO_2R^6$ and $NHCONR^6_2$;
$R^2$ is selected from the group comprising hydrogen atom, $NO_2$, $NH_2$, $CH_3$, OH, $SO_3H$ and halogen;
$R^3$ is a C3-C25 alkyl, linear or branched, saturated or not, cyclic or not;
$R^4$ and $R^5$ are selected from the group consisting of:

- $R^4$ and $R^5$ are together and independently from each other a C2-C6 alkyl, linear or branched, saturated or not, cyclic or not, substituted by at least a hydroxyl group,
- $R^4$ is a C3-C6 alkyl linear or branched, saturated or not, cyclic or not, substituted by at least two hydroxyl groups and $R^5$ is a C1-C6 alkyl linear or branched, saturated or not, cyclic or not, not substituted, and
- $R^5$ is a C3-C6 alkyl linear or branched, saturated or not, cyclic or not, substituted by at least two hydroxyl groups and $R^4$ is a C1-C6 alkyl linear or branched, saturated or not, cyclic or not, not substituted; and

$R^6$ is selected in the group comprising hydrogen atom and a C1-C6 alkyl, linear or branched, saturated or not, cyclic or not,
or a salt or a solvate thereof.

The inventors discovered that the compound of formula I unexpectedly labels the lipid membranes with a high sensitivity to their degree of fluidity. Moreover, unlike C-Laurdan and Laurdan probes, the compound of formula I shows a high fluorescence intensity when inserted into membranes whose lipids are in the liquid-crystal phase. Consequently, the compound of formula I allows for the first time to finely compare the degree of fluidity between two lipid membranes. Moreover, the water solubility of the compound of formula I is very good. As a result, the compound of formula I does not tend to precipitate in the cells and its incorporation efficiency in the cells is very good with a homogeneous distribution in the cell membranes. The results are therefore reproducible and allow its use for cell labelling for application such as diagnosis cancer and evaluating the responsiveness of a patient to a therapeutic treatment.

The "C3-C25 alkyl, linear or branched, saturated or not, cyclic or not" of $R^3$ includes a C3-C25 alkyl linear saturated, a C3-C25 alkyl linear unsaturated, a C3-C25 alkyl linear branched saturated, a C3-C25 alkyl linear branched unsaturated, a C3-C25 cycloalkyl saturated, a C3-C25 cycloalkyl unsaturated, a C3-C25 cycloalkyl branched saturated and a C3-C25 cycloalkyl branched unsaturated. For example, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl, dodecyl, 1,1,3,3,5,5-hexamethylhexyl, tridecyl, tetradecyl, 2,6,10-trimethylundecyl, pentadecyl, 3,7,11-trimethyldodecyl, hexadecyl, heptadecyl, octadecyl, nonadecyl, 2,6,10,14-tetramethylpentadecyl, eicosyl, 3,7,11,15-tetramethylhexadecyl, heneicosyl, docosyl, tricosyl, tetracosyl, 2,6,10,14,18-pentamethylnonadecyl, pentacosyl, 3,7,11,15,19-pentamethyleicosyl, including all R or S stereoisomers, as mixtures or separately,
cyclopropyl, cyclobutyl, cyclopentyl, methylcyclopentyl, dimethylcyclopentyl, cyclohexyl, methylcyclohexyl, dimethylcyclohexyl, trimethylcyclohexyl, tert-butylcyclohexyl, cycloheptyl or cyclooctyl, cyclononyl, cyclodecyl, cycloundecyl, cyclododecyl, cyclotridecyl, cyclotetradecyl, cyclopentadecyl, cyclohexadecyl, cyclooctadecyl, cyclononadecyl, and cycloicosyl,
-indanyl, -norbornyl, -1,2,3,4-tetrahydronaphthalenyl, -5,6,7,8-tetrahydronaphthalenyl, - perhydronaphthalenyl, adamantyl, cyclopentenyl, -cyclopentadienyl, -cyclohexenyl, - cyclohexadienyl, - cycloheptenyl, -cycloheptadienyl, -cycloheptatrienyl, -cyclooctenyl, - cyclooctadienyl, -cyclooctatrienyl, -cyclooctatetraenyl, -cyclononenyl, -cyclononadienyl, - cyclodecenyl, -cyclodecadienyl, -norbornenyl, heptalenyl, azulenyl, -norbornyl, -1,2,3,4-tetrahydronaphthalenyl, -5,6,7,8-tetrahydronaphthalenyl, -perhydronaphthalenyl, bicyclo[3.2.0]hept-2-enyl, -indenyl, naphthyl, -pentalenyl, -naphthalenyl, -azulenyl, - heptalenyl, -1,2,7,8-tetrahydronaphthalenyl, -quinolinyl, -isoquinolinyl, -chromonyl, - coumarinyl, -indolyl, -indolizinyl, -benzo[b]furanyl, -benzo[b]thiophenyl, - benzo[d][1,3]dioxolyl, -indazolyl, -purinyl, -4H-quinolizinyl, -isoquinolyl, - quinolyl, - phthalazinyl, -naphthyridinyl, -carbazolyl, -β-carbolinyl, -indolinyl, isoindolinyl, -1,2,3,4-tetrahydroquinolinyl, -1,2,3,4-tetrahydroisoquinolinyl and pyrrolopyrrolyl, including all E or Z and R or S stereoisomers, as mixtures or separately

methylene, ethylene, trimethylene, propylene, tetramethylene, pentamethylene, hexamethylene, 2-butenyl, 3-butenyl, 2-methyl-2-propenyl, 1-methyl-2-propenyl and 3-methyl-2-butenyl, n-pentenyl, isopentenyl, 1-methylpentenyl, 1,3-dimethylbutenyl, n-hexenyl, 1- methylhexenyl, 3-methylhexenyl, 5-methylhexenyl, n-heptenyl, isoheptenyl, 1,2,3-trimethylbutenyl, 1-methylheptenyl, 3-methylheptenyl, n-octenyl, 2-ethylhexenyl, 1,1,3-trimethylhexenyl, 1,2,3,4-tetramethylpentenyl, nonenyl, decenyl, undecenyl, 1-methylundecenyl, dodecenyl, tridecenyl, tetradecenyl, 2,6,10-trimethylundecenyl, pentadecenyl, 3,7,11-trimethyldodecenyl, hexadecenyl, heptadecenyl, octadecenyl, nonadecenyl, 2,6,10,14-tetramethylpentadecenyl, eicosenyl, 3,7,11,15-tetramethylhexadecenyl, heneicosenyl, docosenyl, tricosenyl, tetracosenyl, 2,6,10,14,18-pentamethylnonadecenyl, pentacosenyl, 3,7,11,15,19-pentamethyleicosenyl, n-pentadienyl, isopentadienyl, 1-methylpentadienyl, 1,3-dimethylbutadienyl, n-hexadienyl, 1-methylhexadienyl, n-heptadienyl, isoheptadienyl, 1,2,3-trimethylbutadienyl, 1-methylheptadienyl, 3-methylheptadienyl, n-octadienyl, 2-ethylhexadienyl, 1,1,3-trimethylhexadienyl, 1,2,3,4-tetramethylpentadienyl, nonadienyl, decadienyl, undecadienyl, 1-methylundecadienyl, dodecadienyl, tridecadienyl, tetradecadienyl, 2,6,10-trimethylundecadienyl, pentadecadienyl, 3,7,11-trimethyldodecadienyl, hexadecadienyl, heptadecadienyl, octadecadienyl, nonadecadienyl, 2,6,10,14-tetramethylpentadecadienyl, eicosadienyl, 3,7,11,15-tetramethylhexadecadienyl, heneicosadienyl, docosadienyl, tricosadienyl, tetracosadienyl, 2,6,10,14,18-pentamethylnonadecadienyl, pentacosadienyl, 3,7,11,15,19-pentamethyleicosadienyl, n-hexatrienyl, 1-methylhexatrienyl, 3-methylhexatrienyl, 5-methylhexatrienyl, n-heptatrienyl, isoheptatrienyl, 1,2,3-trimethylbutatrienyl, 1-methylheptatrienyl, 3-methylheptatrienyl, n-octatrienyl, 2-ethylhexatrienyl, 1,3,5-trimethylhexatrienyl, 1,2,3,4-tetramethylpentatrienyl, nonatrienyl, decatrienyl, undecatrienyl, 1-methylundecatrienyl, dodecatrienyl, tridecatrienyl, tetradecatrienyl, 2,6,10-trimethylundecatrienyl, pentadecatrienyl, 3,7,11-trimethyldodecatrienyl, hexadecatrienyl, heptadecatrienyl, octadecatrienyl, nonadecatrienyl, 2,6,10,14-tetramethylpentadecatrienyl, eicosatrienyl, 3,7,11,15-tetramethylhexadecatrienyl, heneicosatrienyl, heneicosahexaenyl, docosatrienyl, tricosatrienyl, tetracosatrienyl, 2,6,10,14,18-pentamethylnonadecatrienyl, pentacosatrienyl, 3,7,11,15,19-pentamethyleicosatrienyl, nonadecatetraenyl, nonadecapentaenyl, including all E or Z and R or S stereoisomers, as mixtures or separately,

pentadec-8Z-enyl, heptadec-8Z-enyl, nonadec-10Z-enyl, heneicosa-12Z-enyl, tricosa-14Z-enyl, heptadeca-8Z,11Z-dienyl, heptadeca-8Z,10Z-dienyl, heptadeca-5Z,8Z,11Z-trienyl, heptadeca-8Z,11Z,14Z-trienyl, nonadeca-7Z,10Z,13Z-trienyl, nonadeca-4Z,7Z,10Z, 13Z,-tetraenyl, nonadeca-7Z,10Z,13Z,16Z-tetraenyl, heneicosa-3Z,6Z,9Z,12Z,15Z,18Z-pentaenyl.

In a preferred embodiment, $R^3$ is selected in the group consisting in C3, C4, C5, C6, C7, C8, C9, C10, C11, C12, C13, C14, C15, C16, C17, C18, C19, C20, C21, C22, C23, C24 and C25 alkyl, linear or branched, saturated or not, cyclic or not. In a preferred embodiment, R3 is a C3-C20 alkyl, linear or branched, saturated or not, cyclic or not. Advantageously, R3 is a C6-C15 alkyl, linear or branched, saturated or not, cyclic or not, more advantageously, R3 is a C8-C12 alkyl, linear or branched, saturated or not, cyclic or not, advantageously R3 is a C10 alkyl, linear or branched, saturated or not, cyclic or not.

The "C1-C6 alkyl, linear or branched, saturated or not, cyclic or not, not substituted" of $R^4$ $R^5$ includes a C1-C6 linear saturated alkyl, a C1-C6 linear unsaturated alkyl, a C1-C6 branched saturated alkyl, a C1-C6 branched unsaturated alkyl, a C1-C6 saturated cycloalkyl, a C3-C6 unsaturated cycloalkyl, a C3-C6 branched saturated cycloalkyl and a C3-C6 branched unsaturated cycloalkyl. For example, methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, cyclopropyl, cyclobutyl, cyclopentyl, methylcyclopentyl, cyclohexyl,

methylene, ethylene, trimethylene, propylene, tetramethylene, pentamethylene, hexamethylene, 2-butenyl, 3-butenyl, 2-methyl-2-propenyl, 1-methyl-2-propenyl and 3-methyl-2-butenyl.

The "C2-C6 alkyl, linear or branched, saturated or not, cyclic or not, substituted by at least a hydroxyl group" of $R^4$ and $R^5$ includes a C2-C6 alkyl linear saturated substituted by at least a hydroxyl group, a C2-C6 alkyl linear unsaturated substituted by at least a hydroxyl group, a C2-C6 alkyl linear branched saturated substituted by at least a hydroxyl group, a C2-C6 alkyl linear branched unsaturated substituted by at least a hydroxyl group, a C3-C6 cycloalkyl saturated substituted by at least a hydroxyl group, a C3-C6 cycloalkyl unsaturated substituted by at least a hydroxyl group, a C3-C6 cycloalkyl branched saturated substituted by at least a hydroxyl group and a C3-C6 cycloalkyl branched unsaturated substituted by at least a hydroxyl group. For example, 2-hydroxyethyl, 3-hydroxypropyl, 2-hydroxypropyl, 4-hydroxybutyl, 3-hydroxybutyl, 2-hydroxybutyl, 5-hydroxypentyl, 4-hydroxypentyl, 3-hydroxypentyl, 2-hydroxypentyl, 6-hydroxyhexyl, 5-hydroxyhexyl, 4-hydroxyhexyl, 3-hydroxyhexyl, 2-hydroxyhexyl,

2,3-dihydroxypropyl, 1,3-dihydroxyprop-2-yl,

2,3,4-trihydroxybutyl, 2,3,4,5-tetrahydroxypentyl, and 2,3,4,5,6-pentahydroxyhexyl, 2-hydroxycyclopentyl, 3-hydroxycyclopentyl, 2-hydroxycyclohexyl, 3-hydroxycyclohexyl, 4-hydroxycyclohexyl, 1'-hydroxycyclopentylmethyl, 2'-hydroxycyclopentylmethyl, 3'-hydroxycyclopentylmethyl, 2,3-dihydroxypropyl, 2,3-dihydroxybutyl, 2,4-dihydroxybutyl, 3,4-dihydroxybutyl, 1-methyl-2,3-dihydroxypropyl, 1-hydroxymethyl-2-hydroxypropyl, 2,3,4-trihydroxybutyl, 1-hydroxymethyl-2,3-dihydroxypropyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 1-hydroxymethyl-2-hydroxybutyl, 1-hydroxymethyl-3-hydroxybutyl, 1-hydroxymethyl-4-hydroxybutyl, 1-methyl-2,3-dihydroxybutyl, 1-methyl-2,4-dihydroxybutyl, 1-methyl-3,4-dihydroxybutyl, 1-ethyl-2,3-dihydroxypropyl, 1-hydroxyethyl-3-hydroxypropyl, 2-hydroxyethyl-3-hydroxypropyl, 1-hydroxyethyl-2-hydroxypropyl, 2-hydroxyethyl-2-hydroxypropyl, 2,3,4-trihydroxypentyl, 2,3,5-trihydroxypentyl, 3,4,5-trihydroxypentyl, 1-hydroxymethyl-2,3-dihydroxybutyl, 1-hydroxymethyl-2,4-dihydroxybutyl, 1-hydroxymethyl-3,4-dihydroxybutyl, 1-methyl-2,3,4-trihydroxybutyl, 1-hydroxyethyl-2,3-dihydroxypropyl, 2-hydroxyethyl-2,3-dihydroxypropyl, 2,3,4,5-tetrahydroxypentyl, 1-hydroxymethyl-2,3,4-trihydroxybutyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl, 5,6-dihydroxyhexyl, 1,2-dihydroxyethylbutyl, hydroxyethyl-2-hydroxybutyl, 2-hydroxyethyl-3-hydroxybutyl, 2-hydroxyethyl-4-hydroxybutyl, 1-hydroxyethyl-2-hydroxybutyl, 1-hydroxyethyl-3-hydroxybutyl, 1-hydroxyethyl-4-hydroxybutyl, 1-ethyl-2,3-dihydroxybutyl, 1-ethyl-2,4-dihydroxybutyl, 1-ethyl-3,4-dihydroxybutyl, 2,3,4-trihydroxyhexyl, 2,3,5-trihydroxyhexyl, 2,3,6-trihydroxyhexyl, 2,4,5-trihydroxyhexyl, 2,4,6-trihydroxyhexyl, 2,5,6-trihydroxyhexyl, 3,4,5-trihydroxyhexyl, 3,4,6-trihydroxyhexyl, 3,5,6-trihydroxyhexyl, 4,5,6-trihydroxyhexyl, 1-hydroxymethyl-2,3-dihydroxypentyl, 1-hydroxymethyl-2,4-dihydroxypentyl, 1-hydroxymethyl-2,5-dihydroxypentyl, 1-hydroxymethyl-3,4-dihydroxypentyl, 1-hydroxymethyl-3,5-dihydroxypentyl, 1-hydroxymethyl-4,5-dihydroxypentyl, 1-methyl-2,3,4-trihydroxypentyl, 1-methyl-2,3,5-trihydroxypentyl, 1-methyl-3,4,5-trihydroxypentyl, 1,2-dihydroxyethyl-2-hydroxybutyl, 1,2-dihydroxyethyl-3-hydroxybutyl, 1,2-dihydroxyethyl-4-hydroxybutyl, 2-hydroxyethyl-2,3-dihydroxybutyl, 2-hydroxyethyl-2,4-dihydroxybutyl, 2-hydroxyethyl-3,4-dihydroxybutyl, ethyl-2,3,4-trihydroxybutyl, 2,3,4,5-tetrahydroxyhexyl, 2,3,4,6-tetrahydroxyhexyl, 2,3,5,6-tetrahydroxyhexyl, 2,4,5,6-tetrahydroxyhexyl, 3,4,5,6-tetrahydroxyhexyl, 1-hydroxymethyl-2,3,4-trihydroxypentyl, 1-hydroxymethyl-2,3,5-trihydroxypentyl, 1-hydroxymethyl-2,4,5-trihydroxypentyl, 1-methyl-2,3,4,5-tetrahydroxypentyl, 1,2-dihydroxyethyl-2,3-dihydroxybutyl, 1,2-dihydroxyethyl-2,4-dihydroxybutyl, 1,2-dihydroxyethyl-3,4-dihydroxybutyl, 2-hydroxyethyl-2,3,4-trihydroxybutyl, 1-hydroxyethyl-2,3,4-trihydroxybutyl, 2,3,4,5,6-pentahydroxyhexyl, 1-hydroxymethyl-2,3,4,5-tetrahydroxypentyl, 1,2-dihydroxyethyl-2,3,4-trihydroxybutyl, 2,3-dihydroxycyclopentyl, 2,4-dihydroxycyclopentyl, 2,5-dihydroxycyclopentyl, 3,4-dihydroxycyclopentyl 2,3,4-trihydroxycyclopentyl,. ,2,3,5-trihydroxycyclopentyl,. 2,3,4,5-tetrahydroxycyclopentyl,. 2,3-dihydroxycyclohexyl, 2,4-dihydroxycyclohexyl, 2,5-dihydroxycyclohexyl, 2,6-dihydroxycyclohexyl, 3,4-dihydroxycyclohexyl and 3,5-dihydroxycyclohexyl, 2,3,4-trihydroxycyclohexyl,. ,2,3,5-trihydroxycyclohexyl,. 2,3,6-trihydroxycyclohexyl,. 2,4,5-trihydroxycyclohexyl,. 2,4,6-trihydroxycyclohexyl,. 2,5,6-trihydroxycyclohexyl,. 3,4,5-trihydroxycyclohexyl,. 2,3,4,5-tetrahydroxycyclohexyl,. ,. 2,3,4,6-tetrahydroxycyclohexyl,. 2,3,5,6-tetrahydroxycyclohexyl, 2,4,5,6-tetrahydroxycyclohexyl, and 2,3,4,5,6-pentahydroxycyclohexyl.

Advantageously, $R^4$ and $R^5$ are of formula

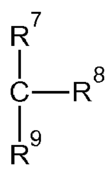

wherein, $R^7$, $R^8$ and $R^9$ are independently from each other hydrogen or a C1-C5 alkyl linear or branched, saturated or not, cyclic or not.

Advantageously, each carbon atom of $R^4$ and $R^5$ is substituted by at most one hydroxyl group.

The "C3-C6 alkyl, linear or branched, saturated or not, cyclic or not, substituted by at least two hydroxyl groups" of $R^4$ and $R^5$ includes a C3-C6 linear saturated alkyl substituted by at least two hydroxyl groups, a C3-C6 linear unsaturated alkyl substituted by at least two hydroxyl groups, a C3-C6 branched saturated alkyl substituted by at least two hydroxyl groups, a C3-C6 branched unsaturated alkyl substituted by at least two hydroxyl groups, a C3-C6 saturated cycloalkyl substituted by at least two hydroxyl groups, a C3-C6 unsaturated cycloalkyl substituted by at least two hydroxyl groups, a C3-C6 branched saturated cycloalkyl substituted by at least two hydroxyl groups and a C3-C6 branched unsaturated cycloalkyl substituted by at least two hydroxyl groups. For example, 2,3,4-trihydroxybutyl, 2,3,4,5-tetrahydroxypentyl and 2,3,4,5,6-pentahydroxyhexyl,

2,3-dihydroxypropyl, 2,3-dihydroxybutyl, 2,4-dihydroxybutyl, 3,4-dihydroxybutyl, 1-methyl-2,3-dihydroxypropyl, 1-hydroxymethyl-2-hydroxypropyl, 2,3,4-trihydroxybutyl, 1-hydroxymethyl-2,3-dihydroxypropyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 1-hydroxymethyl-2-hydroxybutyl, 1-hydroxymethyl-3-hydroxybutyl, 1-hydroxymethyl-4-hydroxybutyl, 1-methyl-2,3-dihydroxybutyl, 1-methyl-2,4-dihydroxybutyl, 1-methyl-3,4-dihydroxybutyl, 1-ethyl-2,3-dihydroxypropyl, 1-hydroxyethyl-3-hydroxypropyl, 2-hydroxyethyl-3-hydroxypropyl, 1-hydroxyethyl-2-hydroxypropyl, 2-hydroxyethyl-2-hydroxypropyl, 2,3,4-trihydroxypentyl, 2,3,5-trihydroxypentyl, 3,4,5-trihydroxypentyl, 1-hydroxymethyl-2,3-dihydroxybutyl, 1-hydroxymethyl-2,4-dihydroxybutyl, 1-hydroxymethyl-3,4-dihydroxybutyl, 1-methyl-2,3,4-trihydroxybutyl, 1-hydroxyethyl-2,3-dihydroxypropyl, 2-hydroxyethyl-2,3-dihydroxypropyl, 2,3,4,5-tetrahydroxypentyl, 1-hydroxymethyl-2,3,4-trihydroxybutyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl, 5,6-dihydroxyhexyl, 2-hydroxyethyl-2-hydroxybutyl, 2-hydroxyethyl-3-hydroxybutyl, 2-hydroxyethyl-4-hydroxybutyl, 1-hydroxyethyl-2-hydroxybutyl, 1-hydroxyethyl-3-hydroxybutyl, 1-hydroxyethyl-4-hydroxybutyl, 1-ethyl-2,3-dihydroxybutyl, 1-ethyl-2,4-dihydroxybutyl, 1-ethyl-3,4-dihydroxybutyl, 2,3,4-trihydroxyhexyl, 2,3,5-trihydroxyhexyl, 2,3,6-trihydroxyhexyl, 2,4,5-trihydroxyhexyl, 2,4,6-trihydroxyhexyl, 2,5,6-trihydroxyhexyl, 3,4,5-trihydroxyhexyl, 3,4,6-trihydroxyhexyl, 3,5,6-trihydroxyhexyl, 4,5,6-trihydroxyhexyl, 1-hydroxymethyl-2,3-dihydroxypentyl, 1-hydroxymethyl-2,4-dihydroxypentyl, 1-hydroxymethyl-2,5-dihydroxypentyl, 1-hydroxymethyl-3,4-dihydroxypentyl, 1-hydroxymethyl-3,5-dihydroxypentyl, 1-hydroxymethyl-4,5-dihydroxypentyl, 1-methyl-2,3,4-trihydroxypentyl, 1-methyl-2,3,5-trihydroxypentyl, 1-methyl-3,4,5-trihydroxypentyl, 2-hydroxyethyl-2,3-dihydroxybutyl, 2-hydroxyethyl-2,4-dihydroxybutyl, 2-hydroxyethyl-3,4-dihydroxybutyl, ethyl-2,3,4-trihydroxybutyl, 2,3,4,5-tetrahydroxyhexyl, 2,3,4,6-tetrahydroxyhexyl, 2,3,5,6-tetrahydroxyhexyl, 2,4,5,6-tetrahydroxyhexyl, 3,4,5,6-tetrahydroxyhexyl, 1-hydroxymethyl-2,3,4-trihydroxypentyl, 1-hydroxymethyl-2,3,5-trihydroxypentyl, 1-hydroxymethyl-2,4,5-trihydroxypentyl, 1-methyl-2,3,4,5-tetrahydroxypentyl, 1,2-dihydroxyethyl-2,3-dihydroxybutyl, 1,2-dihydroxyethyl-2,4-dihydroxybutyl, 1,2-dihydroxyethyl-3,4-dihydroxybutyl, 2-hydroxyethyl-2,3,4-trihydroxybutyl, 1-hydroxyethyl-2,3,4-trihydroxybutyl, ethyl-1,2,3,4-tetrahydroxybutyl, 2,3,4,5,6-pentahydroxyhexyl, 1-hydroxymethyl-2,3,4,5-tetrahydroxypentyl, 1,2-dihydroxyethyl-2,3,4-trihydroxybutyl, 2,3-dihydroxycyclopentyl, 2,4-dihydroxycyclopentyl, 2,5-dihydroxycyclopentyl, 3,4-dihydroxycyclopentyl 2,3,4-trihydroxycyclopentyl,. ,2,3,5-trihydroxycyclopentyl,. 2,3,4,5-tetrahydroxycyclopentyl,. 2,3-dihydroxycyclohexyl, 2,4-dihydroxycyclohexyl, 2,5-dihydroxycyclohexyl, 2,6-dihydroxycyclohexyl, 3,4-dihydroxycyclohexyl and 3,5-dihydroxycyclohexyl, 2,3,4-trihydroxycyclohexyl,. ,2,3,5-trihydroxycyclohexyl,. 2,3,6-trihydroxycyclohexyl,. 2,4,5-trihydroxycyclohexyl,. 2,4,6-trihydroxycyclohexyl,. 2,5,6-trihydroxycyclohexyl,. 3,4,5-trihydroxycyclohexyl,. 2,3,4,5-tetrahydroxycyclohexyl,. ,. 2,3,4,6-tetrahydroxycyclohexyl,. ,. 2,3,5,6-tetrahydroxycyclohexyl,. 2,4,5,6-tetrahydroxycyclohexyl, and 2,3,4,5,6-pentahydroxycyclohexyl.

Combinations of $R^4$ and $R^5$ are advantageously as follows:

- $R^4$ is selected in the group comprising 2-hydroxyethyl, 3-hydroxypropyl, 2-hydroxypropyl, 4-hydroxybutyl, 3-hydroxybutyl, 2-hydroxybutyl, 5-hydroxypentyl, 4-hydroxypentyl, 3-hydroxypentyl, 2-hydroxypentyl, 6-hydroxyhexyl, 5-hydroxyhexyl, 4-hydroxyhexyl, 3-hydroxyhexyl, 2-hydroxyhexyl, 2-hydroxycyclopentyl, 3-hydroxycyclopentyl, 2-hydroxycyclohexyl, 3-hydroxycyclohexyl, 4-hydroxycyclohexyl, and $R^5$ is selected in the group comprising $R^4$ is selected in the group comprising 2-hydroxyethyl, 3-hydroxypropyl, 2-hydroxypropyl, 4-hydroxybutyl, 3-hydroxybutyl, 2-hydroxybutyl, 5-hydroxypentyl, 4-hydroxypentyl, 3-hydroxypentyl, 2-hydroxypentyl, 6-hydroxyhexyl, 5-hydroxyhexyl, 4-hydroxyhexyl, 3-hydroxyhexyl, 2-hydroxyhexyl, 2-hydroxycyclopentyl, 3-hydroxycyclopentyl, 2-hydroxycyclohexyl, 3-hydroxycyclohexyl, 4-hydroxycyclohexyl;
- $R^4$ is selected in the group comprising 2,3-dihydroxypropyl, 1,3-dihydroxyprop-2-yl, 2,3-dihydroxybutyl, 2,4-dihydroxybutyl, 3,4-dihydroxybutyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl and 5,6-dihydroxyhexyl, and
  $R^5$ is selected in the group comprising 2,3-dihydroxypropyl, 1,3-dihydroxyprop-2-yl, 2,3-dihydroxybutyl, 2,4-dihydroxybutyl, 3,4-dihydroxybutyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl and 5,6-dihydroxyhexyl;
- $R^4$ is selected in the group comprising methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, cyclopropyl, cyclobutyl, cyclopentyl, methylcyclopentyl and dimethylcyclopentyl, cyclohexyl, and
  $R^5$ is selected in the group comprising 2,3-dihydroxypropyl, 1,3-dihydroxyprop-2-yl, 2,3-dihydroxybutyl, 2,4-dihydroxybutyl, 3,4-dihydroxybutyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl and 5,6-dihydroxyhexyl;
- $R^4$ is selected in the group comprising 2,3-dihydroxypropyl, 1,3-dihydroxyprop-2-yl, 2,3-dihydroxybutyl, 2,4-dihydroxybutyl, 3,4-dihydroxybutyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl, 4,6-dihydroxyhexyl and 5,6-dihydroxyhexyl,, and
  $R^5$ is selected in the group comprising methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, cyclopropyl, cyclobutyl, cyclopentyl, methylcyclopentyl and dimethylcyclopentyl, cyclohexyl.

Advantageously, $R^4$ and $R^5$ are together and independently from each other

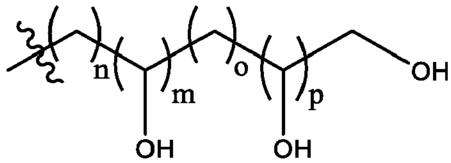

wherein n is an integer from 1 to 5 and m, o and p are together and independently from each other an integer from 0 to 4, and wherein the sum of n, m, o and p is an integer from 1 to 5.

By "from 1 to 5", it is meant in the invention 1, 2, 3, 4 and 5.

By "from 0 to 4", it is meant in the invention 0, 1, 2, 3, and 4.

Advantageously, $R^4$ is

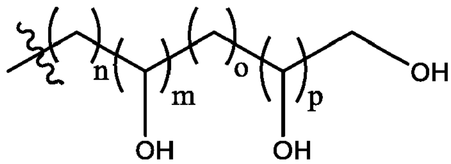

as defined above and $R^5$ is a C1-C6 alkyl, linear or branched, saturated or not, cyclic or not, not substituted

or

$R^4$ is an unsubstituted C1-C6 alkyl, linear or branched, saturated or not, cyclic or not, not substituted and $R^5$ is

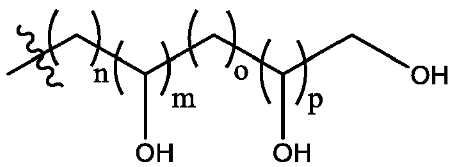

as defined above,

wherein n and m are together and independently from each other an integer from 1 to 4 and o and p are together and independently from each other an integer from 0 to 3,

By "from 1 to 4", it is meant in the invention 1, 2, 3, and 4.

By "from 0 to 3", it is meant in the invention 0, 1, 2, and 3.

In an advantageous embodiment, $R^4$ and $R^5$ are together and independently from each other

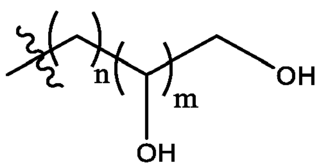

or

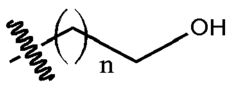

,

or

$R^4$ is

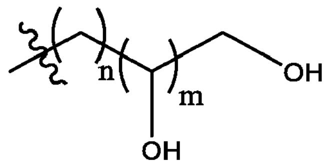

and $R^5$ is

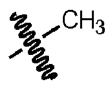

,

or
$R^4$ is

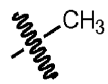

and $R^5$ is

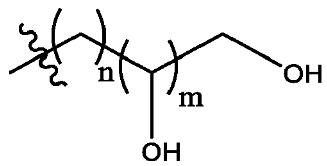

wherein n and m are together and independently from each other an integer from 1 to 4, and wherein the sum of n and m is an integer from 2 to 5.

By "from 1 to 4", it is meant in the invention 1, 2, 3 and 4.

The "C1-C6 alkyl, linear or branched, saturated or not, cyclic or not" of $R^6$ includes a C1-C6 alkyl linear saturated, a C1-C6 alkyl linear unsaturated, a C1-C6 alkyl linear branched saturated, a C1-C6 alkyl linear branched unsaturated, a C1-C6 cycloalkyl saturated substituted by at least a hydroxyl group, a C1-C6 cycloalkyl unsaturated, a C1-C6 cycloalkyl branched saturated and a C1-C6 cycloalkyl branched unsaturated. For example, methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl,

cyclopropyl, cyclobutyl, cyclopentyl, methylcyclopentyl, dimethylcyclopentyl, cyclohexyl, methylene, ethylene, trimethylene, propylene, tetramethylene, pentamethylene, hexamethylene, 2-butenyl, 3-butenyl, 2-methyl-2-propenyl, 1-methyl-2-propenyl and 3-methyl-2-butenyl.

Advantageously, $R^6$ is a C1-C6 alkyl, linear or branched, saturated or not, cyclic or not, not substituted.

In a preferred embodiment, $R^6$ is selected in the group consisting of C1, C2, C3, C4, C5 and C6 alkyl, linear or branched, saturated or not, cyclic or not. In a preferred embodiment, $R^6$ is a C1-C4 alkyl, linear or branched, saturated or not, cyclic or not. Advantageously, $R^6$ is a C1-C2 alkyl, linear or branched, saturated or not, cyclic or not.

Advantageously, said fluorescent compound is of formula I-a:

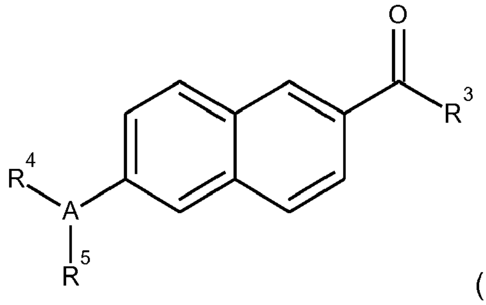

(I-a),

wherein $R^3$, $R^4$ and $R^5$ are as defined above.

More, advantageously, said fluorescent compound is of formula I-a wherein $R^3$, is a C8-C22 alkyl, linear or branched, saturated or not, cyclic or not.

In another advantageous embodiment, the fluorescent compound is of formula:

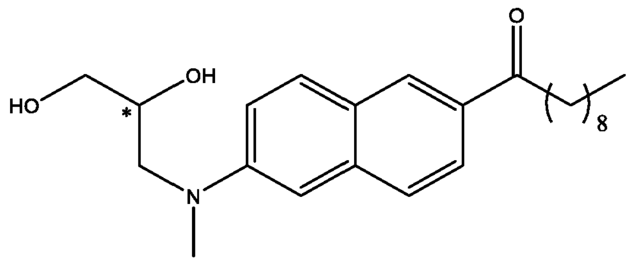

(11)

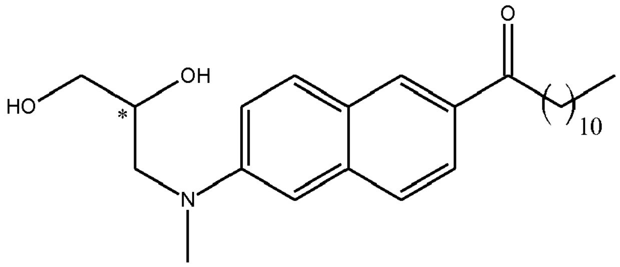

(12),

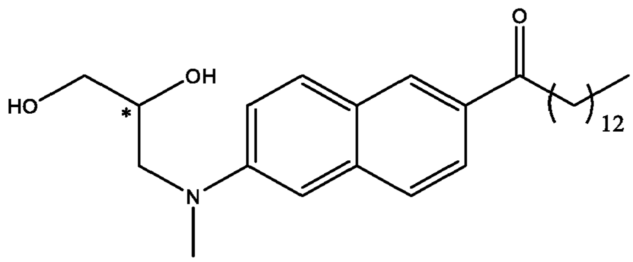

(13),

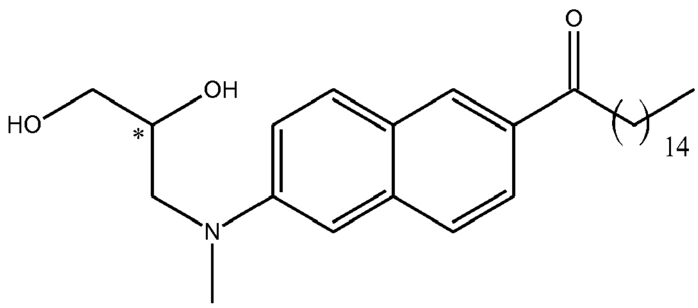

(14),

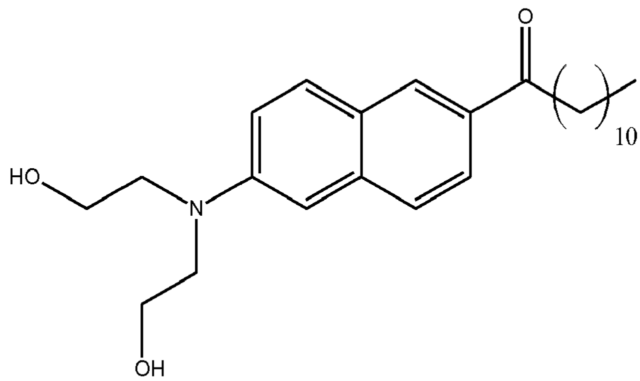

(15),

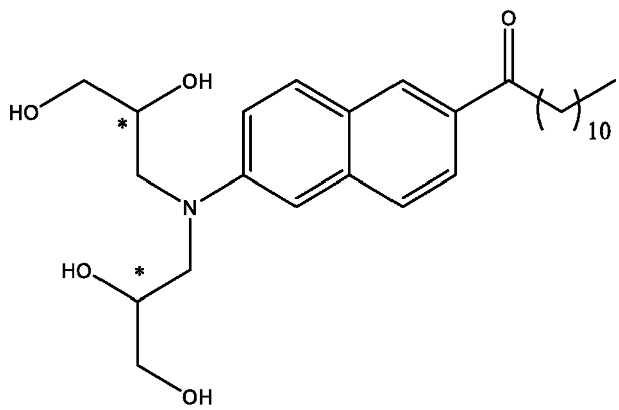

(16),

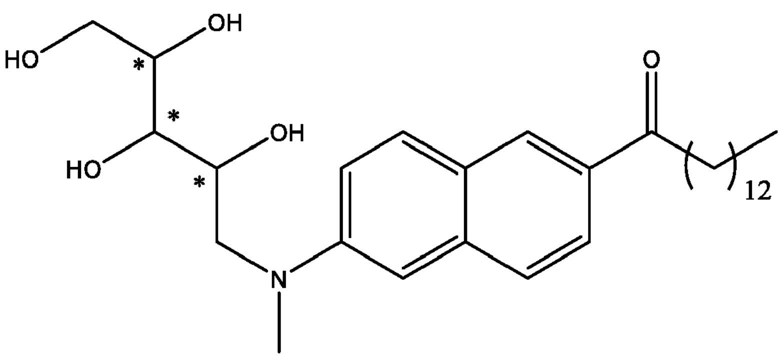

(17),

or

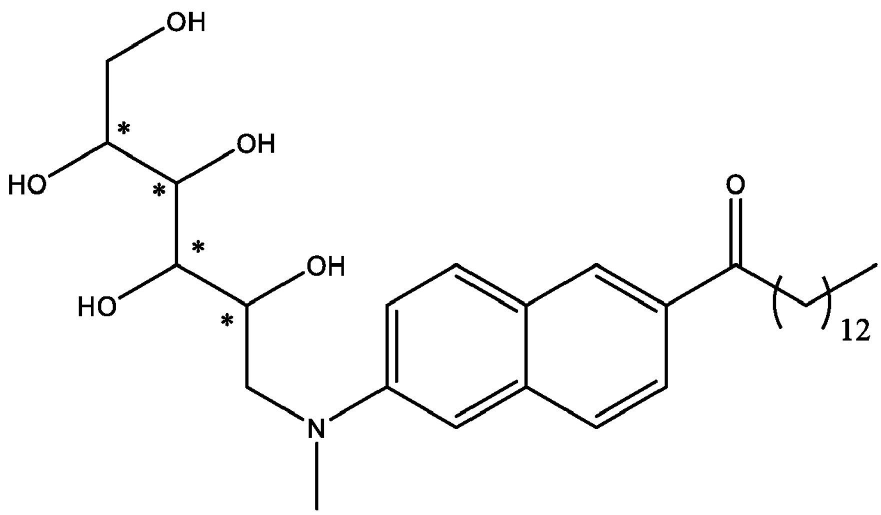

(18)

wherein the asterisk means that the carbon atom is an asymmetric carbon.

Advantageously, the fluorescent compounds of formula 11 to 14 are selected in the group consisting of a racemic mixture, the R enantiomer and the S enantiomer. More advantageously, the fluorescent compounds of formula 11 is a racemic mixture. Particularly, the fluorescent compound of formula 16 is selected in the group consisting in a racemic mixture of diastereomers, a racemic mixture of a single diastereomer, the RR enantiomer, the RS enantiomer, the SS enantiomer and the SR enantiomer. Particularly, the fluorescent compound of formula 17 is selected in the group consisting in a racemic mixture of diastereomers, a racemic mixture of a single diastereomer, the D- or L- arabino, ribo, xylo or lyxo configuration. Particularly, the fluorescent compound of formula 18 is selected in the group consisting in a racemic mixture of diastereomers, a racemic mixture of a single diastereomer, the D- or L- gluco, manno, galacto, allo, altro, talo, gulo, ido configuration.

The invention also relates to the use of a fluorescent compound as defined above, for, preferably *in vitro* and *ex vivo,* labelling a lipid membrane or part thereof.

"In vitro" means in the invention that which takes place outside normal biological context. "Ex vivo" means in the invention that which takes place outside an organism. Specifically, ex vivo refers to experimentation or measurements done in or on tissue from an organism in an external environment with minimal alteration of natural conditions.

Lipid membranes are complex and dynamic systems. They are formed by amphipathic lipids, consisting of polar, hydrophilic head groups and apolar, hydrophobic chains, which tend to aggregate in the lipid-water systems into specific phases such as micelles, lamellar, cubic or inverse hexagonal phases. This supramolecular phase organization is driven by the shape and concentration of the amphipathic molecule as well as the system's temperature. The lipid phase organization reflects lipid packing at the lowest energy needed to balance repulsive head group interactions and hydrophobic chains effects.

Unexpectedly, the fluorescent compound as defined above labels solely lipids which are organized in lipid membranes. That means that the other classes of lipids are not labelled by the said fluorescent compound. This aspect of the invention is an important parameter for applications such as quantifying lipids organized in a lipid membrane and compared the membrane fluidity variation between two cells, as described below.

In a preferred embodiment, the lipid membrane comprises lipids which are selected from the group consisting of phospholipid, sphingolipid, sterol, glycolipid, fatty acid, phosphoglyceride or a mix thereof. Advantageously, the lipid membrane comprises lipids which are selected from the group comprising phospholipid, glycerophospholipid and phosphyglyceride.

In a preferred embodiment, the lipid membrane or part thereof is selected between a lipid monolayer membrane and a lipid bilayer membrane. Lipid bilayer membranes, which constitute the majority of the biological membranes, are composed of two opposing leaflets/layers.

In another preferred embodiment, the lipid membrane or part thereof is selected from the group consisting ofof liposome or biological membrane.

By "liposome", it is meant in the invention a closed vesicle having a lipid bilayer membrane.

By "biological membrane", it is meant in the invention a membrane which pertains to an organism. Particularly, the natural lipid membrane is selected from the group consisting ofof eukaryote lipid membrane, prokaryote lipid membrane and virus lipid membrane. It is notable that the virus lipid membrane is commonly eukaryote or prokaryote lipid membrane.

By "prokaryote", it is meant in the invention both the Gram-negative prokaryote and the Gram-positive prokaryote. Advantageously, the lipid membrane or part thereof is a Gram-positive lipid membrane.

Advantageously, the lipid membrane or part thereof is a eukaryote lipid membrane. More advantageously, the lipid membrane or part thereof is a cell membrane and selected from the group comprising plasma membrane, Golgi membrane, mitochondrial membrane, liposomes, nuclear envelope, endoplasmic reticulum, vesicles and chloroplasts membrane.

By "vesicles", it is meant in the invention both the intracellular vesicles and extracellular vesicles. Examples of intracellular vesicles include vacuoles, lysosomes, transport vesicles and secretory vesicles. Examples of extracellular vesicles include ectosomes/microvesicles and exosomes. Advantageously, the said lipid membrane is an exosome.

The invention further relates to a method for, with preference *in vitro* and *ex vivo,* labelling a lipid membrane or part thereof comprising a step of contacting a fluorescent compound as defined above with a lipid membrane or part thereof, and a step of detecting the fluorescence of the said lipid membrane or part thereof labelled by the said fluorescent compound.

In a preferred embodiment, the lipid membrane or part thereof is selected from a lipid monolayer membrane and a lipid bilayer membrane.

In a preferred embodiment, the lipid membrane comprises lipids which are selected from the group comprising phospholipid, sphingolipid, sterol, glycerophospholipid, glycolipid, fatty acid, phosphoglyceride or a mix thereof. Advantageously, the lipid membrane comprises lipids which are selected from the group comprising phospholipid, glycerophospholipid and phosphyglyceride.

In a preferred embodiment, the lipid membrane or part thereof is selected from the group consisting ofof liposome and biological membrane. Particularly, the natural lipid membrane is selected from the group consisting ofeukaryote lipid membrane, prokaryote lipid membrane and virus lipid membrane.

In a preferred embodiment, the lipid membrane is a eukaryote cell lipid membrane.

Advantageously, the lipid membrane or part thereof is a eukaryote lipid membrane. More advantageously, the lipid membrane or part thereof is selected from the group comprising plasma membrane, Golgi membrane, mitochondrial membrane, liposomes, nuclear membrane, endoplasmic reticulum, vesicles and chloroplasts.

In a preferred embodiment, the compound as described above is dissolved in solution of DMSO and/or ethanol. Advantageously, at the end of the step of contacting the said fluorescent compound with a lipid membrane, the said fluorescent compound has a concentration from 0.01 to 10 μM. By "0.01 to 10 μM", it is meant in the invention 0.01 μM, 0.02 μM, 0.03 μM, 0.04 μM, 0.05 μM, 0.06 μM, 0.07 μM, 0.08 μM, 0.09 μM, 0.10 μM, 0.11 μM, 0.12 μM, 0.13 μM, 0.14 μM, 0.15 μM, 0.16 μM, 0.17 μM, 0.18 μM, 0.19 μM, 0.20 μM, 0.21 μM, 0.22 μM, 0.23 μM, 0.24 μM, 0.25 μM, 0.26 μM, 0.27 μM, 0.28 μM, 0.29 μM, 0.30 μM, 0.31 μM, 0.32 μM, 0.33 μM, 0.34 μM, 0.35 μM, 0.36 μM, 0.37 μM, 0.38 μM, 0.39 μM, 0.40 μM, 0.41 μM, 0.42 μM, 0.43 μM, 0.44 μM, 0.45 μM, 0.46 μM, 0.47 μM, 0.48 μM, 0.49 μM, 0.50 μM, 0.51 μM, 0.52 μM, 0.53 μM, 0.54 μM, 0.55 μM, 0.56 μM, 0.57 μM, 0.58 μM, 0.59 μM, 0.60 μM, 0.61 μM, 0.62 μM, 0.63 μM, 0.64 μM, 0.65 μM, 0.66 μM, 0.67 μM, 0.68 μM, 0.69 μM, 0.70 μM, 0.71 μM, 0.72 μM, 0.73 μM, 0.74 μM, 0.75 μM, 0.76 μM, 0.77 μM, 0.78 μM, 0.79 μM, 0.80 μM, 0.81 μM, 0.82 μM, 0.83 μM, 0.84 μM, 0.85 μM, 0.86 μM, 0.87 μM, 0.88 μM, 0.89 μM, 0.90 μM, 0.91 μM, 0.92 μM, 0.93 μM, 0.94 μM, 0.95 μM, 0.96 μM, 0.97 μM, 0.98 μM, 0.99 μM, 1.0 μM, 1.1 μM, 1.2 μM, 1.3 μM, 1.4 μM, 1.5 μM, 1.6 μM, 1.7 μM, 1.8 μM, 1.9 μM, 2.00 μM, 2.1 μM, 2.2 μM, 2.3 μM, 2.4 μM, 2.5 μM, 2.6 μM, 2.7 μM, 2.8 μM, 2.9 μM, 3.0 μM, 3.1 μM, 3.2 μM, 3.3 μM, 3.4 μM, 3.5 μM, 3.6 μM, 3.7 μM, 3.8 μM, 3.9 μM, 4.0 μM, 4.1 μM, 4.2 μM, 4.3 μM, 4.4 μM, 4.5 μM, 4.6 μM, 4.7 μM, 4.8 μM, 4.9 μM, 5.0 μM, 5.1 μM, 5.2 μM, 5.3 μM, 5.4 μM, 5.5 μM, 5.6 μM, 5.7 μM, 5.8 μM, 5.9 μM, 6.0 μM, 6.1 μM, 6.2 μM, 6.3 μM, 6.4 μM, 6.5 μM, 6.6 μM, 6.7 μM, 6.8 μM, 6.9 μM, 7.0 μM, 7.1 μM, 7.2 μM, 7.3 μM, 7.4 μM, 7.5 μM, 7.6 μM, 7.7 μM, 7.8 μM, 7.9 μM, 8.0 μM, 8.1 μM, 8.2 μM, 8.3 μM, 8.4 μM, 8.5 μM, 8.6 μM, 8.7 μM, 8.8 μM, 8.9 μM, 9.0 μM, 9.1 μM, 9.2 μM, 9.3 μM, 9.4 μM, 9.5 μM, 9.6 μM, 9.7 μM, 9.8 μM, 9.9 μM and 10 μM.

In a preferred embodiment, the step of contacting a fluorescent compound as defined above with a lipid membrane or part thereof is performed during at least 30 seconds. Advantageously, said step is performed from 30 seconds to 60 hours. By "from 30 seconds to 60 hours", it is meant 30 seconds, 31 seconds, 32 seconds, 33 seconds, 34 seconds, 35 seconds, 36 seconds, 37 seconds, 38 seconds, 39 seconds, 40 seconds, 41 seconds, 42 seconds, 43 seconds, 44 seconds, 45 seconds, 46 seconds, 47 seconds, 48 seconds, 49 seconds, 50 seconds, 51 seconds, 52 seconds, 53 seconds, 54 seconds, 55 seconds, 56 seconds, 57 seconds, 58 seconds, 59 seconds, 60 seconds, 1 minute, 2 minutes, 3 minutes, 4 minutes, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes, 21 minutes, 22 minutes, 23 minutes, 24 minutes, 25 minutes, 26 minutes, 27 minutes, 28 minutes, 29 minutes, 30 minutes, 31 minutes, 32 minutes, 33 minutes, 34 minutes, 35 minutes, 36 minutes, 37 minutes, 38 minutes, 39 minutes, 40 minutes, 41 minutes, 42 minutes, 43 minutes, 44 minutes, 45 minutes, 46 minutes, 47 minutes, 48 minutes, 49 minutes, 50 minutes, 51 minutes, 52 minutes, 53 minutes, 54 minutes, 55 minutes, 56 minutes, 57 minutes, 58 minutes, 59 minutes, 60 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours,

38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours and 60 hours.

In a preferred embodiment, the step of detecting the fluorescence is performed by fluorimetry, fluorescence microscopy or flow cytometry. When performed by fluorimetry, the fluorescence is typically measured by using a spectrofluorometer or a photomultiplicator.

A spectrofluorometer is an instrument which takes advantage of fluorescent properties of some compounds in order to provide information regarding their concentration and chemical environment in a sample. A certain excitation wavelength is selected, and the emission is observed either at a single wavelength, or a scan is performed to record the intensity versus wavelength, also called an emission spectrum. The instrument is used in fluorescence spectroscopy.

A photomultiplicator is an extremely sensitive detector of light in the ultraviolet, visible, and near-infrared ranges of the electromagnetic spectrum. These detectors multiply the current produced by incident light by as much as 100 million times or $10^8$ (i.e., 160 dB), in multiple dynode stages, enabling (for example) individual photons to be detected when the incident flux of light is low.

In a preferred embodiment, the step of detecting the fluorescence is performed at a temperature from -50°C to 150°C. by "-50°C to 150°C" it is meant -50°C, -49°C, -48°C, - 47°C, -46°C, -45°C, -44°C, -43°C, -42°C, -41°C, -40°C, -39°C, -38°C, -37°C, -36°C, -35°C, -34°C, -33°C, -32°C, -31°C, -30°C, -29°C, -28°C, -27°C, -26°C, -25°C, -24°C, -23°C, -22°C, -21°C, -20°C, -19°C, -18°C, -17°C, -16°C, -15°C, -14°C, -13°C, -12°C, -11°C, -10°C, -9°C, -8°C, -7°C, -6°C, -5°C, -4°C, -3°C, -2°C, -1°C, 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C, 101°C, 102°C, 103°C, 104°C, 105°C, 106°C, 107°C, 108°C, 109°C, 110°C, 111°C, 112°C, 113°C, 114°C, 115°C, 116°C, 117°C, 118°C, 119°C, 120°C, 121°C, 122°C, 123°C, 124°C, 125°C, 126°C, 127°C, 128°C, 129°C, 130°C, 131°C, 132°C, 133°C, 134°C, 135°C, 136°C, 137°C, 138°C, 139°C, 140°C, 141°C, 142°C, 143°C, 144°C, 145°C, 146°C, 147°C, 148°C, 149°C and 150°C.

In a preferred embodiment, the said fluorescent compound is the compound of formula 12 or of formula 14. Advantageously, the detection step is performed by fluorimetry with an excitation wavelength at 390nm and an emission wavelength from 420nm to 520nm. Alternatively, the detection step is performed by fluorescence microscopy with an excitation wavelength at 405nm and an emission wavelength from 420nm to 520nm. Alternatively, the detection step is performed by fluorescence microscopy with an excitation wavelength at 390nm and an emission wavelength from 420nm to 520nm.

By "from 420nm to 520nm", it is meant in the invention 420nm, 421nm, 422nm, 423nm, 424nm, 425nm, 426nm, 427nm, 428nm, 429nm, 430nm, 431nm, 432nm, 433nm, 434nm, 435nm, 436nm, 437nm, 438nm, 439nm, 440nm, 441nm, 442nm, 443nm, 444nm, 445nm, 446nm, 447nm, 448nm, 449nm, 450nm, 451nm, 452nm, 453nm, 454nm, 455nm, 456nm, 457nm, 458nm, 459nm, 460nm, 461nm, 462nm, 463nm, 464nm, 465nm, 466nm, 467nm, 468nm, 469nm, 470nm, 471nm, 472nm, 473nm, 474nm, 475nm, 476nm, 477nm, 478nm, 479nm, 480nm, 481nm, 482nm, 483nm, 484nm, 485nm, 486nm, 487nm, 488nm, 489nm, 490nm, 491nm, 492nm, 493nm, 494nm, 495nm, 496nm, 497nm, 498nm, 499nm, 500nm, 501nm, 502nm, 503nm, 504nm, 505nm, 506nm, 507nm, 508nm, 509nm, 510nm, 511nm, 512nm, 513nm, 514nm, 515nm, 516nm, 517nm, 518nm, 519nm, 520nm.

The invention also relates to a use of a fluorescent compound as defined above, for, with preference *in vitro* and *ex vivo,* evaluating the degree of fluidity of a lipid membrane.

Membrane fluidity depends upon lateral and rotational diffusion of lipids within a lipid membrane. Lipids acquire thermal energy when they are heated up, so that energetic lipids move around more, arranging and rearranging randomly, making the membrane more fluid. At low temperatures, the lipids are laterally ordered and organized in the membrane, and the lipid chains are mostly in the all-trans configuration and pack well together.

The two-dimensional leaflet(s) of a lipid membrane can exist in different states known as crystalline phase, gel phase, ripple phase and fluid phase. The fluid phase is also called crystal liquid phase. In the solid crystalline phase, lipids are highly ordered into all-trans chain conformation generating a compact lipid environment which is characterized by a large reduction in lateral lipid diffusion and low levels of membrane hydration. Lipids are tightly packed parallel to each other into a hexagonal lattice with a spacing of roughly 0.4 nm. The lipid hydrocarbons chains can be tilted with respect to the crystalline phase, thereby forming a gel phase reliant on lipid composition and hydration levels or the presence of other ions. Depending on the composition of the lipid membrane, the transition between gel and fluid phase arises at a specific temperature called the thermotropic phase transition. This process is usually preceded by a so-called pre-transition inducing the formation of a ripple phase, characterized by periodic one-dimensional ripples. In the fluid phase, the lipids are highly disordered, and the triangular lattice order is lost with an increased lateral and rotational diffusion of the lipids.

In a lipid monolayer membrane, each lipid is free to diffuse through the layer, and in a lipid bilayer membrane

each lipid can either diffuse through the layer they belong to or switch to the counterpart layer.

A lipid membrane in a fluid phase can be characterized by a diffusion coefficient of lipids of about $10^{-8}cm^2$ per second. A lipid membrane in gel phase can be characterized by a diffusion coefficient of lipids of about $10^{-11}cm^2$ to $10^{-1}cm^2$ per second.

The invention further relates to a method for, with preference *in vitro* and *ex vivo,* evaluating the degree of fluidity of a lipid membrane, said method comprising the steps of:

a) labelling a lipid membrane or part thereof with a fluorescent compound as defined above by carrying out the method of labelling as defined above,
b) determining a generalized polarization parameter GP of the lipid membrane or part thereof by the following formula

$$GP = \frac{I_{gel} - I_{lc}}{I_{gel} + I_{lc}}$$

wherein $I_{gel}$ and $I_{lc}$ are intensities of fluorescence measured by fluorimetry, typically by using spectrofluorometer or a photomultiplicator, from the said lipid membrane or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,

c) concluding that the lipid membrane is fluid if GP is lower or equal to 0, or concluding that the first lipid membrane is rigid if GP is higher than 0.

Fluorescence intensities are directly obtained from the emission spectrum at the indicated wavelengths.

In a preferred embodiment, the said fluorescent compound is the compound of formula 12, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

In a preferred embodiment, the said fluorescent compound is the compound of formula 14, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

The invention also relates to a method for *in vitro* and *ex vivo* evaluating the degree of fluidity of a lipid membrane, said method comprising the steps of:

a) labelling a lipid membrane or part thereof with a fluorescent compound as defined above by carrying out the method of labelling as defined above,
b) determining a generalized polarization parameter GP of the lipid membrane or part thereof by the following formula

$$GP = \frac{I_{gel} - I_{lc}}{I_{gel} + I_{lc}}$$

wherein $I_{gel}$ and $I_{lc}$ are intensities of fluorescence measured by fluorimetry, typically by using a spectrofluorometer or a photomultiplicator, from the lipid membrane or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,
c) comparing said GP with a reference generalized polarization parameter $GP_R$ from a reference lipid membrane, and
d) concluding that the lipid membrane is more fluid or has higher fluidity compared to the reference lipid membrane if GP is lower than $GP_R$,
concluding that the first lipid membrane is more rigid or has lower fluidity compared to the reference lipid membrane if GP is higher than $GP_R$, or
concluding that the first lipid membrane has the same fluidity or degree of fluidity compared to the reference membrane if GP is equal to $GP_R$.

In a preferred embodiment, the said fluorescent compound is the compound of formula 12, wherein at step b)

the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

In a preferred embodiment, the said fluorescent compound is the compound of formula 14, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

The invention also relates to a use of a fluorescent compound as defined above, for, with preference *in vitro* and *ex vivo,* quantification of lipids organized in at least one lipid membrane or part thereof in an aqueous solution.

The invention also relates to a method for, with preference in vitro and ex vivo, quantification of lipids organized in at least one lipid membrane or part thereof in an aqueous solution, comprising the steps of:

a) labelling a lipid membrane or part thereof with a fluorescent compound as defined above by carrying out the method of labelling as defined above,
b) measuring the intensity of fluorescence $I_{lc}$ by fluorimetry, typically by using a spectrofluorometer or a photomultiplicator, at a wavelength characteristic of said fluorescent compound in gel lipid phase,
c) determining the concentration of said at least one lipid membrane or part thereof, by reporting the value obtained in step b) on a calibration curve of the fluorescence intensity as function of the concentration, the calibration curve being a hyperbolic function of the formula y=ax/(b+x) with y being the intensity of fluorescence and x the concentration of the at least one lipid membrane.

In a preferred embodiment, the said fluorescent compound is the compound of formula 12, wherein at step b) the $I_{lc}$ is measured after an excitation wavelength at 385 nm, and wherein $I_{lc}$ is the intensity of the wavelength 490 nm.

In a preferred embodiment, the said fluorescent compound is the compound of formula 14, wherein at step b) the $I_{lc}$ is measured after an excitation wavelength at 385 nm, and wherein $I_{lc}$ is the intensity of the wavelength 490 nm.

The calibration curve of the intensity of fluorescence is obtained with a lipid membrane of the same nature as the tested at least one lipid membrane. Advantageously, the calibration curve is obtained with several known concentrations of a lipid membrane for which the intensity of fluorescence is measured. Each obtained intensity fluorescence is then plotted on a graph for the respective concentration. A calibration curve of formula y=ax/(b+x) is determined from the graph, with y being the intensity of fluorescence and x the concentration of the at least one lipid membrane. When assessing the concentration of the at least one lipid membrane, because y, a and b are known, x (the concentration) can be determined.

It has to be noted that the position of the maximum fluorescence emission largely depends on the order state of the lipid membrane. This characteristic, which makes them valuable for determining the order state of the membrane may be inconvenient when sample fluidity does not match that of the standard solution. Therefore, the fluorescence intensity parameter can be replaced by the surface under the peak, which does not depend on the fluidity state of the lipid membrane.

The surface under the peak, also called surface under the curve or surface under the spectrum, corresponds to the area under the curve of the fluorescence intensity.

Hence, the invention also relates to a method for, with preference in vitro and ex vivo, quantification of lipids organized in at least one lipid membrane or part thereof in an aqueous solution, comprising the steps of:

a) labelling a lipid membrane or part thereof with a fluorescent compound as defined above by carrying out the method of labelling as defined above,
b) determining the intensity of fluorescence by fluorimetry, typically by using a spectrofluorometer or a photomultiplicator, between two limit wavelengths,
c) calculating the surface under the peak of fluorescence,
d) determining the concentration of said at least one lipid membrane or part thereof, by reporting the value obtained in step c) on a calibration curve of the surface under the peak as function of the concentration, the calibration curve being a hyperbolic function of the formula y=ax/(b+x) with y being the surface under the peak and x the concentration of the at least one lipid membrane.

In a preferred embodiment, the said fluorescent compound is the compound of formula 12, wherein at step b) an excitation wavelength at 385 nm is used.

In a preferred embodiment, the said fluorescent compound is the compound of formula 14, wherein at step b) an excitation wavelength at 385 nm is used.

For step b), the said two limit wavelengths advantageously surround the wavelength $I_{gel}$ characteristic of said fluorescent compound in gel lipid phase. More advantageously, a first wavelength $I_1$ is chosen with $I_1 > I_{gel} + 10$ nm, and a second wavelength $I_2$ is chosen with $I_2 < I_{gel}$ -10nm.

The calibration curve of the surface under the peak is also obtained with a lipid membrane of the same nature

as the tested at least one lipid membrane, and the intensities of fluorescence are determined between the same two limit wavelengths. Advantageously, the calibration curve is obtained with several known concentrations of a lipid membrane for which the surface under the peak is calculated. Each obtained value of surface under the peak is then plotted on a graph for the respective concentration. A calibration curve of formula y=ax/(b+x) is determined from the graph, with y being the surface under the peak and x the concentration of the at least one lipid membrane. When assessing the concentration of the at least one lipid membrane, because y, a and b are known, x can be determined.

The invention also relates to a use of a fluorescent compound as defined above, for, with preference *in vitro* and *ex vivo,* diagnosis of a cancer in an individual.

Biological membrane of cancer cells mostly present a different degree of fluidity from their normal counterparts. Hence, because the compound as defined above can in one hand be used for *in vitro* labelling cell membrane, and on the other hand can be used to finely determined a difference of the degree of fluidity between two lipid membrane, it becomes possible to diagnosis cancer in an individual by the measure of the lipid membrane fluidity of a cell membrane.

By "cancer", it is meant in the invention any type of cancer, i.e. solid tumours, leukaemia and lymphoma. Examples of cancer include lymphoma, melanoma, breast cancer, lung cancer, liver cancer, bladder cancer, cervical cancer, prostate cancer, gastric cancer, leukemia, head cancer, head and neck cancer, colorectal cancer, kidney cancer, colon cancer, ovarian cancer, testicular germ cell tumors, all sarcoma, neuroblastoma and glioblastoma such as glioblastoma in Li-Fraumeni syndrome and sporadic glioblastoma, malignant melanomas, pancreatic ductal adenocarcinomas, myeloid leukemia, acute myelogenous leukemia, myelodysplastic syndrome, myeloproliferative syndrome, gynaecological cancer, Kaposi's sarcoma, Hansen's disease and collagenous colitis.

In a preferred embodiment, the said fluorescent compound is the compound of formula 12.

In a preferred embodiment, the said fluorescent compound is the compound of formula 14.

In a preferred embodiment, said diagnosis is of a metastasis state of cancer.

By "Metastasis", it is meant in the invention the propagation of cancer from the organ where it started to a different organ. It generally occurs through the blood or lymphatic system. When the cancer cells spread and form a new tumour, the latter is called a secondary or metastatic tumour. The cancer cells forming the secondary tumour are like those of the original tumour. If a breast cancer, for example, spreads (metastasizes) to the lung, the secondary tumour is formed of malignant breast cancer cells. The disease in the lung is metastatic breast cancer and not lung cancer.

The invention also relates to a use of a fluorescent compound as defined above, for, with preference *in vitro* and *ex vivo,* diagnosis of a neurodegenerative disease, such as Parkinson and Huntington's chorea, schizophrenia, Niemann-Pick disease or cardiovascular diseases in an individual.

The invention further relates to a method for the *in vitro* and *ex vivo* diagnosis of a cancer in an individual, said method comprising the steps of:

a) labelling a cell membrane or part thereof in a sample of an individual with a fluorescent compound as defined above by carrying out the method of labelling as defined above,

b) determining a generalized polarization parameter GP of said cell membranes or part thereof by the following formula:

$$GP = \frac{I_{gel} - I_{lc}}{I_{gel} + I_{lc}}$$

wherein $I_{gel}$ and $I_{lc}$ are intensities of fluorescence measured by fluorimetry, typically by using a spectrofluorometer or a photomultiplicator, from cell membranes or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,

c) comparing said GP with a reference generalized polarization parameter $GP_R$ of said cell membranes or part thereof, and

d) concluding that said individual is afflicted by a cancer if said GP is lower than said $GP_R$, or
concluding that said individual is not afflicted by a cancer if said GP is equal or higher than said $GP_R$.

In a preferred embodiment, the said fluorescent compound is the compound of formula 12, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

In a preferred embodiment, the said fluorescent compound is the compound of formula 14, wherein at step b) the $I_{gel}$ and $I_{lc}$ are measured after an excitation wavelength at 385 nm, and wherein $I_{gel}$ is the intensity of the wavelength

440 nm and $I_{lc}$ is the intensity of the wavelength 490 nm.

By "sample" it is meant in the invention a biological material isolated from an individual. The biological sample may contain any biological material suitable for labelling lipid membrane. The sample can be isolated from any suitable biological tissue or fluid. Exemplary tissues include tissue obtained from a patient's brain, gastrointestinal tract, lung, pancreas, liver, skin, kidney, eye, heart, blood vessels, lymph nodes, spine, and skeletal or smooth muscle. Exemplary of fluids include blood, blood plasma, serum, urine or cerebral spinal fluid (CSF).

In a preferred embodiment, at step d), it is concluded that said individual is afflicted by a cancer if said GP is lower than at most 0.99x$GP_R$, or

concluding that said individual is not afflicted by a cancer if said GP is equal or higher than said at most 0.99x$GP_R$.

By "at most 0.99" it is meant in the invention 0.99, 0.98, 0.97, 0.96, 0.95, 0.94, 0.93, 0.92, 0.91, 0.90, 0.89, 0.88, 0.87, 0.86, 0.85, 0.84, 0.83, 0.82, 0.81, 0.80, 0.79, 0.78, 0.77, 0.76, 0.75, 0.74, 0.73, 0.72, 0.71, 0.70, 0.69, 0.68, 0.67, 0.66, 0.65, 0.64, 0.63, 0.62, 0.61, 0.60, 0.59, 0.58, 0.57, 0.56, 0.55, 0.54, 0.53, 0.52, 0.51, 0.50, 0.49, 0.48, 0.47, 0.46, 0.45, 0.44, 0.43, 0.42, 0.41, 0.40, 0.39, 0.38, 0.37, 0.36, 0.35, 0.34, 0.33, 0.32, 0.31, 0.30, 0.29, 0.28, 0.27, 0.26, 0.25, 0.24, 0.23, 0.22, 0.21, 0.20, 0.19, 0.18, 0.17, 0.16, 0.15, 0.14, 0.13, 0.12, 0.11, 0.10, 0.9, 0.8, 0.7, 0.6, 0.5, 0.4, 0.3, 0.2 and 0.1.

In a preferred embodiment, the said cell membrane is an exosome, obtained from blood.

The invention also relates to a use of a fluorescent compound as defined above, for *in vitro* and *ex vivo* diagnosis of the response to a therapeutic treatment of a patient afflicted with a cancer, said therapeutic treatment being directed to said cancer.

In a preferred embodiment, the said fluorescent compound is the compound of formula 12.

In a preferred embodiment, the said fluorescent compound is the compound of formula 14.

By "treatment" it is meant in the invention any type of therapy, which aims at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or maybe therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

The invention finally relates to a method for *in vitro* and *ex vivo* evaluation of the response to a therapeutic treatment of a patient afflicted with a cancer, said therapeutic treatment being directed to said cancer, said method comprising the steps of:

a) labelling a cell membrane or part thereof in a sample from a tumour of said patient obtained after said therapeutic treatment, with a fluorescent compound as defined above by carrying out the method of labelling as defined above,

b) determining a generalized polarization parameter GP of said cell membrane or part thereof by the following formula:

$$GP = \frac{I_{gel} - I_{lc}}{I_{gel} + I_{lc}}$$

wherein $I_{gel}$ and $I_{lc}$ are intensities of fluorescence measured by fluorimetry, typically by using a spectrofluorometer or a photomultiplicator, from said cell membrane or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,

c) comparing said GP with a reference generalized polarization parameter $GP_R$ of cell membranes or part thereof, said $GP_R$ being a prior determination of GP according to steps a) and b) from cell membrane or part thereof in a sample from the tumour of said patient obtained before said therapeutic treatment, and

d) concluding that said patient is considered to be responsive to the said therapeutic treatment if GP is higher than $GP_R$, or

concluding that patient is not considered to be responsive to the said therapeutic treatment if GP is lower or equal to $GP_R$.

In a preferred embodiment, the said fluorescent compound is the compound of formula 12, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

In a preferred embodiment, the said fluorescent compound is the compound of formula 14, wherein at step b) the $I_{gel}$ and $I_{lc}$ are measured after an excitation wavelength at 385 nm, and wherein $I_{gel}$ is the intensity of the wavelength 440 nm and $I_{lc}$ is the intensity of the wavelength 490 nm.

In a preferred embodiment, at step d), it is concluded that said patient is considered to be responsive to the said therapeutic treatment if GP is higher than at most $0.99xGP_R$, or concluding that patient is not considered to be responsive to the said therapeutic treatment if GP is equal or higher than said at most $0.99xGP_R$.

By "at most 0.99" it is meant in the invention 0.99, 0.98, 0.97, 0.96, 0.95, 0.94, 0.93, 0.92, 0.91, 0.90, 0.89, 0.88, 0.87, 0.86, 0.85, 0.84, 0.83, 0.82, 0.81, 0.80, 0.79, 0.78, 0.77, 0.76, 0.75, 0.74, 0.73, 0.72, 0.71, 0.70, 0.69, 0.68, 0.67, 0.66, 0.65, 0.64, 0.63, 0.62, 0.61, 0.60, 0.59, 0.58, 0.57, 0.56, 0.55, 0.54, 0.53, 0.52, 0.51, 0.50, 0.49, 0.48, 0.47, 0.46, 0.45, 0.44, 0.43, 0.42, 0.41, 0.40, 0.39, 0.38, 0.37, 0.36, 0.35, 0.34, 0.33, 0.32, 0.31, 0.30, 0.29, 0.28, 0.27, 0.26, 0.25, 0.24, 0.23, 0.22, 0.21, 0.20, 0.19, 0.18, 0.17, 0.16, 0.15, 0.14, 0.13, 0.12, 0.11, 0.10, 0.9, 0.8, 0.7, 0.6, 0.5, 0.4, 0.3, 0.2 and 0.1.

In a preferred embodiment, the said cell membrane is an exosome, obtained from blood.

**Legend to the Figures**

**Figure 1** represents the fluorescence emission spectra (arbitrary unit) of Dioll in comparison with Laurdan and C-Laurdan in organic solvents of increasing polarity. Full lines represent Dioll; dotted lines represent Laurdan; dash lines represent C-Laurdan. A represents the Toluene condition; B represents the Chloroform condition; C represents the DMSO condition; D represents the Ethanol condition.

**Figure 2A** represents the fluorescence emission spectra (arbitrary unit) at excitation wavelength 390 nm of Laurdan probe in DMPC liposomes at different temperatures conditions (y-axis is the normalized fluorescent intensity; x-axis is the emission wavelength in nm). A presents the fluorescence emission spectra at 5°C; B represents the fluorescence emission spectra at 9°C; C represents the fluorescence emission spectra at 13°C; D represents the fluorescence emission spectra at 18°C; E represents the fluorescence emission spectra at 23°C; F represents the fluorescence emission spectra at 28°C; G represents the fluorescence emission spectra at 33°C; H represents the fluorescence emission spectra at 40°C; I represents the fluorescence emission spectra at 55°C; J represents the fluorescence emission spectra at 65°C.

**Figure 2B** represents the fluorescence emission spectra (arbitrary unit) at excitation wavelength 390 nm of C-Laurdan probe in DMPC liposomes at different temperatures conditions (y-axis is the normalized fluorescent intensity; x-axis is the emission wavelength in nm). A presents the fluorescence emission spectra at 5°C; B represents the fluorescence emission spectra at 9°C; C represents the fluorescence emission spectra at 13°C; D represents the fluorescence emission spectra at 18°C; E represents the fluorescence emission spectra at 23°C; F represents the fluorescence emission spectra at 28°C; G represents the fluorescence emission spectra at 33°C; H represents the fluorescence emission spectra at 40°C; I represents the fluorescence emission spectra at 55°C; J represents the fluorescence emission spectra at 65°C.

**Figure 2C** represents the fluorescence emission spectra (arbitrary unit) at excitation wavelength 390 nm of DIOLL probe in DMPC liposomes at different temperatures conditions (y-axis is the normalized fluorescent intensity; x-axis is the emission wavelength in nm). A presents the fluorescence emission spectra at 5°C; B represents the fluorescence emission spectra at 9°C; C represents the fluorescence emission spectra at 13°C; D represents the fluorescence emission spectra at 18°C; E represents the fluorescence emission spectra at 23°C; F represents the fluorescence emission spectra at 28°C; G represents the fluorescence emission spectra at 33°C; H represents the fluorescence emission spectra at 40°C; I represents the fluorescence emission spectra at 55°C; J represents the fluorescence emission spectra at 65°C.

**Figure 2D** represents the GP values (arbitrary unit) of Laurdan (A), C-Laurdan (B) and DIOLL (C) probes as a function of temperature (in °C).

**Figures 3A** and **3B** represent the membrane fluidity estimation with DIOLL probe using fluorescence spectroscopy and flow cytometry. In **Figure 3A,** GP values (y-axis, arbitrary unit) are measured from the spectra of DIOLL inserted in liposomes of increasing fluidity. In **Figure 3B,** the number of events (y-axis) as a function of the ratio 525/450 fluorescence intensity values (x-axis) is measured from liposomes of increasing fluidity. For **Figure 3A** and **3B,** A

represents liposome DSPC; B represents liposome DPPC; C represents liposome DMPC; D represents liposome POPC, E represents liposome DOPC; F represents liposome DLPC.

**Figure 4** represents a fluorescence image of HMEC cells incubated with DIOLL probe. Excitation was performed at 405 nm. This image is an overlay of images recovered at emission 424 nm, 432 nm, 441, 468 nm, 477 nm, 486 nm, 495 nm, 504 nm and 513 nm. In this image, the shades of grey correspond to different fluorescence intensity obtained for each emission which leads to the differentiation of the fluidity state of the cell membranes: light grey corresponds to fluidic membrane while dark grey corresponds to rigid membrane.

**Figure 5** represents the mean GP values (arbitrary unit) obtained for different lipid membranes. The standard deviation is also represented for each mean GP values. Data sets are representative of at least 3 independent measurements. A represents the plasma membrane, B represents the endoplasmic reticulum, C represents mitochondrial membrane and D represents the nuclear membrane.

**Figure 6A** represents the fluorescence emission intensity (arbitrary unit) of DIOLL probe inserted in the lipid bilayer of POPC liposomes as a function of the emission wavelength (in nm). Excitation was performed at 390 nm. 2 $\mu$M of DIOLL probe was added to increasing liposome concentrations. 1 represents 0 $\mu g.ml^{-1}$, 2 represents 5 $\mu g.ml^{-1}$, 3 represents 10 $\mu g.ml^{-1}$, 4 represents 15 $\mu g.ml^{-1}$, 5 represents 20 $\mu g.ml^{-1}$, 6 represents 30 $\mu g.ml^{-1}$, 7 represents 40 $\mu g.ml^{-1}$, 8 represents 60 $\mu g.ml^{-1}$ and 9 represents 80 $\mu g.ml^{-1}$ POPC liposomes.

**Figure 6B** represents the maximum fluorescence intensity (arbitrary unit) at emission at 490 nm as function of lipid concentration ($\mu g.ml^{-1}$) of POPC liposomes labelling by DIOLL probe. Experimental data were fitted to a hyperbolic function: $y=2.8*10^6*x/(27+x)$.

**Figure 6C** represents the surface under the peak of fluorescence (arbitrary unit), calculated from **Figure 6A** curves, as function of the lipid concentration ($\mu g.ml^{-1}$) of POPC liposomes labelling by DIOLL probe. Experimental data were fitted to a hyperbolic function: $y=29625*x/(25+x)$.

**Figure 7A** represents the fluorescence emission intensity (arbitrary unit) of DIOLL probe inserted in the lipid bilayer of DMPC liposomes as a function of the emission wavelength (in nm). Excitation was performed at 390 nm. 2 $\mu$M of DIOLL probe was added to increasing liposome concentrations. 1 represents 0 $\mu g.ml^{-1}$, 2 represents 5 $\mu g.ml^{-1}$, 3 represents 10 $\mu g.ml^{-1}$, 4 represents 15 $\mu g.ml^{-1}$, 5 represents 20 $\mu g.ml^{-1}$, 6 represents 30 $\mu g.ml^{-1}$, 7 represents 40 $\mu g.ml^{-1}$, 8 represents 60 $\mu g.ml^{-1}$ and 9 represents 80 $\mu g.ml^{-1}$ DMPC liposomes.

**Figure 7B** represents the fluorescence emission intensity (arbitrary unit) of DIOLL probe inserted in the lipid bilayer of DPPC liposomes as a function of the emission wavelength (in nm). Excitation was performed at 390 nm. 2 $\mu$M of DIOLL probe was added to increasing liposome concentrations. 1 represents 0 $\mu g.ml^{-1}$, 2 represents 5 $\mu g.ml^{-1}$, 3 represents 10 $\mu g.ml^{-1}$, 4 represents 15 $\mu g.ml^{-1}$, 5 represents 20 $\mu g.ml^{-1}$, 6 represents 30 $\mu g.ml^{-1}$, 7 represents 40 $\mu g.ml^{-1}$, 8 represents 60 $\mu g.ml^{-1}$ and 9 represents 80 $\mu g.ml^{-1}$ DPPC liposomes.

**Figure 7C** represents the surface under the peak of fluorescence (arbitrary unit), calculated from **Figure7A** curves, as a function of the lipid concentration ($\mu g.ml^{-1}$) of DMPC liposomes labelling by DIOLL probe.

**Figure 7D** represents the surface under the peak of fluorescence (arbitrary unit), calculated from **Figure7B** curves, as a function of the lipid concentration ($\mu g.ml^{-1}$) of DPPC liposomes labelling by DIOLL probe.

**Figure 7E** represents the cross calculations of concentrations ($\mu g.ml^{-1}$) of lipids in POPC liposomes (1), in DMPC liposomes (2) and DPPC liposomes (3) using POPC (white bars), DMPC (light grey), DMPC (grey) standard solutions or by Stewart method (black bars). Error bars represent standard deviation (SD) obtained with 3 independent measurements.

**Figure 8** represents the cholesterol effect on liposome quantification with DIOLL. Quantification of POPC-cholesterol liposomes ($\mu g.ml^{-1}$) is performed using POPC liposomes as standard (white bars): 10 (x-axis) represents POPC-cholesterol liposomes with 10 mass % of cholesterol; 20 (x-axis) represents POPC-cholesterol liposomes with 20 mass % of cholesterol; 30 (x-axis) represents POPC-cholesterol liposomes with 30 mass % of cholesterol. Phospholipid content for each POPC-cholesterol liposome was determined by Stewart method (black bars). Dotted bars represent total lipid content (phospholipid as determined by Stewart method and 10, 20 or 30 % cholesterol) for each POPC-cholesterol liposome. Error bars are SD obtained with 3 independent measurements.

**Figure 9** represents the charge effect on liposome quantification with DIOLL probe. Quantification of POPC- POPS (1:1 molar ratio) liposomes ($\mu g.ml^{-1}$) is performed using POPC liposomes as standard (white bars) and by Steward method (black bars). Error bars are SD obtained with 3 independent measurements.

**Figure 10** represents the quantification of exosomes. The exosome preparation was quantified ($\mu g.ml^{-1}$) using DIOLL probe and POPC liposomes as standard (white bars). Lipids were extracted with chloroform and quantified with Steward method (black bars). Error bars are SD obtained with 3 independent measurements.

**Figure 11** represents the quantification of GPMV. The GPMV preparation was quantified ($\mu g.ml^{-1}$) using DIOLL probe and POPC liposomes as standard (white bars). Lipids were extracted with chloroform and quantified with Steward Method (black bars). Error bars are SD obtained with 3 independent measurements.

**Figure 12A** represents the effect of temperature on fluorescence emission of Dioll-NE. Fluorescence emission spectra at excitation wavelength 390 nm of Dioll-NE is measured at different temperatures conditions (y-axis is the

fluorescent intensity in arbitrary unit; x-axis is the emission wavelength in nm). A presents the fluorescence emission spectra at 5°C; B represents the fluorescence emission spectra at 7°C; C represents the fluorescence emission spectra at 9°C; D represents the fluorescence emission spectra at 11°C; E represents the fluorescence emission spectra at 13°C; F represents the fluorescence emission spectra at 15°C; G represents the fluorescence emission spectra at 17°C; H represents the fluorescence emission spectra at 19°C; I represents the fluorescence emission spectra at 21°C; J represents the fluorescence emission spectra at 23°C. K represents the fluorescence emission spectra at 25°C. L represents the fluorescence emission spectra at 27°C. M represents the fluorescence emission spectra at 37°C.

**Figure 12B** represents the Generalized Polarization (GP) of DIOLL probe inserted in Dioll-NE as a function of temperature.

**Figure 13** represents the genetic plasticity as a function of epithelial character for different prostate and mammary cell lines wherein A represents claudin-low cell lines, B represents luminal B cell lines, C represents luminal A cell lines, and D represents her2 cell lines and E represents basal cell lines.

**Figure 14** represents the Generalized Polarization (GP) (arbitrary unit) of DIOLL probe inserted in the lipid membrane of tumorous cell lines as a function of the temperature at which the $I_{440}$ and $I_{490}$ were recorded. Black scare represents MCF 7 cell line. Black spheres represent ZR75 cell line. Black triangles represent MDA MB 436 cell line. Black diamonds represent HS578T cell line. Grey squares represent MDA MB 231 cell line. Grey triangles represent HCC1954 cell line. Grey spheres represent BT20 cell line.

**Figure 15** represents the Generalized Polarization (GP) (arbitrary unit) of DIOLL probe inserted in the lipid membrane of basal and mesenchymal mammary gland cell lines as a function of the temperature at which the $I_{440}$ and $I_{490}$ were recorded. Black spheres represent HCC 1954 mesenchymal cell line. Black triangles represent HCC 1937 mesenchymal cell line. Grey diamonds represent HCC 1954 basal cell line. Grey squares represent HCC 1937 basal cell line. **Figure 15**

**Figures 16A** and **16B** represent the distinguishing of differentiated HMEC and dedifferentiated HMEC with DIOLL probe using fluorescence spectroscopy and flow cytometry, respectively. In **Figure 16A,** the Generalized Polarization (GP) (arbitrary unit) of DIOLL probe inserted in the lipid membrane of HMEC cells is represented as a function of the temperature at which the $I_{440}$ and $I_{490}$ were recorded. Black spheres represent differentiated HMEC cells. Grey spheres represent dedifferentiated HMEC cells. Error bars are SD obtained with 3 independent measurements. In **Figure 16B,** the number of events (y-axis) as a function of the ratio 525/450 fluorescence intensity values (x-axis) is measured from differentiated HMEC (1) and dedifferentiated HMEC (2).

**Figure 17** represents the Generalized Polarization (GP) (arbitrary unit) of DIOLL probe inserted in the lipid membrane of several prostate cell lines as a function of the temperature at which the $I_{440}$ and $I_{490}$ were recorded. Black squares represent C4-2B mesenchymal cell line. Black spheres represent LNCaP cell line. Grey triangles represent WPMY-1 cell line. Grey diamonds represent PC-3 cell line.

**Figures 18A** and **18B** represent the distinguishing of exosomes from differentiated HMEC and dedifferentiated HMEC with DIOLL probe using fluorescence spectroscopy and flow cytometry, respectively. In **Figure 18A,** the Generalized Polarization (GP) (arbitrary unit) of DIOLL probe inserted in the lipid membrane of exosomes from HMEC cells is represented as a function of the temperature at which the $I_{440}$ and $I_{490}$ were recorded. Black spheres represent exosomes from differentiated HMEC cells. Grey spheres represent exosomes from dedifferentiated HMEC cells. In **Figure 18B,** the number of events (y-axis) as a function of the ratio 525/450 fluorescence intensity values (x-axis) is measured from exosomes from differentiated HMEC (1) and exosomes from dedifferentiated HMEC (2).

**Figures 19A** and **19B** represent the distinguishing of exosomes from HMEC cells mediated from 0 to 12 days by ZEB1 transcription factor, with DIOLL probe using fluorescence spectroscopy and flow cytometry, respectively. In **Figure 19A,** the Generalized Polarization (GP) (arbitrary unit) of DIOLL probe inserted in the lipid membrane of exosomes from the mediated HMEC cells is represented as a function of the temperature at which the $I_{440}$ and $I_{490}$ were recorded. Black spheres represent exosomes from original HMEC cells. Black squares represent exosomes from HMEC cells mediated for 3 days. Black triangles exosomes from HMEC cells mediated for 7 days. Grey spheres represent exosomes from HMEC cells mediated for 10 days. Grey squares with a black star represent exosomes from HMEC cells mediated for 12 days. In **Figure 19B,** the number of events (y-axis) as a function of the ratio 525/450 fluorescence intensity values (x-axis) is measured for exosomes from original HMEC cells (1), exosomes from HMEC cells mediated for 3 days (2), exosomes from HMEC cells mediated for 7 days (3), exosomes from HMEC cells mediated for 10 days (4) and exosomes from HMEC cells mediated for 12 days (5). For more readability, the origin point of y-axis is moved for each condition 1 to 5.

## EXAMPLES

### Example 1: Synthesis of DIOLL probe

### 1. Chemicals and reagents

Lauroylchloride and nitrobenzene were purchased from Acros. 2-bromonaphthalene, aluminium chloride, N-methylallylamine, 2-dicyclohexylphosphino2'4'6'triisopropylbiphenyl (X-Phos), Palladium acetate (Pd (Ac)2), N-methylmorpholine oxide monohydrate, catalytic OsO4 2.5% solution in tert-butanol and anhydrous dioxane were purchased from Sigma-Aldrich. Potassium tert-butanolate was purchased from Lancaster. Nitrobenzene and anhydrous dioxane were used without further purification. Flash chromatographies were performed on silica gel purchased from Acros Organics.

### 2. Synthesis

Reactions were carried out in dried glassware and under argon atmosphere. All reactions were magnetically stirred and monitored by thin-layer chromatography (TLC) on silica gel 60 F254 (Merck) plates using UV light to visualize the compounds. Yields refer to chromatographically and spectroscopically pure compounds unless otherwise noted. $^1H$ and $^{13}C$ NMR spectra were recorded at 23 °C using Bruker Avance DRX300, DRX400 or DRX500. Chemical shifts are reported relative to the residue peaks of the solvent (CDCl3: 7.26 ppm for $^1H$ and 77.16 ppm for $^{13}C$). The following abbreviations are used to denote the multiplicities: s = singlet, d = doublet, dd = doublet of doublets, t = triplet, m = multiplet and br s = broad singlet. NMR solvents were purchased from Euriso-Top (Saint Aubin, France). Low resolution mass spectra (ESI) and HRMS were recorded in positive ion mode using a Thermo Finnigan LCQ spectrometer.

a. Synthesis of 1-(6-bromonaphthalen-2-yl) dodecan-1-one

2-bromonaphthalene (2g, 9.7 mmol) was dissolved in 9.6 mL of nitrobenzene under an Argon atmosphere. The solution was stirred in an ice bath. Lauroylchloride (2.8 mL, 11.64 mmol, 1.2 equiv.) was added drop wise. The reaction mixture was treated with aluminium chloride (1.4 g, 10.67 mmol, 1.1 equiv.) and stirred at room temperature for at least 18 hours. The reaction mixture was quenched with distilled water and extracted three times with ethylacetate. The organic phases were combined, washed with brine, dried over sodium sulphate and concentrated under reduced pressure. The product was purified by crystallization from cold ethanol to obtain a yellowish powder; yield 1.6305 g (43.4%).
$^1H$ NMR (300 MHz, $CDCl_3$): $\delta$ = 0.88 (t, 3H, J=6.6 Hz, $CH_3$) ; 1.26-1.37 (m, 16H, $CH_2$) ; 1.79 (m, 2H, $CH_2$) ; 3.07 (t, 2H, J=7.5 Hz, $CH_2CO$) ; 7.62 (d, 1H, J=8.7 Hz, H ar); 7.81 (m, 2H, H ar); 8.05 (m, 2H, H ar); 8.42 (s, 1H, H ar).
$^{13}C$ NMR: $\delta$ = 14.1 ; 22.7 ; 24.5; 29.4 ; 29.5; 29.6 ; 29.7 ; 31.9 ; 38.8 ; 122.7 ; 125.2 ; 127.5 ; 129.4 ; 129.9 ; 130.2 ; 131.0 ; 134.8 ; 136.4 ; 200.2.
HRMS: m/z ($MH^+$) $C_{22}H_{30}BrO$ calculated: 389.1475, measured: 389.1471.

b. Synthesis of 1-(6-(allyl(methyl)amino)naphthalen-2-yl)dodecan-1-one (NMAAL)

An oven-dried Schlenk flask was evacuated and backfilled with argon. The flask was charged with 1-(6-bromonaphthalen-2-yl) dodecan-1-one (200 mg, 0.514 mmol); ($Pd (Ac)_2$) (9 mg, 41.1 $\mu$mol, 0.08 equiv.); X-Phos (39 mg, 82.2 $\mu$mol, 0.16 equiv.) and cesium carbonate (503 mg, 1.54 mmol, 3 equiv.). The Schlenk flask was capped with a rubber septum, and then evacuated and backfilled with argon three times. N-methylallylamine (64 $\mu$L, 0.668 mmol, 1.3 equiv.) and anhydrous dioxane (1.5 mL) were added through the septum. The mixture was heated to 100°C in an oil bath and stirred for 15 hours. The mixture was cooled down to room temperature, diluted with ethylacetate, filtered through Celite and concentrated under reduce pressure to obtain a brown solid product. The crude product was purified by flash chromatography on silica gel eluting with ethylacetate/petroleum ether = 3/97. A yellow powder (51.5 mg, yield 21%) was obtained. The final product is pure at 80%.
$^1H$ NMR (300 MHz, $CDCl_3$): $\delta$ = 0.88 (t, 3H, J=5.1 Hz, $CH_3$) ; 1.25-1.37 (m, 16H, $CH_2$) ; 1.77 (m, 2H, $CH_2$) ; 3.03 (t, 2H, J=7.7 Hz, $CH_2CO$) ; 3.10 (s, 3H, $NCH_3$) ; 4.07 (d, 2H, J=3.6 Hz, $CH_2N$) ; 5.17-5.22 (m, 2H, $CH_2$=CH) ; 5.89 (m, 1H, *CH*=$CH_2$) ; 6.96 (br s, 1H, H ar) ; 7.16 (dd, 1H, J= 6.6 Hz, J=1.2 Hz, H ar); 7.64 (d, 1H, J=6.6 Hz, H ar); 7.80 (d, 1H, J=6.9 Hz, H ar); 7.93 (dd, 1H, J= 6.3 Hz, J=1.2 Hz, H ar); 8.32 (s, 1H, H ar).
$^{13}C$ NMR: $\delta$ =14.1 ; 22.7 ; 24.9 ; 29.3 ; 29.5 ; 29.6 ; 29.7 ; 31.9 ; 38.3 ; 38.4 ; 55.1 ,116.2 ; 124.7 ; 126.1 ; 129.7 ; 130.7 ; 133.0 ; 137.6 ; 200.2.
HRMS: m/z ($MH^+$) $C_{26}H_{38}NO$ calculated: 380.2948, measured: 380.2938.

c. Synthesis of 1-(6-((2,3-dihydroxypropyl)(methyl)amino)naphthalen-2-yl)dodecan-1-one (DIOLL probe, compound of formula 12)

The NMAAL (80 % purity) product is disposed in 25 mL glassware. 5 mL of acetone, 2 mL of water, 141 mg of N-methylmorpholine oxide monohydrate and 50 $\mu$L of catalytic $OSO_4$ (100 $\mu$L of a 2.5% solution in *tert*-butanol) are added and the reaction is stirred overnight at room temperature. The mixture is then diluted in ethylacetate, extracted with water (2x30 mL) and washed with brine (30 mL). The organic phase is dried over magnesium sulphate and concentrated under reduce pressure to obtain a crude product purified by flash chromatography on silica gel. Elution is carried first with 100 mL of ethylacetate/petroleum ether (10/90) to eliminate contaminants, then with 100 mL ethylacetate/petroleum ether (50/50) to eliminate intermediate products of reaction and finally with 100 mL of ethylacetate 100% for the recuperation of a dark green pure final product (18.7 mg, yield 41.7 %).
$^1$H NMR (500 MHz, $CDCl_3$): $\delta$ = 0.88 (t, 3H, J=7.0 Hz, $CH_3$); 1.25-1.41 (m, 16H, $CH_2$) ; 1.77 (m, 2H, $CH_2$); 3.03 (t, 2H, J=7.5 Hz, $CH_2CO$); 3.14 (s, 3H, $CH_3N$); 3.51 (dd, 1H, J=15 Hz, J=5.5 Hz); 3.61 (m, 2H); 3.82 (dd, 1H, J=11.5 Hz, J=3.5 Hz); 4.11 (m, 1H, CHO); 6.95 (br s, 1H, H ar) ; 7.22 (dd, 1H, J= 9.5 Hz, J=2.5 Hz, H ar) ; 7.63 (d, 1H, J=8.5 Hz, H ar) ; 7.80 (d, 1H, J=9 Hz, H ar); 7.93 (dd, 1H, J= 8.5 Hz, J=1.5 Hz, H ar); 8.32 (s, 1H, H ar).
$^{13}$C NMR: $\delta$ = 14.1 ; 22.7 ; 24.8 ; 29.4 ; 29.5 ; 29.6 ; 29.7 ; 31.9 ; 38.4 ; 39.5 ; 55.3 ; 64.2 ; 69.8 ; 105.9 ; 116.5 ; 124.8 ; 125.5 ; 126.3 ; 129.7 ; 130.9 ; 131.1 ; 137.5 ; 149.5 ; 200.3.
HRMS: m/z ($MH^+$) $C_{26}H_{40}NO_3$ calculated: 414.3003, measured: 414.2995.

**Example 2: Labelling lipid membrane with DIOLL probe**

**1. Material and method**

a. Chemicals

DIOLL probe was synthesis as described in Example 1.

Laurdan was purchased from GE healthsicences and C-Laurdan was synthesized as described below.

Lipids were purchased from Avanti Polar Lipids (Alabaster, Al). PBS was purchased from Sigma-Aldrich. Ultrapure water was obtained with a PureLabOption Q7 system (Veolia Water STI, Decines, France).

Fetal bovine serum (FBS) was purchased from Eurobio (ref: CVFSVF 00-01), Dulbecco's Modified Eagle's Medium/ Nutrient Mixture F-12 (DMEM/F-12) nutrient mixture (Ham) (ref: 31331-028) and penicillin-streptomycin were purchased from Gibco, human epidermal growth factor (EGF) was purchased from Promocell and insulin was purchased from NovoRapid.

Dil is purchased from life technologies (ref: M7513), Er-tracker is purchased from life technologies, Mito tracker is purchased from life technologies.

b. C-Laurdan synthesis

i. Synthesis of 1-(6-Bromonaphthalen-2-yl)dodecan-1-one and 1-(6-chloronaphthalen-2-yl)dodecan-1-one

2-Bromonaphthalene (4 g, 19.3 mmol) was dissolved in 20 mL of nitrobenzene under an argon atmosphere. The solution was stirred in an ice bath. Aluminium chloride (2.836 g, 21.23 mmol, 1.1 equiv.) was added by portions and lauroyl chloride (5.036 mL, 23.16 mmol, 1.2 equiv.) was added drop- wise. The reaction mixture was stirred at room temperature for 18 hours. The reaction mixture was quenched with distilled water and extracted three times with ethyl acetate. The organic phases were combined, washed with brine, dried over sodium sulfate and concentrated under reduced pressure. The product was purified by crystallization from cold ethanol to obtain a white powder; yield 4.215 g (56%).
$^1$H NMR (300 MHz, CDCl3): $\delta$ = 0.88 (t, 3H, J=6.6 Hz, $CH_3$); 1.26-1.37 (m, 16H, $CH_2$); 1.79 (m, 2H, $CH_2$); 3.07 (t, 2H, J=7.5 Hz, $CH_2CO$); 7.62 (d, 1H, J=8.7 Hz, H ar); 7.81 (m, 2H, H ar); 8.05 (m, 2H, H ar); 8.42 (s, 1H, H ar). $^{13}$C NMR: $\delta$ = 14.1; 22.7; 24.5; 29.4; 29.5; 29.6; 29.7; 31.9; 38.8; 122.7; 125.2; 127.5; 129.4; 129.9; 130.2; 131.0; 134.8; 136.4; 200.2. HRMS: m/z ($MH^+$) $C_{22}H_{30}BrO$ calculated: 389.1475, found: 389.1471.

2-Chloronaphthalene (1 g, 6.1 mmol) was dissolved in 6 mL of nitrobenzene under an argon atmosphere. The solution was stirred in an ice bath. Lauroylchloride (1.7 mL, 7.32 mmol, 1.2 equiv.) was added drop wise. The reaction mixture was treated with aluminium chloride (854 mg, 6.71 mmol, 1.1 equiv.) and stirred at room temperature for at least 18 hours. The reaction mixture was quenched with distilled water and extracted three times with ethylacetate. The organic phases were combined, washed with brine, dried over sodium sulphate and concentrated under reduced pressure. The product was purified by crystallization from cold ethanol to obtain a yellow powder; yield 1.06 g (50%).
$^1$H NMR (300 MHz, CDCl3): $\delta$ = 0.88 (t, 3H, J=6.9 Hz, $CH_3$); 1.26-1.41 (m, 16H, $CH_2$); 1.79 (m, 2H, $CH_2$); 3.07 (t, 2H,

J=7.5 Hz, CH2CO); 7.49 (dd, 1H, J=8.7 Hz, J=2.1 Hz, H ar); 7.79-7.91 (m, 3H, H ar); 8.05 (dd, 1H, J=8.7 Hz, J=1.8 Hz, H ar); 8.43 (br s, 1H, H ar). $^{13}$C NMR: $\delta$ = 14.1; 22.7; 24.5; 29.3; 29.4; 29.5; 29.6; 31.9; 38.7; 125.1; 126.6; 127.6; 127.7; 129.3; 130.8; 131.0; 134.3; 134.6; 136.0; 200.2. MS: m/z (MNa+) = 367.2; HRMS: m/z ($MH^+$) $C_{22}H_{30}ClO$ calculated: 345.1980, measured: 345.1973.

ii. Synthesis of Ethyl 2[(6-dodecanoylnaphthalen-2-yl)methylamino]acetate

An oven-dried Schlenk flask was evacuated and backfilled with argon. The flask was charged with 1-(6-bromonaphthalen-2-yl) dodecan-1-one (1 g, 2.57 mmol), $Pd(OAc)_2$ (46 mg, 206 mmol, 0.08 equiv.), X-Phos (196 mg, 411 mmol, 0.16 equiv.), cesium carbonate (3.35 g, 10.28 mmol, 4 equiv.), and sarcosine ethyl ester (790 mg, 5.14 mmol, 2 equiv.). The Schlenk flask was capped with a rubber septum, then evacuated and backfilled with argon three times. Anhydrous dioxane (7.5 mL) was added through the septum. The mixture was heated to 100 °C in an oil bath and was stirred for 18 hours. The mixture was cooled to room temperature, diluted with ethyl acetate, filtered through Celite, and concentrated under reduced pressure to obtain a yellow solid. The crude product was purified by flash chromatography on silica gel eluting with ethyl acetate/petroleum ether (10/90) to afford a yellow powder (569 mg, yield 52%).
$_1$H NMR (300 MHz, CDCl3): $\delta$ = 0.88 (t, 3H, J=7.2 Hz, CH3); 1.21-1.36 (m, 16H, CH2); 1.76 (m, 2H, $CH_2$); 3.00 (t, 2H, J =7.5 Hz, CH2CO); 3.16 (s, 3H, $CH_3N$); 4.16 (s, 2H, $COCH_2N$); 4.18 (q, 2H, J =7.2 Hz, $CH_3CH_2O$); 6.86 (d, 1H, J =2.4 Hz, H ar); 7.06 (dd, 1H, J =9.3 Hz, J = 2.7 Hz, H ar); 7.62 (d, 1H, J = 8.7 Hz, H ar); 7.78 (d, 1H, J = 9.0 Hz, H ar); 7.92 (dd, 1H, J = 8.7 Hz, J = 1.8 Hz, H ar); 8.31 (br s, 1H, H ar). $^{13}$C NMR: $\delta$ =14.1; 14.2; 22.7; 24.8; 29.3; 29.5; 29.6; 29.7; 31.9; 38.4; 39.8; 54.4; 61.2; 106.0; 114.6; 115.7; 124.7; 125.6; 126.4; 129.6; 130.8; 131.1; 137.4; 148.6; 170.4; 200.2. MS: m/z ($MH^+$) = 426.3. HRMS: m/z ($MH^+$) $C_{27}H_{40}NO_3$ calculated: 426.3003, found: 426.2988.

iii. Synthesis of 6-Dodecanoyl-2-[N-methyl-N-(carboxymethyl)amino] naphthalene (C-Laurdan)

211 mg of ethyl 2[(6-dodecanoylnaphthalen-2-yl)methylamino] acetate was dissolved in 7 mL of a freshly prepared solution of potassium hydroxide (0.25 M) in ethanol. The reaction mixture was stirred for 2 days at room temperature. The solution was acidified with HCl (1 M) until pH 1 and was extracted with ethyl acetate (3 x 20 mL). The ethyl acetate layers were combined, dried over magnesium sulfate and concentrated under reduced pressure to obtain a brown solid which was purified by crystallization from chloroform/petroleum ether to provide 164 mg (83% yield) of C-Laurdan. $^1$H and $^{13}$C NMR and HRMS data of the product are identical with the published data (H. M. Kim, H. J. Choo, S. Y. Jung, Y. G. Ko, W. H. Park, S. J. Jeon, C. H. Kim, T. Joo and B. R. Cho, ChemBioChem, 2007, 8, 553-559).

c. Probe fluorescence in organic solvents

Fluorescence measurements were performed with a Hitachi F4500 fluorometer. Excitation and emission slits were fixed at 5 nm and spectra were recorded at 25°C using a 1 cm path length thermostated quartz micro-cuvette. 0.26 mM solutions of the probes were prepared in a dichloromethane: methanol mixture (3: 1 volume ratio). Measurements were performed with 2.6 mM probes diluted in the different solvents (see **Table 1**). The initial solvent of the stock solution thus represents 1% of the final volume.

d. Probe fluorescence in liposomes

Liposomes were prepared in PBS buffer using a classical freeze/though protocol. In brief, lipids dissolved in chloroform were dried under vacuum. The lipid film formed was rehydrated with the PBS solution and agitated until the complete suspension of the lipid film. The vesicle solution was then submitted to 6 freeze/thaw (5 min in liquid nitrogen/ 10 min in 37°C water bath) cycles and then extruded 19 times sequentially trough 400 nm and 200 nm polycarbonate membranes (Avanti Polar Lipids Liposome Extruder). The 200 nm liposome suspension was stored at 4°C.
Fluorescence data was obtained using a Hitachi F4500 fluorometer. Emission spectra were recorded between 420 and 600 nm with an excitation wavelength fixed at 385 nm and 5 nm excitation and emission slits. Ethanol solutions of fluorescent probes were prepared and added to liposomes at a 1: 400 (probe: phospholipid) molar ratio. Liposomes were used at a 40 $\mu$g.mL$^{-1}$ final concentration. Measurements were done at 21°C for all liposome compositions indicated in **Table 2,** except for DMPC where a range of temperature from 5 to 65°C was also applied.

e. Flow cytometry

Fluorescence measurements were performed with an LSR Fortessa. Cells passing in front of the laser were exited with a laser of 405 nm and the filters 450/50 and 525/50 were used to respectively observed the rigid state intensity and fluid state intensity. Like the spectrofluorometer, Ethanol solutions of Dioll were prepared and added to liposomes

at a 1: 400 molar ratio. Liposomes were used at a 40 μg.mL$^{-1}$ final concentration. Measurements were done at 21°C for all liposome composition indicated in **Table 2.** Data processing was effectuated on FlowJo.

f. Cell culture

Human mammary epithelial cells (HMECs) were provided by Lonza. HMEC were cultured in 1:1 Dulbecco's Modified Eagle's Medium (DMEM)/HAMF12 medium (Gibco) complemented with 10% FBS (Gibco), 91 U.mL$^{-1}$ penicillin-streptomycin (Gibco), 10 ng.mL$^{-1}$ human epidermal growth factor (EGF) (Promocell), 262 mU.mL$^{-1}$ insulin (NovoRapid) and 0,5 μg.ml$^{-1}$ hydrocortisone (Sigma) under a humidified atmosphere of 5% CO2 at 37 °C. Cell morphology and growth curve were routinely checked.

g. Confocal microscopy experiments

Cells were cultured in Lab-TEK® at 30% of confluence and Dioll was added to obtain 0.5 μM 48 hours before observation.
48 hours later cells were at 80 % of confluence and ready for observation.
Specific organelle markers (plasma membrane (Dil), endoplasmic reticulum (ER tracker), mitochondria (Mito tracker)) were added 20 minutes before observation. Nuclear stain (Hoechst) was injected automatically in the chamber after dioll fluorescence recording.
The images were taken using a Zeiss LSM 880 AxioObserver inverted spectral confocal microscope. This is a spectral confocal microscope on stand with incubation chamber with environmental control temperature, $CO_2$ and hygrometry. The light source is a laser diode. The objective is an alpha Plan-Apochromat 63x/1.46 Oil DIC M27.
For colocalisation experiments, Dioll excitation wavelength was 405 nm and the emission wavelength was a band from 424nm to 513nm. For spectral mode acquisition excitation wavelength was 405 nm and images were taken at 424, 432, 441, 450, 459, 468, 477, 486, 495, 504, 513 nm emission wavelength.
For ER-tracker and Mito-tracker, the excitation wavelength was 561 nm and detection wavelength was 611 nm.
For Dil, the excitation wavelength was 561 nm and emission wavelength was 621 nm.
For Hoechst, the excitation wavelength was 405nm and emission wavelength was 430nm

h. Determination of the GP parameter from the images obtained bv confocal microscopy

A composite image was created from the image obtained with the Dioll and the image obtained with one of the specific subcellular markers (Dil, ER-tracker, Mito tracker) to precisely identify areas were the probed colocalized. The emission intensity values in the 440 and 490 nm channels were measured using ImageJ after background correction for the areas identified. GP values were calculated with the same formula as for spectroscopy experiments:

$$GP = \frac{I_{440} - I_{490}}{I_{440} + I_{490}}$$

where $I_{440}$ and $I_{490}$ are recorded fluorescence intensities at wavelengths of 440 nm and 490 nm, respectively.
GP values were calculated from at least 5 independent measurements.

**2. Results**

a. Spectroscopic properties of DIOLL probe: a comparison with Laurdan and C-Laurdan probes in organic solvents of increasing polarity

The spectroscopic properties of DIOLL probe were compared to the one of Laurdan and C-Laurdan in organic solution (**Figure 1**). As shown in **Figure 1**, Laurdan sensitivity to the polarity of its environment translates itself in a shift of the fluorescence emission maximum from lower wavelength for non-polar solvents (423 nm for toluene, 440 nm for chloroform) to higher wavelength for polar solvents (460 nm for DMSO, 485 nm for ethanol) (**Table 1**). This effect, named bathochromic effect, is at the basis of the probe's use in the sensing of the order state of the membranes.
Therefore, the same effect is searched for when alternative fluorophores are proposed: C-Laurdan and DIOLL probe perfectly respond to this requirement as shifts in the maximum emission wavelength are recorded with the increasing solvent polarity (416 nm and 425 nm in toluene, 432 and 441 nm in chloroform, 450 and 460 nm in DMSO and 485 nm and 486 nm in ethanol, for C-Laurdan and DIOLL probe, respectively).

**Table 1:** Maximum emission wavelength of Laurdan, C-Laurdan and DIOLL probe in organic solvents of increasing polarity.

| Solvent | Probe | Toluene | Chloroform | DMSO | Ethanol |
|---|---|---|---|---|---|
| λ emission maximum (nm) | Laurdan | 423 | 440 | 460 | 485 |
| | C-Laurdan | 416 | 432 | 450 | 485 |
| | DIOLL | 424 | 424 | 441 | 486 |
| Maximum fluorescence intensity (A.U.) | Laurdan | 3814 | 4441 | 5533 | 4221 |
| | C-Laurdan | 359 | 3115 | 2428 | 1491 |
| | **DIOLL** | **5041** | **5041** | **9384** | **7647** |

Fluorescence intensity is however very different for the two Laurdan analogues, with Dioll remarkably showing a high fluorescence intensity, going from 1.5 to 2 fold higher than for Laurdan whatever the solvent used. Inversely, C-Laurdan shows very low fluorescence intensities: 1.3 to 10 times lower than that of Laurdan (hypochromic effect). The good fluorescence yield recorded for DIOLL probe makes it a good candidate for microscopy and spectroscopy applications.

b. Fluorescence of DIOLL probe in DMPC liposomes

To check whether these promising fluorescence properties may serve for membrane state characterization, the inventors compared fluorescence properties of DIOLL probe with those of Laurdan and C-Laurdan in liposomes made of 1,2-dimyristoyl-sn-glycero-3-phosphocholine (DMPC). This lipid is known to present a gel to liquid disordered phase transition at 24°C that makes it a good model system to evaluate fluorophore capacity to evidence membrane fluidity changes, as PC phosphocholine is the main phospholipid in cell membranes.

The fluorescence spectrum of Laurdan inserted in DMPC liposomes evolved when temperature increased from 5°C to 65°C **(Figure 2A)**. At temperatures lower than the phase transition temperature the fluorescence spectrum shows a maximum at 440 nm as expected for a gel phase. Above 24°C, the intensity at 440 decreases in favour of an increase in intensity around 490 nm. The decrease in 440 nm emission intensity becomes more pronounced with temperature, i.e. with the amount of lipid molecules in a disordered state. The 490 nm contribution becomes predominant at temperatures above 28°C. This behavior is characteristic for Laurdan and has been described in numerous reports (Parasassi et al., Biophysical Journal Volume 66 January 1994 120-132; Parasassi and Gratton, Journal of Fluorescence, Vol. 5, No. 1, 1995).

C-Laurdan in DMPC also shifts towards higher wavelength, but an overall decrease in the fluorescence intensity is recorded with the increase in temperature **(Figure 2B).**

Dioll has a behavior similar to Laurdan, with a shift in the maximum emission wavelength from 440 nm for the gel phase to 490 nm in the liquid crystalline phase **(Figure 3C).** Moreover, the decrease in the fluorescence intensity between the gel and the fluid phase is more limited than for Laurdan with a gel over liquid crystalline ratio of 0.8 vs 0.6 for Laurdan.

Most of the commonly used fluorophores show better fluorescence intensity in the rigid (apolar) conditions, making thus the disordered contribution of the membrane underestimated. The good fluorescence yield of DIOLL probe in the disordered state, comparable with that obtained in the ordered state, opens the possibility of a more accurate quantification of the fluidity state of the membrane with respect to fluorophores commercially available to date.

The spectral shift described above was quantified by calculation of the Generalized Polarization (GP) parameter. This parameter provides a quantitative index of membrane fluidity and is calculated as the difference between fluorescence emission intensity at a 440 nm (lipids with gel properties) and the intensity at 490 nm (wavelength evidenced in lipids with liquid crystal properties), divided by the sum of these two intensities.

The GP parameter was thus calculated from fluorescence emission intensities according to the following formula:

$$GP = \frac{I_{gel} - I_{lc}}{I_{gel} + I_{lc}}$$

where $I_{gel}$ and $I_{lc}$ are, respectively, intensities of fluorescence at wavelengths characteristic of probe emission, respectively in gel and liquid crystal lipid phases.

Graphs of GP evolution as a function of temperature are plotted in **Figure 2D.** One can notice that Laurdan and DIOLL probes have a similar evolution. At a temperature lower than 20° GP values are rather high, which is expected for lipids in a gel phase. GP values decrease with the increase in temperature, with a rather sharp drop at 21°C, where the liquid crystal phase starts to appear. The decrease in GP is rather sharp as phase transition is a cooperative phenomenon and once lipid chains began to adopt disordered conformation the process is amplified (less energy is required to induce melting of the remaining chains). First derivatives of the GP function vs temperature show an inflexion point at 23°C for Laurdan and Dioll, which is in line with previously reported values of melting temperature (Tm). At this point, 50 % of lipid chains are in liquid crystalline state. For both Dioll and Laurdan, above the transition temperature, the GP values continue to decrease, also less sharply than around the phase transition temperature. The two fluorophores are sensitive to the mobility of water molecules in the vicinity of the excited naphthalene moiety and, although lipids are in a liquid crystal phase, the solvent mobility at the polar/non-polar interface continues to increase. This is of interest as, theoretically, GP values would permit to discriminate membrane fluidity far beyond the phase transition temperature. C-Laurdan also shows a decrease in GP values with temperature, but the amplitude of this decrease was lower than for Laurdan and Dioll and GP did not evolve above the phase transition temperature.

In conclusion, Dioll and Laurdan show a large difference in GP values between the ordered/gel and disordered/liquid crystalline states. This property is of importance for microscopy use where a clear-cut separation of fluorescence coming from gel and liquid crystalline phases is required for visualization of membranes in different states.

c. Degree of order quantification in liposomes bv fluorescence spectroscopy and flow cytometry using DIOLL

As membrane fluidity state is determined by lipid acyl chain length and unsaturation, the inventors checked whether Dioll was equally able to render variations in membrane state depending on lipid characteristics. Therefore, Dioll was inserted in liposomes constituted of phosphatidylcholines with different melting temperatures, dictated by different chain length and unsaturation (ranging from 14 to 18 carbons for the chain length and from 0 to 2 for the chain unsaturation, respectively; **Table 2**).

Fluorescence spectra were registered and GP parameter was measured at 21 °C.

**Table 2:** Liposome preparations used and corresponding phase transition temperatures (Tm).

| Liposome preparation | Lipid | Acyl chain composition | Schematic structure | Tm |
|---|---|---|---|---|
| DLPC | 1,2-dilinoleoyl-sn-glycero-3-phosphocholine | 18:2 ($\Delta^{9,12}$-Cis) PC | 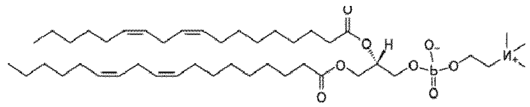 | -57°C |
| DOPC | 1,2-dioleoyl-sn-glycero-3-phosphocholine | 18:1 ($\Delta^{9}$-Cis) PC | 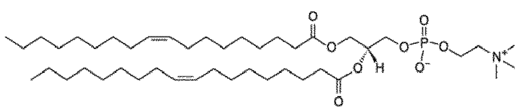 | -17°C |
| POPC | 1-palmitoyl-2-oleoyl-glycero-3-phosphocholine | 16:0-18:1 PC | 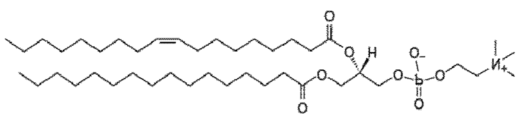 | -4°C |
| DMPC | 1,2-dimyristoyl-sn-glycero-3-phosphocholine | 14:0 PC | 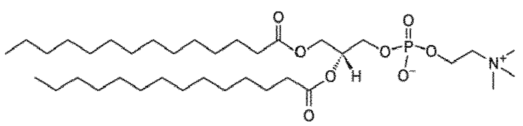 | 24°C |
| DPPC | 1,2-dipalmitoyl-glycero-3-phosphocholine | 16:0 PC | 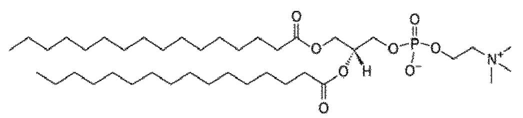 | 41°C |
| DSPC | 1,2-distearoyl-sn-glycero-3-phosphocholine | 18:0 PC | 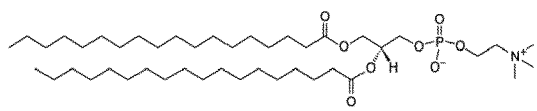 | 55°C |

Results plotted in **Figure 3A** show clear differences in the GP values according to the nature of the liposome going from the most rigid, DSPC (C18:0) with a GP value of 0.58 to the most fluid DLPC (C18:2) with a GP value of -0.26. GP values obtained are in correlation with Tm ranging from 55° for DSPC to -57°C for DLPC **(Table 2).**

Moreover, the new fluorophore proved to be an excellent tool to measure liposome fluidity by flow cytometry. This technique allows one to visualize and discriminate events (cells or particles) within a heterogeneous population via differences of size, granularity or fluorescence. Unlike the spectrofluorometer, which gives a bulk fluorescence spectrum and thus a bulk value of GP, by using flow cytometry, one may obtain the GP parameter of individual particles in a heterogeneous population.

To take advantage of a routine flow cytometer, the inventors replaced the GP parameter by the ratio 525 nm fluorescence emission intensity (corresponding to the fluid phase emission) on 450 nm fluorescence emission intensity (corresponding to the gel phase emission) which is equivalent to a ratio fluid state intensity on rigid state intensity. Results are plotted in **Figure 3B.**

Indeed, the correlation GP and fluidity state observed through spectroscopy is also found with a flow cytometer. The entire fluidity range is discriminated with the DSPC liposomes, theoretically the most rigid at 21°C, which has a lowest ratio 525/450, while the DLPC liposomes which the most fluid has a highest ratio 525/450.

d. <u>Labelling of membranes with DIOLL probe as a function of their fluidity state</u>

To check whether the probe empirically distinguishes differences in membrane fluidity in cell membranes, HMEC cells were incubated with DIOLL probe for 48h and then observed by confocal microscopy. Fluorescence images were recorded each 10 nm from 425 to 512 nm (excitation 405 nm). Dioll labeled uniformly all cell membranes, yet the fluorescence profile was different for each emission channel, with membrane structures that were more intensively labeled in the blue edge and others in the red edge of the spectrum. An overlay of images obtained is shown in **Figure 4,** where one clearly distinguishes different shades of grey corresponding to the sum of different fluorescence intensities obtained in each channel.

Different shades of grey corresponding to different fluorescence intensity obtained in each channel leads to the differentiation of the fluidity state of the cell membranes. To precisely identify plasma and organelle membranes, co-localization experiments with specific organelle markers were performed. Dioll fluorescence co-localized at the plasma membrane level with membrane label Dil. Dioll also showed a good co-localization with ER-tracker, which is a specific label of the endoplasmic reticulum. It also co-localized with Mito-tracker, a specific label of mitochondria. Of note, Dioll perfectly labels membrane structures of the nucleus. Nuclear content was labeled with Hoechst.

From Dioll membrane labelling, GP values were estimated from the relative intensities at 440 and 490 nm for each type of membrane identified above **(Figure 5).**

High GP values (0.05 ± 0.03) were obtained for plasma membrane structures (A). This is in line with literature data concerning plasma membrane composition, with a high percentage of sphingomyelin and cholesterol. Indeed, sphingolipids are lipids with high melting temperature and this type of lipids are expected to give stiffness to bilayers. On the other hand, membranes of endoplasmic reticulum (B), mitochondria (C) and nucleus (D) are rather fluid with GP values of -0.14± 0.06, -0.18±0.02 and -019±0.04, respectively. This is also in line with the membrane composition of these organelles. Endoplasmic reticulum displays extremely low concentrations of sterols and complex sphingolipids, despite being the main site of synthesis. The loose packing of endoplasmic reticulum membrane lipids is consistent with the function of the organelle in the insertion and transport of newly synthesized lipids and proteins. In mitochondria, cholesterol and sphingolipid content is also low. The major lipid constituents are phosphatidylcholine and phosphatidylethanolamine, which account together for about 80% of total phospholipids. Cardiolipin is present in the range of 10-20% of total mitochondrial phospholipids. In mammalian cells, approximately 50% of mitochondrial fatty acids are unsaturated, linoleic acid (18:1), linolenic acid (18:2) and arachidonic acid (20:4) being the most prominent fatty acids, which gives mitochondrial membranes a very high fluidity also reported with DIOLL probe fluorescence.

**Example 3: Quantification of lipid membranes in aqueous solutions**

Liposomes, various supported or tethered bilayers, monolayers, bicelles, to name only some, are largely used systems to mimic plasma and organelle membranes.

Among such models, liposomes are widely used to probe protein-lipid interactions, lipid organization and molecule penetration across membranes. They are also versatile assemblies in drug delivery systems, as they are non-toxic, biodegradable, and readily produced on a large scale. They are also extensively used for the reconstitution of transmembrane proteins, to form proteoliposomes, the lipid bilayer offering a native-like medium for membrane-spanning domains. For all these applications, liposomes undergo various fabrication processes: extrusion, centrifugation, protein reconstitution, cell-free protein synthesis, molecule grafting, etc. It is therefore of interest to determine lipid concentrations after various processes and in different production batches.

To estimate liposome concentration, several methods are popular. Among them, colorimetric methods such as Bartlett. and Rouser. methods are based on the reaction between the inorganic phosphate obtained after liposome treatment with perchloric acid and ammonium molibdate to obtain phosphomolibdate. The addition of a reducing agent such as 1-amino-2-naphtol-4 sulfonic acid (Bartlett method) and ascorbic acid (Rouser method) leads to formation of a violet complex absorbing at 820-830 nm. Both methods are rather sensitive (LOD and LOQ around 5 and 20 $\mu g.ml^{-1}$, respectively). Alternatively, the Stewart method (Stewart, Analytical Biochemistry 104, 10-14 (1980)) probably the most widely used method for phospholipid determination, is based on the formation of a complex between the phosphate group of the lipid polar head and ammonium ferro-thiocyanate, absorbing at 485 nm. It is rather fast and easy to implement, but requires lipid extraction in organic solvent and the use of chloroform-resistant recipes and protection. LOD and LOQ methods are evaluated at 10 $\mu g.ml^{-1}$ and 100 $\mu g.ml^{-1}$, respectively.

Chromatographic methods of lipid separation, together with mass spectroscopy or NMR are also methods of choice for lipid detection and quantification, as one can obtain not only the lipid amount, but also lipid class distribution. Yet, they are time consuming, rather expensive and not readily used in routine experiments.

Given the number of studies dwelling on liposomes, proteoliposomes and on cell-derived vesicles, the inventors propose a simple method to estimate the amount of membranes in aqueous solutions.

**1. Material and method**

a. Materials

DIOLL probe was synthesis as described in Example 1.

Lipids were purchased from Avanti Polar Lipids (Alabaster, Al). Hepes, PBS, NaCl, ferric chloride hexahydrate ($FeCl_3(H_2O)_6$) and ammonium thiocyanate ($NH_4SCN$) were purchased from Sigma Aldrich (St Quentin Falavier, France). Ultrapure water was obtained with a PureLabOption Q7 system (Véolia Water STI, Decines, France).

b. Methods

i. Liposome preparation

Liposomes were prepared using a classical freeze/thaw protocol. Briefly, the appropriate lipid mixture solubilized in chloroform was dried under vacuum, then solubilized with 1 ml buffer under strong agitation. Assays were done either in HEPES 20 mM pH 7.4 buffer 100 mM NaCl or in PBS. The liposome emulsion obtained was submitted to 6 freeze/thaw cycles (5 min in liquid nitrogen/10 min in a water bath at 37°C, 1 minute strong agitation) to decrease the number of bilayers and obtain unilamellar vesicles. To obtain homogenous vesicles, the liposome solution was extruded successively through a 400 nm and 100 nm polycarbonate membrane using an extruder (Avanti Polar Lipids). Liposomes were routinely prepared at lipid concentrations between 1 and 10 $mg.ml^{-1}$ and subsequently diluted at the desired concentration.

ii. DIOLL probe fluorescence-based liposome quantification

A POPC solution of known concentration (100 $\mu g.ml^{-1}$) was used as standard. Increasing volumes of the standard solution were diluted with buffer in a 96 well plate (Nunc). The final volume per well was 200 $\mu l$ and lipid concentrations between 0 and 80 $\mu g.ml^{-1}$ were used. Samples were prepared in parallel to a final volume of 200 $\mu l$ per well.

A constant volume of Dioll solution in ethanol (5 $\mu l$) was added in each well to obtain a final Dioll concentration of 2 $\mu M$. The volume of ethanol added represented 2.5 % of the final volume and had no effect on liposome integrity. The plate was incubated at room temperature for 10 min. Fluorescence emission spectra were recorded between 420 nm and 600 nm for an excitation wavelength of 390 nm with a TECAN InfiniteM200 plate reader. Spectra were recorded at 21°C.

The surface under the spectrum was calculated for each condition using an Excel complementary function, IntegralP (www.fordom.free.fr) which allows one to calculate the integral of a function when discrete data sets are available. Maximum fluorescence intensities or surfaces under the curve were plotted as a function of the lipid concentration to obtain the standard curve. They both show a hyperbolic variation and the equation of the standard curve was obtained using a hyperbolic fit $y=ax/(x+b)$ (SigmaPlot). The calculation of the a and b coefficients allowed us to determine unknown concentrations in the samples. As various volumes were used for the assays, the concentration of the stock liposome solution was presented taking into account various dilution factors. Measurements were performed in triplicates.

For the estimation of the limit of detection (LOD) and of quantification (LOQ), the fluorescence spectra of 2 $\mu M$ Dioll in aqueous buffer were recorded in the absence of liposomes and the surface under the curve was calculated to estimate the blank value on 5 independent measurements. LOD and LOQ were calculated as 3 times or 10 times the standard deviation of the blank, respectively.

Sensitivity was estimated from the slope of the linear fit for low liposome concentrations. As the fluorescence intensity is dependent on the instrument used, in this study the inventors estimated that an increase of 100 A.U. in the fluorescence intensity obtained with TECAN InfiniteM200 was significant.

To conclude upon the repeatability of the method, the coefficient of variation (CV) was calculated as the percentage of the standard deviation on the mean of 5 independent measurements. Accuracy was deduced from the difference between the concentrations obtained with the Dioll-based method and a standard method of phospholipid quantification, in our case the Stewart method.

For the Stewart method, lipids were extracted from the various samples with chloroform. In brief, aqueous samples were mixed with chloroform (10 times the volume of the sample) under vigorous shaking. The samples were then centrifuged at 300 x g for 10 min to accelerate phase separation (Multifuge 3 LR, Heraeus). The chloroform (bottom) phase was recovered with a glass pipette.

A POPC standard solution was prepared at 100 $\mu g.ml^{-1}$ in chloroform. Increasing volumes of the standard solution were diluted with chloroform in glass centrifuge tubes to a final volume of 2 ml. Samples extracted in chloroform were prepared in parallel and diluted to 2 ml. 2 ml of Ferrothiocyanate solution (obtained by mixing 20.7 g of Ferric chloride hexahydate and 30.4 g of ammonium thiocyanate in 1L of ultrapure water) was added to each tube. The solutions were vigorously mixed and then centrifuged at 300 x g for 10 min (Multifuge 3 LR, Heraeus). The chloroform (bottom) phase was recovered with a glass pipette and the absorbance was measured at 485 nm in glass cuvettes with a Jasco V-730 spectrophotometer. For quantification absorbance at 485 nm was plotted against phospholipid concentration.

iii. Purification of exosomes

The purification of exosomes was realized by a series of differential centrifugations. In brief, 120 ml of HMEC culture medium were collected after two days of culture, and centrifuged at 2 000 x g at 4°C during 10 min (Multifuge 3LR Heraus™). Supernatants were collected and ultracentrifuged at 20 000 x g, 4°C during 30 min (Optima LE-80 Beckman™ with a fixed rotor angle 50.2 Ti Beckman™). A second ultracentrifugation was realized at 100 000 x g, 4 °C during 2 h. Pellets were resuspended with 1 mL of PBS buffer, regrouped in the same tube and ultracentrifuged at 100 000 x g, 4 °C during 1 h. Finally, the pellet was resuspended in 200 μL of PBS buffer to obtain concentrated exosomes.

iv. Giant Plasma Membrane Vesicles preparation

Giant Plasma Membrane Vesicles (GPMV) were prepared according to previously described protocol (Baumgart, T. et al. Large-scale fluid/fluid phase separation of proteins and lipids in giant plasma membrane vesicles. Proc. Natl. Acad. Sci. (2007). doi:10.1073/pnas.0611357104) but adapted to PC-3 cell culture. In brief, PC-3 were cultured in RPMI-1640 10% FBS 100 U/mL penicillin and 100 μg/mL streptomycin under a humidified atmosphere of 5% $CO_2$ at 37°C at 80 % cell confluence in 25 $cm^2$ plastic flasks. Growth medium was removed and cells were incubated for 1h with 25 mM formaldehyde 2 mM DTT in PBS buffer at room temperature under shaking. The supernatant containing GPMVs was recovered and the presence of GPMVs was checked with an optical microscope. For Dioll-based measurements, 200 μl of the GPMV preparation was incubated with 5 μl DIOLL probe ethanol solution (2 μM final concentration) and fluorescence spectra were recorded as described above. The lipid concentration was determined against a standard curved obtained with POPC liposomes of known concentration.

## 2. Results

a. <u>Quantification of liposomes using DIOLL probe</u>

To probe the dependency of DIOLL probe fluorescence upon liposome amount, increasing concentrations of POPC liposomes were incubated with a constant concentration of 2 μM Dioll. Fluorescence spectra were recorded and plotted in **Figure 6A.** Fluorescence intensity is rather low in the absence of liposomes and increases progressively with lipid amount, but seems to reach saturation at concentrations over 50 $\mu g.ml^{-1}$. The fluorescence intensity emission maximum was obtained at 490 nm, which corresponds to lipid membranes in liquid disordered state, as expected for POPC liposomes at 21°C (Tm= -4°C). Fluorescence intensity at 490 nm as a function of the lipid concentration in the well **(Figure 6B)** showed a hyperbolic evolution. Yet, for polarity-sensitive probes, such as DIOLL probe, the position of the maximum fluorescence emission largely depends on the order state of the lipid membrane. This characteristic, which makes them valuable for determining the order state of the membrane, is an inconvenient when sample fluidity does not match that of the standard solution. Therefore, to overcome this inconvenient, the fluorescence intensity parameter was replaced by the surface under the peak, which does not depend on the fluidity state of the membrane. The surface under the peak was estimated by integrating the spectra obtained between 420 and 560 nm using an Excel complementary function, IntegralP (www.fordom.free.fr). When plotted as a function of the liposome concentration, the surface under

the peak also nicely fits a hyperbolic function **(Figure 6C).** Therefore, the hyperbolic dependency between the surface under the experimental fluorescence emission spectra and the liposome concentration in the well can be used as a calibration curve to measure liposome concentration in various samples.

The limit of detection (LOD), the lowest concentration that can be detected but not quantified, and the limit of quantification (LOQ), the lowest concentration that can be quantified with a reasonable degree of confidence, of the calibration curve were determined from the standard deviation of the blank solution (2 μM Dioll in buffer) (**Table 3).** The LOD, which corresponds to the lipid concentration obtained at 3 times the standard deviation of the blank, which was of 0.4 μg.ml$^{-1}$, whereas the LOQ corresponded to a lipid concentration of 1.2 μg.ml$^{-1}$ obtained for 10 times the standard deviation of the blank. These limits are rather low and make this method interesting for measuring low liposome concentrations, when small or diluted sample volumes are available.

**Table 3:** Validation of the detection method

| LOD | LOQ | Sensitivity | Repeatability | Accuracy |
|---|---|---|---|---|
| 0.4 μg.ml$^{-1}$ | 1.2 μg.ml$^{-1}$ | 10 000 (A.U) 0.4 μg.ml$^{-1}$ | 1.2% | 6.0% |

The sensitivity of the detection method is defined as the dependency between the instrumental response and the variation in the concentration of the compound of interest. It is instrument-dependent. In our conditions, the slope of the linear part was equal to $10^5$A.U (arbitrary unit). per concentration unit (μg.ml$^{-1}$). That means that, considering that the sensitivity limit of TECAN InfiniteM200 used in this study is 100 A.U., one can detect variations in liposome concentration of the sample as low as 1 ng.ml$^{-1}$.

The repeatability of the method, which represents the determination of how close values are to each other, under identical experimental conditions, was assessed by determination of the concentration of a POPC liposome solution prepared independently of the standard solution. A concentration of 914 ± 56 μg ml$^{-1}$ was obtained with 3 replicates, which gave a repeatability of 6 %. To assess the accuracy of the new quantification method, the same POPC liposome solution was measured using the largely used Stewart method. A difference of 1.2 % was obtained between mean values obtained with the two methods. Results were therefore consistent and opened the possibility to routinely use the DIOLL as a probe to measure lipid membrane amount in various samples, without lipid extraction and in a rather short time (less than 30 min).

Robustness of the Dioll method was checked with respect to various parameters. One major point of interest was whether the membrane fluidity, which largely influences Dioll fluorescence, had an impact on the Dioll-based quantification method. To this purpose, liposomes constituted of DMPC (1,2-dimyristoyl-sn-glycero-3-phosphocholine) and DPPC (1,2-dipalmitoyl-glycero-3-phosphocholine) were prepared **(Table 4).**

**Table 4:** Liposome preparations used and corresponding phase transition temperatures (Tm)

| Liposome preparation | Lipid | Acyl chain composition | Schematic structure | Tm |
|---|---|---|---|---|
| POPC | 1-palmitoyl-2-oleoyl-glycero-3-phosphocholine | 16:0-18:1 PC | 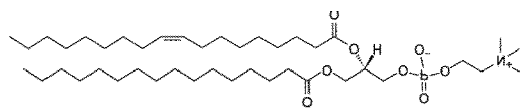 | -4°C |
| DMPC | 1,2-dimyristoyl-sn-glycero-3-phosphocholine | 14:0 PC | 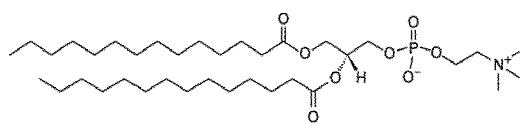 | 24°C |
| DPPC | 1,2-dipalmitoyl-glycero-3-phosphocholine | 16:0 PC | 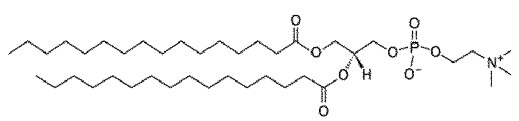 | 41°C |

In our experimental conditions (21°C), DMPC (gel to liquid crystalline transition temperature (Tm) of 23°C) is expected to be in an ordered state, but close to the liquid ordered-liquid disordered transition, and DPPC (Tm 41°C) in an ordered configuration. Independently, a standard liposome solution was prepared for each type of liposomes to obtain the corresponding calibration curves. DMPC-inserted Dioll fluorescence spectra **(Figure 7A)** showed a maximum fluorescence emission at 440 nm consistent with lipid membranes in an ordered state, with a contribution at 490 nm corresponding to the fluid phase. Although it is difficult to calculate the proportion of each state, the fluorescence emission spectra indicate that, in our experimental conditions, DMPC liposomes make a good model of membranes in which two phases coexist. When inserted in DPPC liposomes, Dioll maximum fluorescence emission was obtained at 440 nm, which is consistent with a membrane in gel/ordered state **(Figure 7B).** For both DMPC and DPPC liposomes, the surface under the peak estimated as described above gave hyperbolic evolutions **(Figure 7C** and **D**). The equations obtained by fitting the experimental data to a hyperbola allowed us to estimate the concentration of liposomes in various samples.

To check the influence of the membrane fluidity on the quantification method, POPC, DMPC and DPPC liposomes were prepared and their concentration was determined using either POPC, DMPC or DPPC calibration curves or by the Stewart method with POPC in chloroform solution as standard. Results are presented in **Figure 7E.** The concentration of the POPC solution measured using either POPC, DMPC or DPPC calibration curves was consistent with that obtained by the Stewart method. Similar results were obtained for DMPC and DPPC liposomes quantified using Dioll-based calibration curves or by Stewart method. Therefore, the surface under the peak parameter as a function of the liposome concentration can successfully replace the fluorescence intensity to measure sample concentration, whatever the membrane fluidity state. This is particularly interesting as membrane fluidity in samples of interest is usually unknown.

Cholesterol is one major component of membranes and also a major modulator of membrane order. Depending on membranes, the proportion of cholesterol varies between 10 and 30 % of lipid total mass. Liposomes containing a mixture of POPC and cholesterol where prepared and the concentration was measured using Dioll method and POPC liposomes of known concentration as a standard curve. The concentration obtained for 10, 20 and 30 % cholesterol correlated well with the concentration of phospholipids obtained using the Stewart method (**Figure 8,** white and black bars), but not with the total amount of lipids (if one takes into account 10, 20 or 30 mass % of cholesterol in the sample) (**Figure 8,** dotted bars).

Membranes contain non negligible amounts of negatively charged lipids with precise and fundamental functions. It is thus of interest to check the robustness of the Dioll-based quantification method towards negatively charged lipids. As an example, the inventors have chosen to prepare liposomes containing 50 % POPC and 50 % POPS (1-palmitoyl-2-oleoyl-glycero-3-phosphoserine). The concentration of the prepared liposome solution was estimated either by the Dioll-based method, with POPC liposomes of known concentration as standard curve, either by the Stewart method with POPC chloroform solution as standard **(Figure 9).** The two methods very nicely correlated.

To sum up, by using the surface under the curve parameter instead of the fluorescence emission intensity, the new fluorescence-based quantification method of membrane proved to be robust with respect to the order state of the membrane. By using a simple standard curve such as plain liposomes of increasing concentration and a constant fluorophore concentration, one can determine membrane amount in samples of different compositions: DMPC, DPPC, cholesterol-containing liposomes or negatively charged liposomes.

b. Quantification of exosomes using DIOLL probe

Exosomes are small vesicles (50 to 150 nm) secreted by cells, specific of their origin cell and which can be extracted from numerous body fluids (blood, urine...). They are therefore of interest for studies of pathologies, but also for the development of non-invasive diagnostic tools, notably in oncology. Measuring exosome concentration rapidly, with small sample volumes and in a non-destructive manner is of growing interest. Therefore, the inventors adapted the Dioll-based quantification method to this type of vesicles. A standard curve based on POPC liposomes of known concentration in the presence of a constant Dioll concentration (2 $\mu$M) was recorded as the surface under the curve vs. liposome concentration in the well, as described above. Exosome samples were incubated with the same Dioll concentration as the standard solution and the surface under the curve was calculated. Vesicle concentration was obtained using a hyperbolic fit of the standard curve and correlated with phospholipid determination using Stewart method (98 $\pm$ 10 $\mu g.ml^{-1}$ vs 102 $\pm$ 10 $\mu g.ml^{-1}$) **(Figure 10).**

c. Quantification of GPMVs vesicles using DIOLL probe

A second type of vesicles used was obtained from living cells by plasma membrane budding. GPMVs are derived from biological cells, by chemically induced (here with 25 mM formaldehyde, 2 mM DTT in PBS) plasma membrane vesiculation or "blebbing".

This type of vesicles is largely used to analyze phase coexistence in systems with realistic biological membrane compositions or peptide penetration across membranes. Given the raising interest in the study of plasma membrane heterogeneities, the inventors determined the amount of membranes in the GPMVs samples. As expected from literature data, GPMVs obtained by this method show simple, low-curvature geometries of giant unilamellar vesicles and appear free of cytoskeletal constraints. Using Dioll fluorescence a lipid concentration of 84 $\pm$ 1 $\mu g.ml^{-1}$ was obtained from an estimated PC-3 cell population of 5 million. Using the Stewart method, a good correlation was observed with an estimated

phospholipid sample concentration of 87 ± 1 μg.ml$^{-1}$ **(Figure 11).**

### 3. Discussion

Quantification methods of membrane content in various samples are scarce and require either lengthy protocols involving lipid extraction and costly instruments (GC, MS, NMR), organic solvents or energetic conditions.

The inventors present a new, rapid and easy to use method of lipid membrane quantification in artificial and natural vesicles based on the fluorescence of DIOLL probe. This fluorophore has a very low fluorescence in aqueous buffers, but when it spontaneously inserts into lipid bilayers its fluorescence drastically increases. the inventors checked therefore whether this was a regular increase, by incubating a constant amount of fluorophore with increasing amounts of liposomes. As shown in **Figure 6,** there was a hyperbolic dependency not only between the fluorescence intensity and the lipid amount in the sample, but also between the surface under the fluorescence emission spectra and the lipid amount. This parameter, unlike the fluorescence intensity, does not depend on the membrane fluidity and may serve as a universal parameter to quantify membranes.

To check this hypothesis, 3 types of liposomes were prepared with lipids with different ordering degrees at room temperature (23°C): POPC (fluid state), DMPC (phase transition) and DPPC (gel state). The amount of liposomes was quantified using as standards either liposome solutions of the same nature, either liposome solutions of different fluidity. Quantification results **(Figure 7)** show that similar results were obtained whatever the composition of the standard solution. Moreover, the lipid concentrations in the samples correlated well with those obtained by Stewart method after lipid extraction in chloroform. Thus, a plain solution of POPC liposomes of known concentration may serve as a standard curve for samples of diverse lipid composition and diverse membrane states.

Detection and quantification limits of the POPC standard curve attained values as low as 0.4 and 1.2 μg.ml$^{-1}$, comparable with those of Bartlett and Stewart methods, which allows one to quantify low amounts of biological samples. Although a fluorophore is inserted into the bilayer, this method is not destructive and liposomes, proteoliposomes or diverse vesicles may be used for further applications.

The sensitivity of the method was good up to 40 μg.ml$^{-1}$. Above this value, the intensity of fluorescence reaches a plateau and samples require dilutions. The sensitivity of the method depends on the instrumental response to the variation in the analyte concentration, therefore it is instrument-dependent. In our conditions, by using an instrument such as TECAN InfiniteM200, the inventors detect variations in the liposome concentrations the range of 1 ng.ml$^{-1}$.

Robustness of the detection method was checked with respect to the presence of cholesterol **(Figure 8)** and negatively charged lipids **(Figure 9)** and a good correlation was obtained with phospholipid quantification by Stewart method. Therefore, this method was used for quantification of more complex vesicles, and natural vesicles, such as exosomes and GPMVs. If the inventors consider the insertion mode of its analogue, Laurdan, Dioll is expected to fit between lipids at the level of the glycerol backbone. Therefore, the fluorescence intensity is dependent on the amount of phospholipid membranes. Cholesterol and other components, like proteins, or glycolipids, in natural vesicles, largely influence membrane fluidity and, consequently, the shape of the spectrum, but did not influence fluorescence yield in terms of surface under the curve. Therefore, systematic correlation was obtained between membrane content determined by Dioll and phospholipid concentration determined by Stewart method.

To conclude, Dioll fluorescence can be used as a parameter to measure the amount of phospholipid membranes in complex samples without solvent extraction and that by using simple liposome compositions as standards. The detection method, based on fluorescence, is fast (less than 30 min with a plate reader, less than 1h with a classical fluorimeter), simple to use, sensitive and realizable with standard lab equipment

### Example 4: Fluidity characterisation of nanoemulsions preparation with DIOLL probe

### 1. Materials and methods

a. Material

DIOLL probe was synthesis as described in Example 1.

Medium chain triglycerides, MCT (Miglyol®812) purchased from CREMER OLEO GmbH & Co. KG (Hamburg, Germany) was used as the oil phase. Polyoxyethylene (40) stearate (Myrj®52) from Sigma-Aldrich (St Quentin-Fallavier, France) and oleoyl polyoxyl-6 glycerides (Labrafil®M1944CS) from Gattefosse (Saint-Priest, France) were used as nonionic surfactants. The aqueous phase used to prepare emulsions was sodium phosphate buffer solution (5 mM; pH 7.4). Tacrolimus (FK-506) was purchased from LC Laboratories (Woburn, MA, USA). Hydrochloric acid 37%, AnalaR NORMAPUR® Reag. Ph. Eur. was from VWR International (Fontenay-sous-Bois, France). Dichloromethane, methanol, acetonitrile (HPLC grade), dodecyl sulfate sodium salt pure, sodium taurocholate hydrate 96% and sodium hydroxide were purchased from Fisher Scientific (Illkirch, France). Egg phospholipids with 70% phosphatidylcholine (Lipoid E80S)

were from Lipoid GmbH (Ludwigshafen am Rhein, Germany). Acetic acid was obtained from Chem-Lab NV (Zedelgem, Belgium). Maltodextrin MD (Glucidex® 12D) from Roquette Frères (Lestrem, France) and trehalose 100 (TR) as a gift of Hayashibara Co. Ltd (Okayama, Japan) were used as cryoprotectants in the drying studies. Milli-Q water, used as the aqueous phase to prepare all solutions and emulsions, was obtained using a Milli-Q Academic System (Millipore, Saint Quentin, Yvelines France).

b. Methods

i. Nanoemulsion preparation

Nanoemulsions (NE) were prepared by emulsion phase inversion (EPI) technique. The oil phase was prepared by mixing the oil (MCT) and surfactants (Polyoxyethylene (40) stearate: S1; and oleoyl polyoxyl-6 glycerides: S2) under magnetic stirring (750 rpm) at 80°C. Then, the aqueous phase (PBS 5 mM), heated up at 80°C as well, was added all at once into the organic melt phase. Stirring was performed using a rotor-stator disperser (T25 digital Ultra-Turrax® equipped with a S25N-8G shaft (IKA®-Werke GmbH & Co. KG, Staufen, Germany) at 11,000 rpm. The process was designed so that the final emulsion always had a total mass of 5 g. The resulting colloidal system was cooled to room temperature under magnetic stirring during 30 min.

ii. Determination of NE fluidity

The fluidity of the NE shell was determined by means of DIOLL probe. The DIOLL probe was dissolved in ethanol (263 μM). NE was diluted 1:1000 (0.027% w/v). 10 μL of Dioll solution was added to 1 mL NE to reach a 2.6 μM final concentration of Dioll, corresponding to a Dioll-NE ratio of 1:290 (w/w) The sample was incubated for 20 min at room temperature. As controls, the fluorescence of Dioll solutions (2.6 μM) in MCT and in water, and the fluorescence of blank NE (without Dioll) were recorded. In this case, 10 μL of ethanol was added to 1 mL NE. Fluorescence data were obtained using a FP-8500 spectrofluorometer (JASCO applied science, Halifax, Canada). The excitation wavelength was 390 nm and the emission spectra were recorded between 400 and 600 nm at several temperatures ranging from 5 to 27°C in steps of 2°C and at 37°C (2.5 nm bandwidth). The generalized polarization (GP) parameter was calculated from emission intensities according to:

$$GP = \frac{I_{440} - I_{490}}{I_{440} + I_{490}}$$

where $I_{440}$ and $I_{490}$ are recorded fluorescence intensities at wavelengths of 440 nm and 490 nm, respectively.

**2. Results**

Fluorescence measurements using polarity sensitive fluorophores were performed. Laurdan (6-dodecanoyl-2-dimethylaminonaphthalene) is a polarity sensitive fluorophore commonly used for the assessment of liposomes rigidity and the study of biological membranes. Yet, when incubated with NE solution, its fluorescence emission spectrum was characteristic of a highly hydrophobic environment, single peak at 440 nm, suggesting that the fluorophore was inserted in the hydrophobic core of the NE.

DIOLL probe was post-inserted in the NE shell by simple mixing the probe with the NE suspension (Dioll-NE). Insertion of Dioll in the NE shell was checked by measurements of emission spectra of controls. Due to the lack of fluorescent activity in hydrophilic environment, no fluorescence emission was detected when the probe was dissolved in water. Neither blank NE emitted fluorescence. A single peak at 440 nm was observed when Dioll was dissolved in MCT related to the radiative de-excitation of a "locally excited" state of Dioll in oil. Emission spectra of Dioll-NE at temperatures ranging from 5°C to 37°C showed two peaks at 440 nm and 490 nm, (**Figure 12)**, which, based on literature data on Laurdan, corresponded to fluorophore inserted into a rigid or a fluid environment, respectively, each band depending on the physical state of the medium and on the capacity of the probe to undergo interactions with the surrounding water molecules. So, the inventors could conclude that Dioll was emitting from NE surfactant shell and not from the MCT core of NE. Indeed, the Dioll hydrophobic tail could be oriented towards the NE oil core, while the hydrophilic and fluorescent naphthalene moiety could be aligned with the PEG components of S1, towards the surrounding water phase. NE size, *PdI* and surface charge did not vary upon insertion of the probe (size: 105 ± 1 nm, *PdI:* 0.21, zeta potential: -18 ± 1 mV).

Decreasing the temperature from 37°C to 5°C led to the progressive increase of peak intensity at 440 nm (**Figure 12A**). To quantify membrane fluidity state at each temperature, the GP values were calculated as described in

Materials and methods section. GP increased as the temperature was lowered (**Figure 12B**), proving decreased fluidity of the NE shell. However, GP values remained negative (fluid state) and no significant phase transition from fluid to rigid state occurred over the full temperature range. The NE shell was in its fluid state, even at low temperature (5°C).

**Example 5: Distinguishing cancer cells from healthy cells with DIOLL probe**

A large number of pathologies originate in the deregulation of cellular differentiation processes leading to the appearance of non-adapted phenotypes, a phenomenon summarized as abnormal cellular genetic plasticity such as pulmonary, hepatic or cardiac. The mechanisms driving these processes are complex and rely heavily on epigenetic control of the genome. These epigenetic controls can be influenced by environmental stresses such as infections, toxicological agents or poor nutrition, but also by intrinsic genetic alterations such as those found during tumour development.

In general, cancer cells have greater genetic plasticity than healthy cells. However, there are different expressions of this plasticity which are difficult to quantify since only *in vitro* functional tests can measure the ability of cancer cells to modulate their differentiation program according to the signals imposed on them. On the other hand, a large amount of data from the literature suggests that the fluidity of cancer cell membranes is increased. the inventors thus assumed that, in general, the fluidity of the biological membranes could represent the degree of plasticity of the cell, thus allowing the measurement of a plasticity index based on a biophysical parameter that can easily be quantified by DIOLL probe.

In order to demonstrate and validate this concept, the inventors used cancer cells from two different tissues, but with common characteristics allowing their comparison: the mammary gland and the prostate. Among these characteristics, it is possible to highlight the existence of different forms of tumours with varying and well-characterized degrees of dedifferentiation. On one hand, little aggressive tumours can retain their glandular structure and on another hand, cells can retain their dependence on hormones, while conversely, the most aggressive tumours are hormone-independent and totally unstructured. Finally, there are pathologies in which cancer cells are no longer only dedifferentiated, but also acquire new phenotypes that are outliers in relation to their original tissue. These tumours testify that after having seen a sharp increase in their plasticity, the phenotype of cancer cells is fixed in an abnormal state and different from their original state, which is then a *priori* characterized by a reduction in their plasticity.

**1. Distinguishing mammary gland and the prostate tumorous cell line as function to their membrane fluidity**

In order to validate the use of the DIOLL probe to measure the genetic plasticity of cancer cells, the inventors first analyzed the expression of a genetic signature associated with this plasticity by grouping together mammary and prostate cell lines (**Figure 13**).

As shown in this figure, the most plastic mammary lines correspond to a subtype qualified as basal and hormone-independent. In contrast, luminal cell lines show some conservation of their original mammary characteristics such as their ability to respond to hormones, while the Her2 subgroup is in an intermediate position. There is also a subgroup known as "Claudin-Low" with outlier mesenchymal differentiation.

By performing a main component analysis based on epithelial identity and cell plasticity, the inventors discriminate mammary lines as expected. On the other hand, prostate lines are dispersed in these subgroups, showing that this concept of plasticity is not specific to tissue but reflects a particular physiological situation.

From cell lines representative of these subgroups, the inventors to measure by various methods the fluidity of their cell membrane using the DIOLL probe as analytical tools. **Table 5** below summarizes the different lines studied and their known characteristics.

**Table 5:** Known Mammary gland and prostate tumour cell lines and their specifications

| **Human tumour cell lines** | | **Specifications** |
|---|---|---|
| Mammary gland | MCF-7 | Luminal cell lines responding to hormones, which are very close to epithelial cells |
| | ZR-75 | |
| | BT20 | Basal cell lines, dedifferentiated |
| | HCC1954 | |
| | HCC1937 | |
| | HS578T | "Claudin-low" cell lines, aberrant mesenchymal differentiation |
| | MDA-MB-231 | |
| | MDA-MB-436 | |
| Prostate | LNCaP | Hormone-sensitive epithelial adenocarcinoma epithelial cells |
| | C4-2B | Cell line derived from the LNCap line, hormone-independent |
| | PC-3 | Very aggressive metastatic cell line, total loss of its prostate markers, hormone-independence |

Two control lines were also used, HMEC cells representing normal mammary epithelial luminal progenitors and WPMY-1 cells corresponding to the prostatic stromal compartment (presence of a normal mesenchymal phenotype). The absence of expected plasticity in these cells should make it possible to validate that membrane fluidity reflects cellular plasticity, independently of epithelial or mesenchymal character.

After incorporation of the DIOLL probe into the cell membranes of these different cell lines (48h incubation), the generalized polarization (GP) parameter was calculated from emission intensities after excitation at 390 nm according to:

$$GP = \frac{I_{440} - I_{490}}{I_{440} + I_{490}}$$

where $I_{440}$ and $I_{490}$ correspond to measured fluorescence intensities of the whole cells at wavelengths of 440 nm and 490 nm, respectively.

The results are summarized in **Figure 14.** From these results, the inventors can validate that the cell lines genetically defined as the most plastic have the most fluid membranes (HCC1954, BT20). On the other hand, luminal and "Claudin-Low" cell lines, regardless of the conservation of their epithelial identity, have a larger GP showing the greater rigidity of their membrane in accordance with their supposed lower plasticity. Among the Claudin-Low cell lines, the inventors can also note the existence of lines such as MDA-MB231 with a membrane fluidity similar to that of the basal lines. In light of the genetic analysis described above, this result becomes quite relevant since this cell line also has, through this analysis, a plasticity similar to that of basal lines. These results, therefore, confirm the existence of a very good correlation between the epigenetic plasticity of the cell and membrane fluidity.

It is worth noting that the cells lines were distinguished with DIOLL probe regardless of the temperature at which the $I_{440}$ and $I_{490}$ were recorded.

**2. Variation of the membrane fluidity in mammary gland cell lines**

a. Epithelial cells subjected to an aberrant mesenchymal differentiation protocol

To complete the previous result, basal cell lines, also called epithelial cell lines, HCC1954 and HCC1937 were subjected during three weeks to an aberrant mesenchymal differentiation protocol (HCC1954mes/HCC1937mes) through a combination of growth factor (TGFb at 10μg/ml) and hormones (hydrocortisone 500nM).

After derivation, the inventors validate that in relation to this differentiation process and the decrease in cellular plasticity, the fluidity of the membrane systems of these cells was attenuated, as demonstrated by the increase in the GP associated with them (**Figure 15**).

b. Epithelial cells genetically forced to transit to a mesenchymal stage

In order to confirm that membrane fluidity was indeed a reflection of a plasticity change independent of genetic variations between individuals and likely to disrupt previous measurements, the inventors analysed a genetically pure model where mammary epithelial cells are genetically forced to transit to a mesenchymal stage. During the transition, the cells go through a stage of high plasticity necessary for this phenotypic change.

Human mammary epithelial cells (HMEC) have been transduced by a genetic construct encoding ZEB1, under the control of a promoter repressed in the absence of doxycycline in the cell medium. Doxycycline at 2μg/ml can dynamically activate the expression of the transcription factor ZEB1 causing a epithelial-mesenchymal transition mechanism. Thereby, the original differentiated, epithelial, HMEC cells after ZEB1 induction, initially progress into dedifferentiated, cells with partial mesenchymal features.

We easily separate, by both spectrofluorimetry (**Figure 16A**) and flow cytometry (**Figure 16B),** the differentiated cells from the dedifferentiated cells in the transition to a mesenchymal phenotype and characterized by a very high degree of plasticity.

**3. Variation of the membrane fluidity in prostate cell lines**

Then the inventors verified that the relationship between the degree of genetic plasticity and the fluidity of the membranes was not a particularity of mammary cells but could extend to other neoplasia such as that affecting prostate tissue. 4 cell lines were tested: LNCaP which is a basal cell line, PC-3 which an aggressive metastatic cell line, C4-2B cell line which are mesenchymal cell lines derived from LNCap and WPMY-1 which is an epithelial and mesenchymal control cell line.

The results are presented in **Figure 17** and confirm the modulation of membrane fluidity as a function of the level of plasticity attributed to these cell lines. It can be noted that the normal mesenchymal WPMY-1 cell line conserves relatively rigid membranes, while the PC-3 cell line, genetically close to basal mammary gland cell lines, has a very high membrane fluidity. Besides, it can be noted that the derived hormone-independent C4-2B cell line, which reflects its dedifferentiated state compared to its original LNCaP cell line, has a much greater fluidity than the latter.

It is worth noting here again that the cells lines were distinguished with DIOLL probe regardless of the temperature at which the $I_{440}$ and $I_{490}$ were recorded.

**4. Variation of the membrane fluidity in exosomes**

The analysis of the membranes of exosomes is of twofold interest. Indeed, on one hand, these vesicles are released into the circulation and are therefore easily accessible, making it possible to consider a less invasive diagnostic tool than a tumour biopsy and longitudinal patient follow-up, as the sampling can be repeated several times. On the other hand, the membranes of these vesicles are not subjected to any mechanical stress related to tissue architecture, unlike the cells recovered by biopsy, which eliminates a factor that strongly constrains membrane fluidity and overlaps with the intrinsic properties of these membranes.

a. exosomes from differentiated and dedifferentiated HMEC cells

The exosomes from the HMEC cells described above were purified, as described in Example 3, in order to analyse the GP of their membrane. Regardless of the method used, spectrofluorometry (**Figure 18A**) or flow cytometry (**Figure 18B**), the inventors demonstrate that the fluidity of the membranes of these exosomes was perfectly correlated with that of the cells from which they originated, thus making them excellent witnesses of the degree of genetic plasticity of these cells.

b. exosomes from dedifferentiated HMEC cells at different times of induction of dedifferentiation

Here is studied the kinetic analysis of exosomes from HMEC cells described above in transition to the mesenchymal phenotype, purified as described in Example 3 (**Figure 19A** in spectrofluorometry and **Figure 19B** in cytometry). The HMEC cells were mediated from 0 to 12 days by the ZEB1 transcription factor, leading to an evolutive phenotype from differentiated HMEC cells (0 days) to dedifferentiated HMEC cells (12 days).

The inventors observe that the progressive increase in the fluidity of the membranes of these vesicles is well related to the increase in the level of genetic plasticity of the cells from which they are derived. Therefore, these experiments confirm that the membrane fluidity of exosomes reflects an index of the genetic plasticity of the cells from which their originate.

**Claims**

1. Fluorescent compound of formula I:

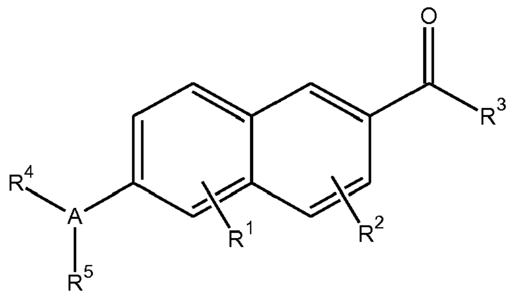

(I)

wherein A is selected from P, P=O and N;

$R^1$ is selected from the group comprising hydrogen atom, $NO_2$, $NR^6_2$, $CH_3$, $OR^6$, $SO_3H$; Cl, F, $CO_2H$, $CONR^6_2$, $O_2CR^6$, $NHCOR^6$; $NHCO_2R^6$ and $NHCONR^6_2$ $R^2$ is selected from the group comprising hydrogen atom, $NO_2$, $NH_2$, $CH_3$, OH, $SO_3H$ and halogen;
$R^3$ is a C3-C25 alkyl, linear or branched, saturated or not, cyclic or not;
$R^4$ and $R^5$ are selected from the group consisting of:

- $R^4$ and $R^5$ are either together and independently from each other a C2-C6 alkyl, linear or branched, saturated or not, cyclic or not, substituted by at least a hydroxyl group,
- $R^4$ is a C3-C6 alkyl linear or branched, saturated or not, cyclic or not, substituted by at least two hydroxyl groups and $R^5$ is a C1-C6 alkyl linear or branched, saturated or not, cyclic or not, not substituted, and
- $R^5$ is a C3-C6 alkyl linear or branched, saturated or not, cyclic or not, substituted by at least two hydroxyl groups and $R^4$ is a C1-C6 alkyl linear or branched, saturated or not, cyclic or not, not substituted; and

$R^6$ is selected in the group comprising hydrogen atom and a C1-C6 alkyl, linear or branched, saturated or not, cyclic or not,
or a salt or a solvate thereof,
preferably said fluorescent compound being of formula I-a:

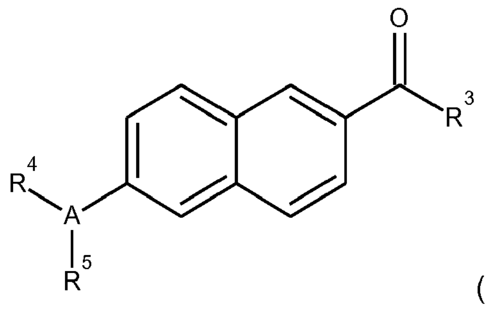

(I-a),

wherein $R^3$, $R^4$ and $R^5$ are as defined above.

2. The fluorescent compound according to claim 1, wherein $R^4$ and $R^5$ are together and independently from each other

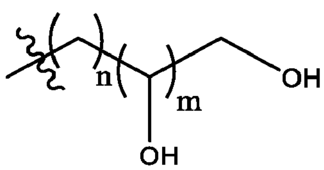

or

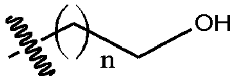

,

or

$R^4$ is

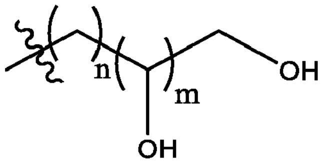

and $R^5$ is

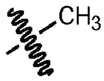

,

or

$R^4$ is

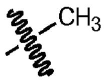

and $R^5$ is

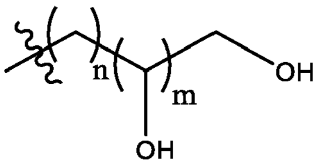

wherein n and m are together and independently from each other an integer from 1 to 4, and wherein the sum of n and m is an integer from 2 to 5.

**3.** The fluorescent compound according to claim 3 of the following formula:

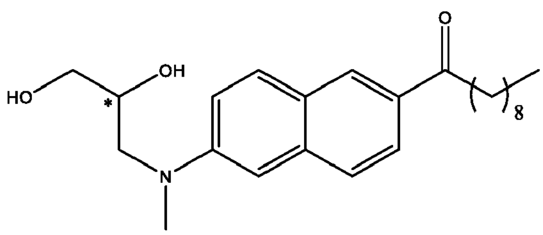

(11)

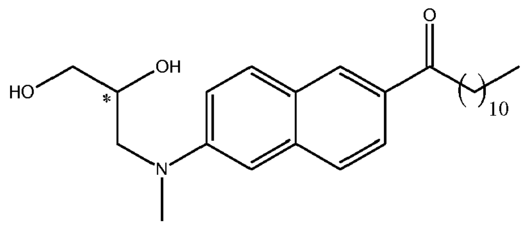

(12),

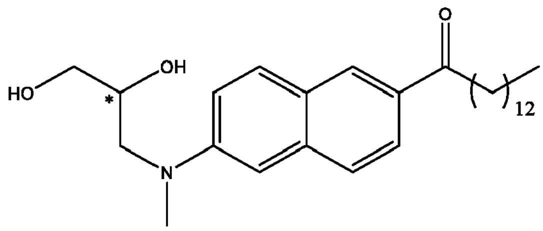

(13),

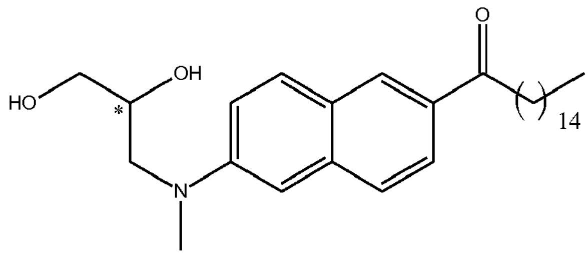

(14),

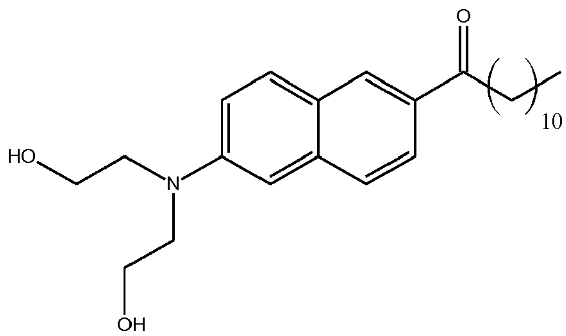

(15),

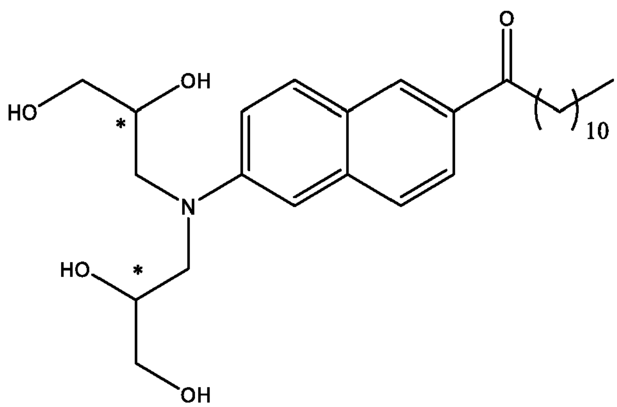

(16),

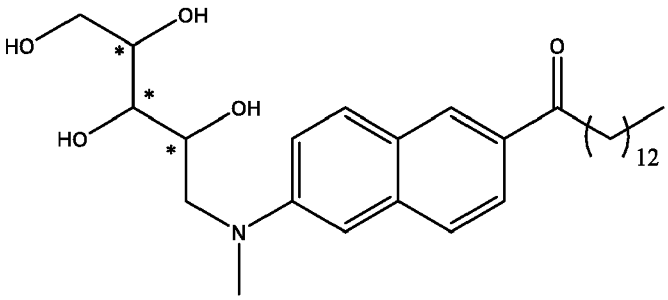

(17),

or

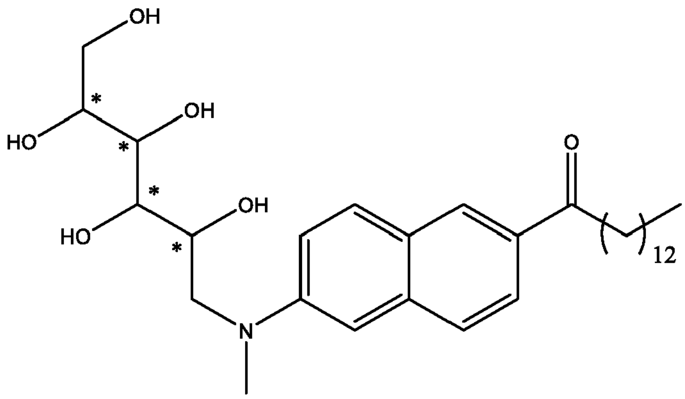

(18).

4. Use of a fluorescent compound according to any of claims 1 to 3, for *in vitro* and ex *vivo* labelling a lipid membrane or part thereof.

5. Method for *in vitro* and *ex vivo* labelling a lipid membrane or part thereof comprising a step of contacting a fluorescent compound according to any of claims 1 to 3 with a lipid membrane or part thereof, and a step of detecting the fluorescence of the said lipid membrane or part thereof labelled by the said fluorescent compound.

6. The method for *in vitro* and *ex vivo* labelling a lipid membrane according to claim 5, wherein the lipid membrane or part thereof is selected from a lipid monolayer membrane and a lipid bilayer membrane.

7. The method *in vitro* and *ex vivo* for labelling a lipid membrane according to claim 5 or 6, wherein the lipid membrane is a eukaryote cell lipid membrane.

8. Use of a fluorescent compound according to any of claims 1 to 3, for *in vitro* and ex *vivo* evaluating the degree of fluidity of a lipid membrane.

9. Method for *in vitro* and *ex vivo* evaluating the degree of fluidity of a lipid membrane, said method comprising the steps of:

a) labelling a lipid membrane or part thereof with a fluorescent compound according to any of claims 1 to 3 by carrying out the method of labelling according to claims 4 to 7,
b) determining a generalized polarization parameter GP of the lipid membrane or part thereof by the following formula

$$GP = \frac{I_{gel} - I_{lc}}{I_{gel} + I_{lc}}$$

wherein $I_{gel}$ and $I_{lc}$ are intensities of fluorescence measured by fluorimetry from the said lipid membrane or part thereof labeled by said fluorescent compound, the measurement of said intensity $I_{gel}$ being performed at a

wavelength characteristic of said fluorescent compound in gel lipid phase, and the measurement of said intensity $I_{lc}$ being performed at a wavelength characteristic of said fluorescent compound in liquid crystal lipid phase,
c) concluding that the lipid membrane is fluid if GP is lower or equal to 0, or concluding that the first lipid membrane is rigid if GP is higher than 0.

**10.** Method for *in vitro* and *ex vivo* evaluating the degree of fluidity of a lipid membrane, said method comprising the steps of:

a) labelling a lipid membrane or part thereof with a fluorescent compound according to any of claims 1 to 3 by carrying out the method of labelling according to claims 4 to 7,
b) determining a generalized polarization parameter GP of the lipid membrane or part thereof by the following formula

$$GP = \frac{I_{gel} - I_{lc}}{I_{gel} + I_{lc}}$$

wherein $I_{gel}$ and $I_{lc}$ are intensities of fluorescence measured by fluorimetry from the lipid membrane or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,
c) comparing said GP with a reference generalized polarization parameter $GP_R$ from a reference lipid membrane, and
d) concluding that the lipid membrane is more fluid or has a higher fluidity compared to the reference lipid membrane if GP is lower than $GP_R$, concluding that the first lipid membrane is more rigid or has a lower fluidity compared to the reference lipid membrane if GP is higher than $GP_R$, or concluding that the first lipid membrane has the same fluidity or degree of fluidity compared to the reference membrane if GP is equal to $GP_R$.

**11.** Use of a fluorescent compound according to any of claims 1 to 3, for *in vitro* and ex *vivo* quantification of lipids organized in at least one lipid membrane or part thereof in an aqueous solution.

**12.** Use of a fluorescent compound according to any of claims 1 to 3, for *in vitro* and *ex vivo* diagnosis of a cancer in an individual.

**13.** Method for the *in vitro* and *ex vivo* diagnosis of a cancer in an individual, said method comprising the steps of:

a) labelling a cell membrane or part thereof in a sample of an individual with a fluorescent compound according to any of claims 1 to 3 by carrying out the method of labelling according to any of claims 5 to 7,
b) determining a generalized polarization parameter GP of said cell membranes or part thereof by the following formula:

$$GP = \frac{I_{gel} - I_{lc}}{I_{gel} + I_{lc}}$$

wherein $I_{gel}$ and $I_{lc}$ are intensities of fluorescence measured by fluorimetry from cell membranes or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,
c) comparing said GP with a reference generalized polarization parameter $GP_R$ of said cell membranes or part thereof, and
d) concluding that said individual is afflicted by a cancer if said GP is lower than said $GP_R$, or
concluding that said individual is not afflicted by a cancer if said GP is equal or higher than said $GP_R$.

**14.** Use of a fluorescent compound according to any of claims 1 to 3, for *in vitro* and ex *vivo* diagnosis of the response to a therapeutic treatment of a patient afflicted with a cancer, said therapeutic treatment being directed to said cancer.

**15.** Method for *in vitro* and *ex vivo* evaluation of the response to a therapeutic treatment of a patient afflicted with a cancer, said therapeutic treatment being directed to said cancer, said method comprising the steps of:

a) labelling a cell membrane or part thereof in a sample from a tumour of said patient obtained after said therapeutic treatment, with a fluorescent compound according to any of claims 1 to 3 by carrying out the method of labelling according to any of claims 5 to 7,
b) determining a generalized polarization parameter GP of said cell membrane or part thereof by the following formula:

$$GP = \frac{I_{gel} - I_{lc}}{I_{gel} + I_{lc}}$$

wherein $I_{gel}$ and $I_{lc}$ are intensities of fluorescence measured by fluorimetry from said cell membrane or part thereof labeled by said fluorescent compound, the measurement of said intensity $I_{gel}$ being performed at a wavelength characteristic of said fluorescent compound in gel lipid phase, and the measurement of said intensity $I_{lc}$ being performed at a wavelength characteristic of said fluorescent compound in liquid crystal lipid phase,
c) comparing said GP with a reference generalized polarization parameter $GP_R$ of cell membranes or part thereof, said $GP_R$ being a prior determination of GP according to steps a) and b) from cell membrane or part thereof in a sample from the tumour of said patient obtained before said therapeutic treatment, and
d) concluding that said patient is considered to be responsive to the said therapeutic treatment if GP is higher than $GP_R$, or
concluding that patient is not considered to be responsive to the said therapeutic treatment if GP is lower or equal to $GP_R$.

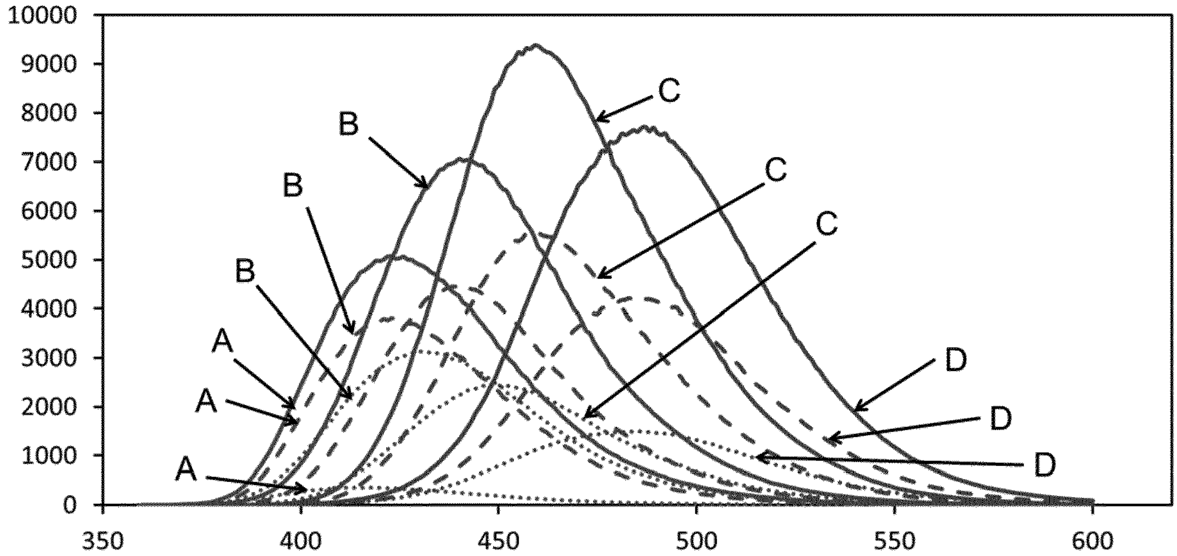


Figure 1

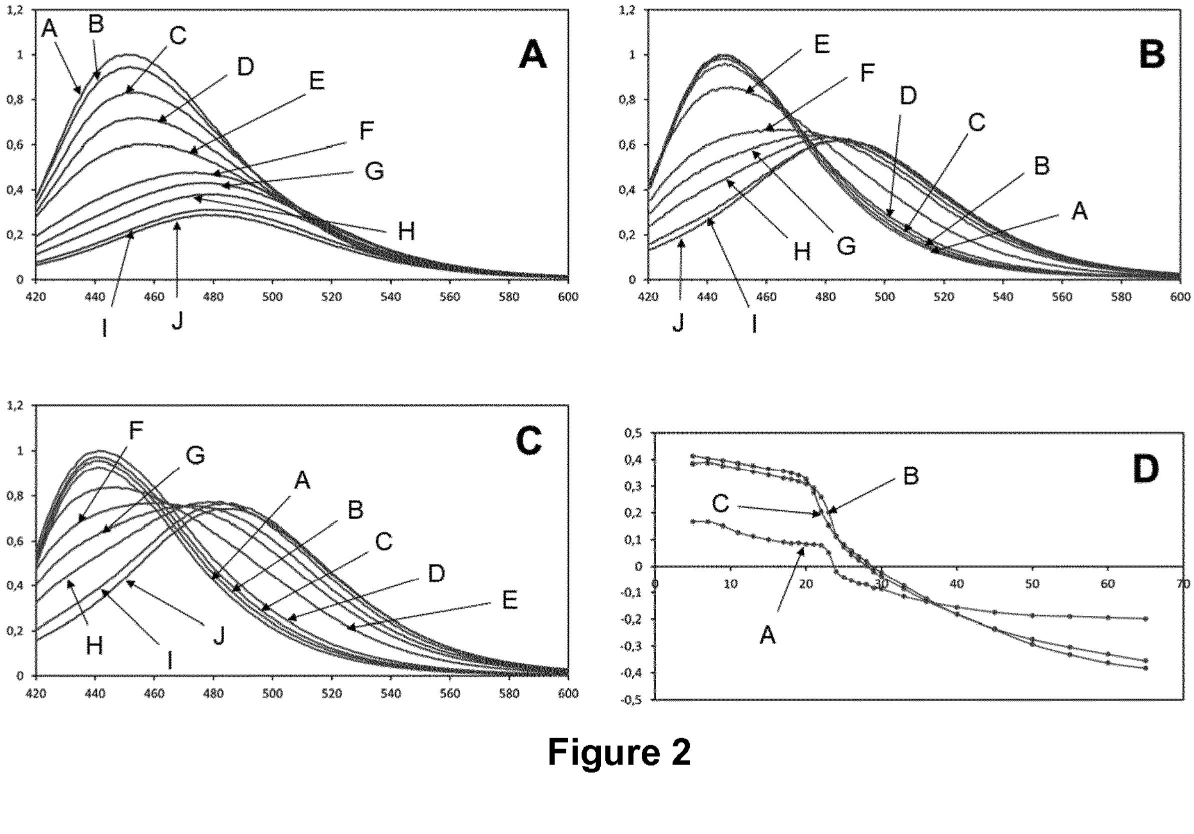


Figure 2

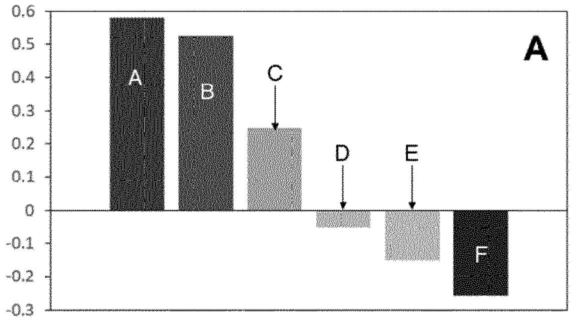


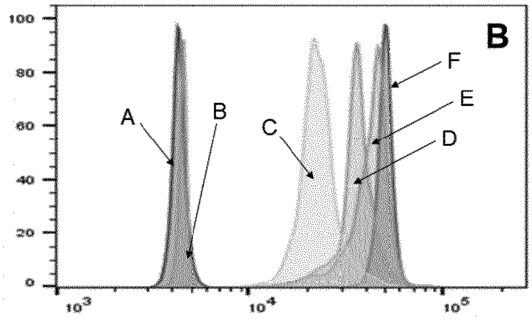


Figure 3

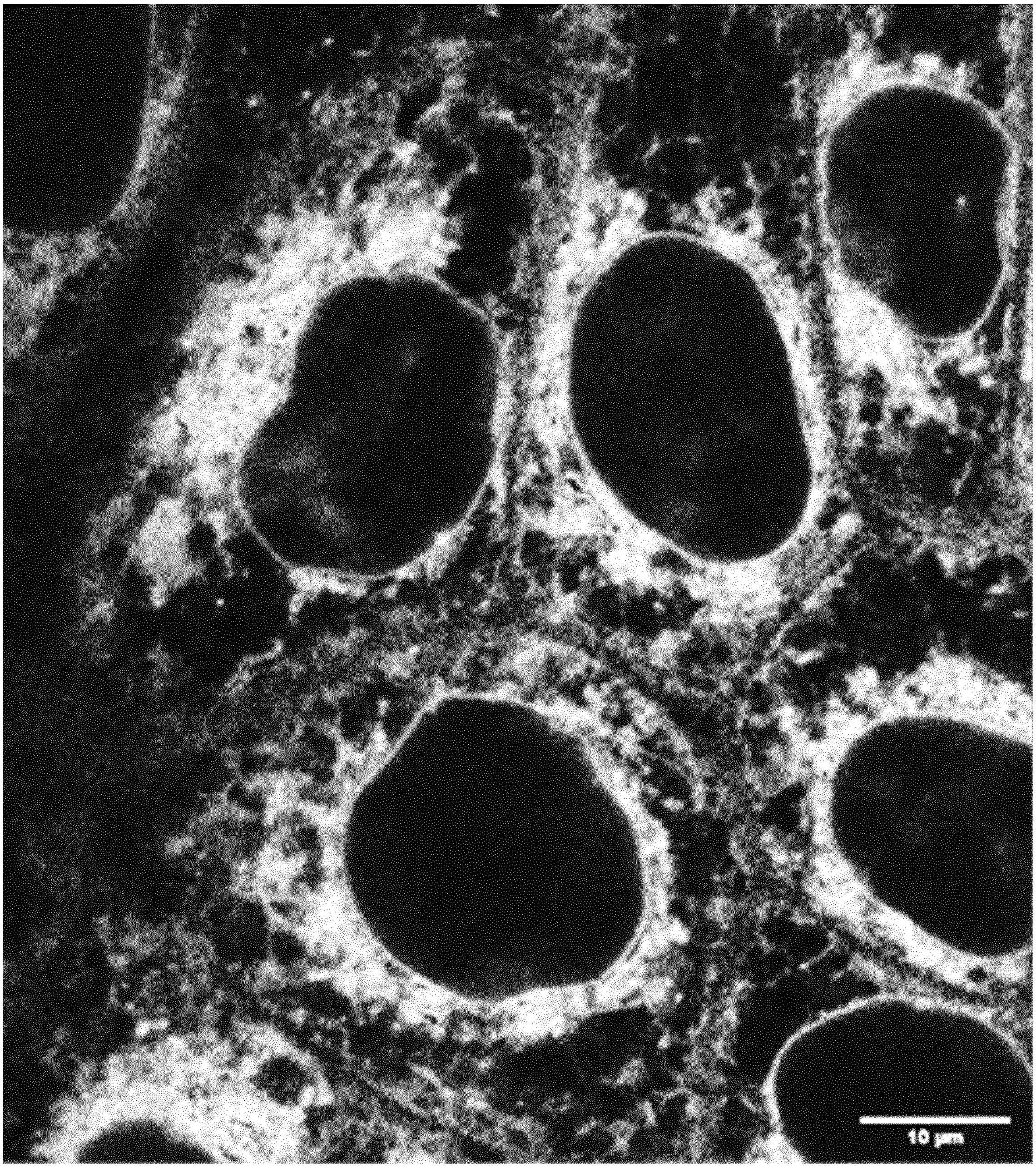


Figure 4

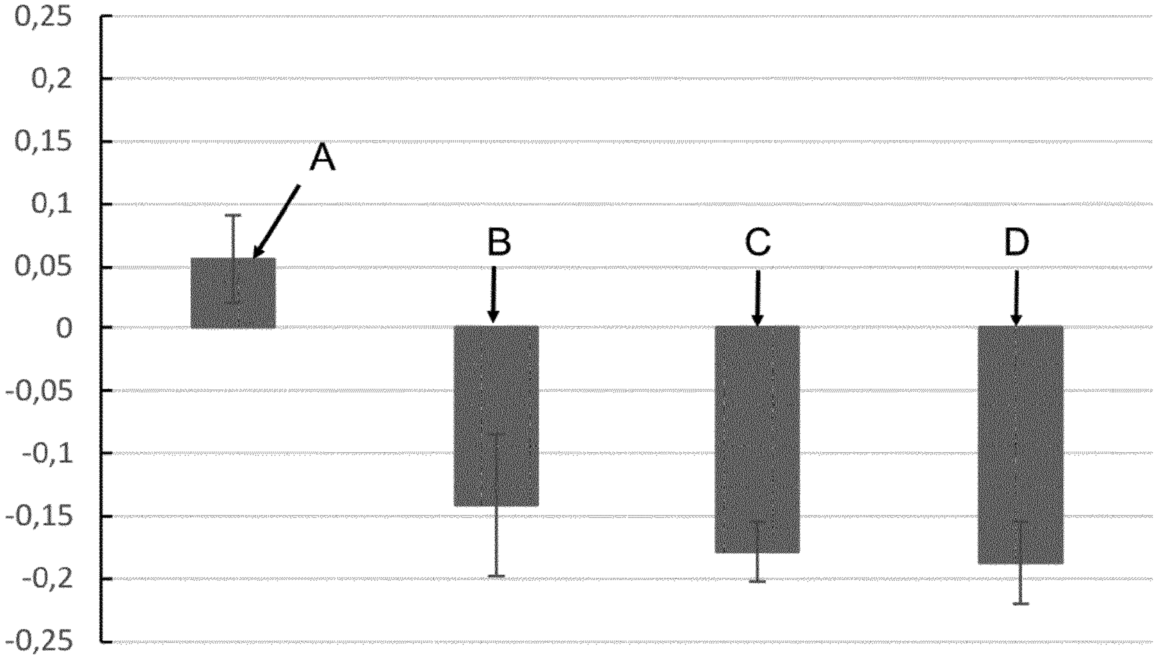


**Figure 5**

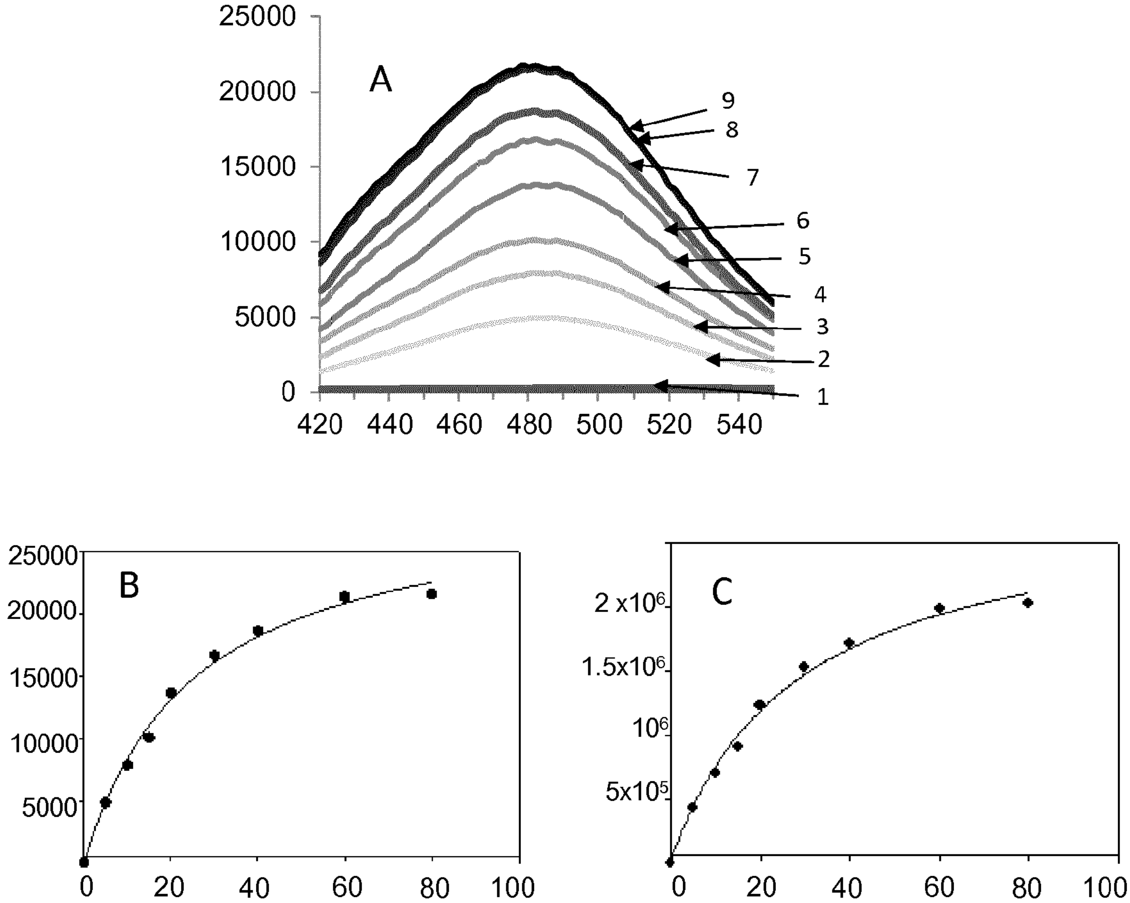


**Figure 6**

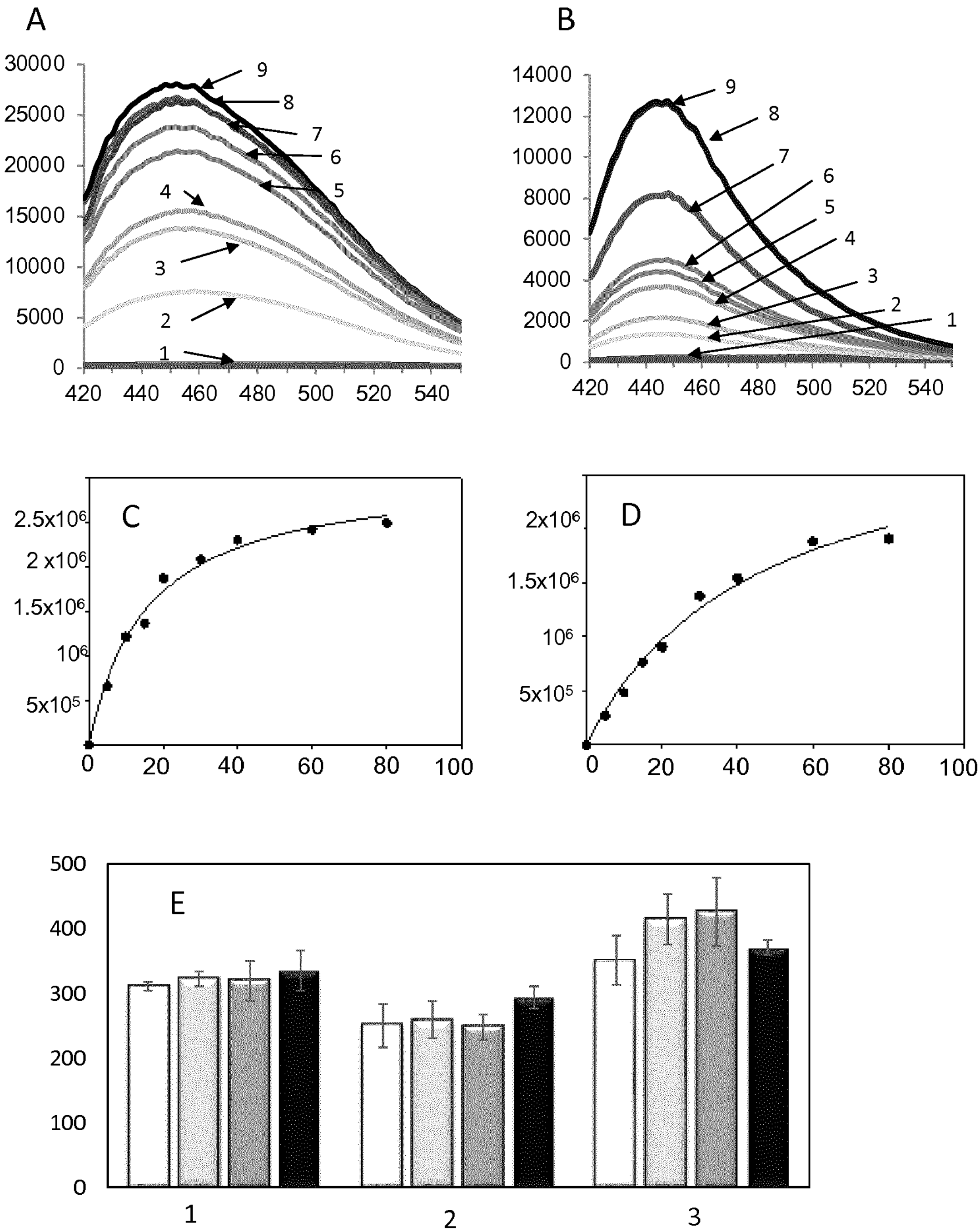
A
30000
25000
20000
15000
10000
5000
0
420 440 460 480 500 520 540
1
2
3
4
5
6
7
8
9
B
14000
12000
10000
8000
6000
4000
2000
0
420 440 460 480 500 520 540
1
2
3
4
5
6
7
8
9
C
2.5x10⁶
2 x10⁶
1.5x10⁶
10⁶
5x10⁵
0 20 40 60 80 100
D
2x10⁶
1.5x10⁶
10⁶
5x10⁵
0 20 40 60 80 100
E
500
400
300
200
100
0
1
2
3

Figure 7

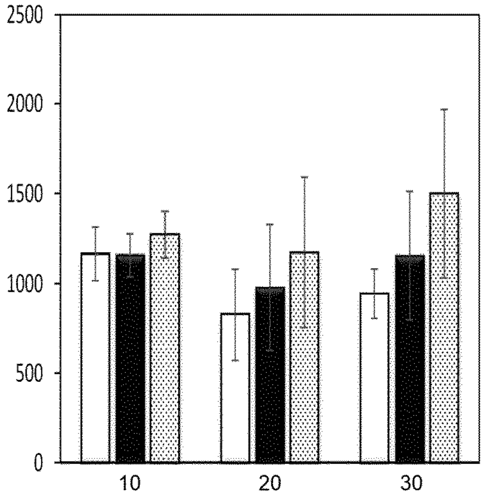


Figure 8

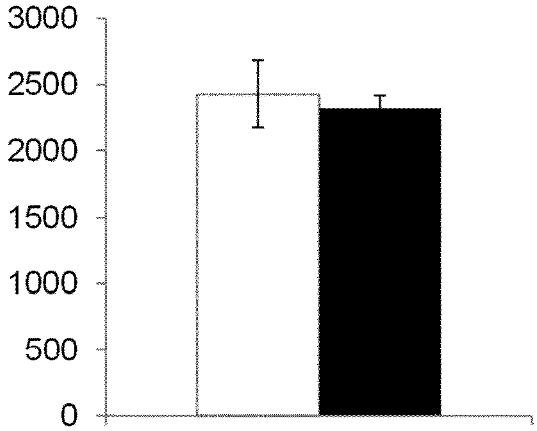


Figure 9

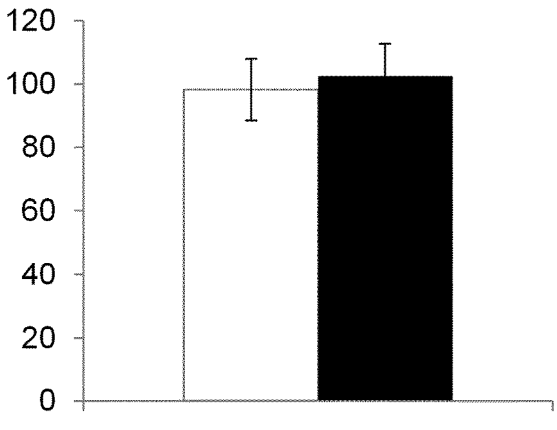


Figure 10

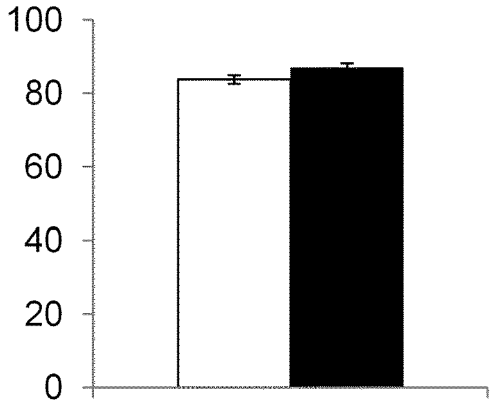


Figure 11

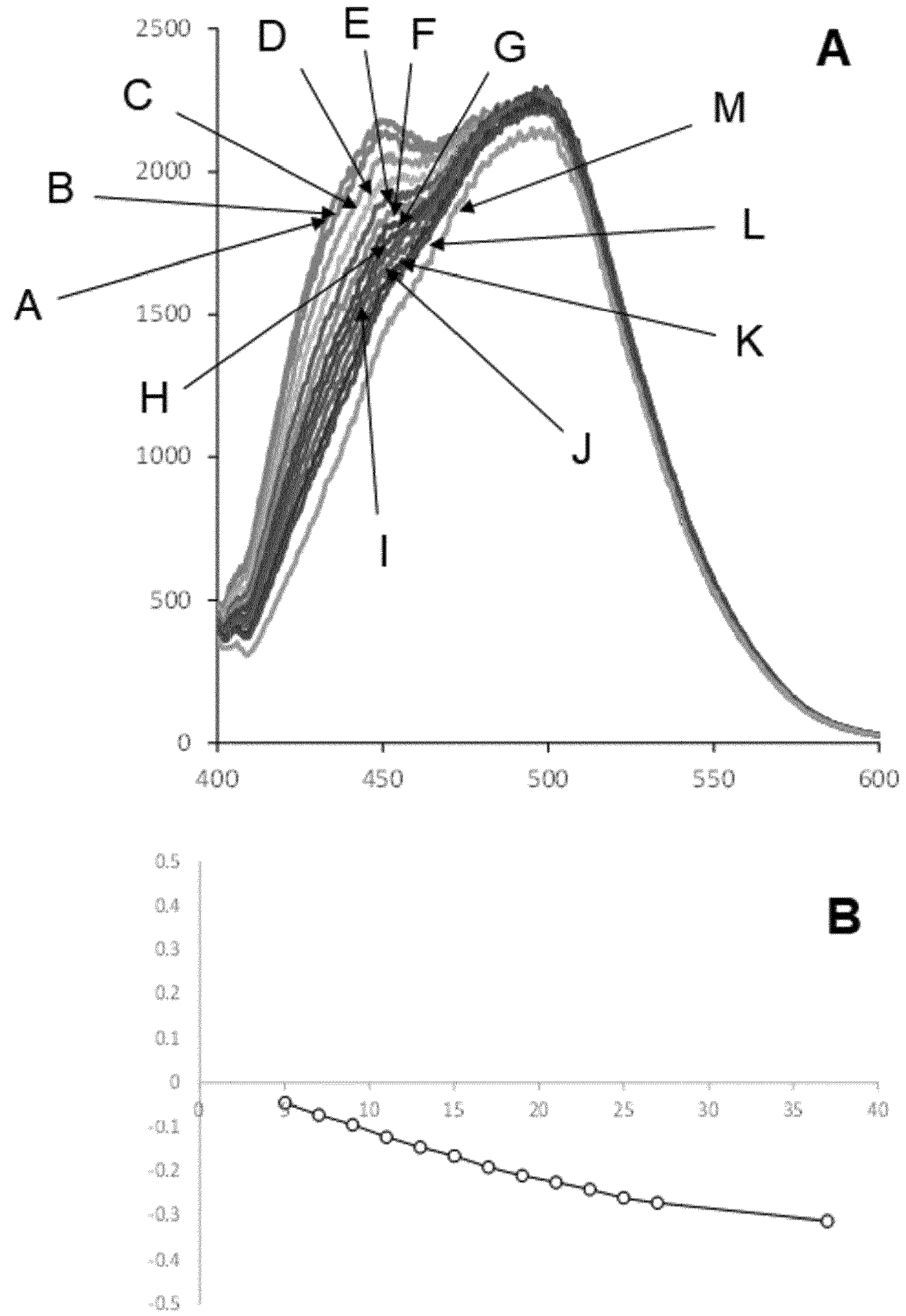
A
B
C
D
E
F
G
H
I
J
K
L
M
2500
2000
1500
1000
500
0
400
450
500
550
600
B
0.5
0.4
0.3
0.2
0.1
0
-0.1
-0.2
-0.3
-0.4
-0.5
0
5
10
15
20
25
30
35
40

Figure 12

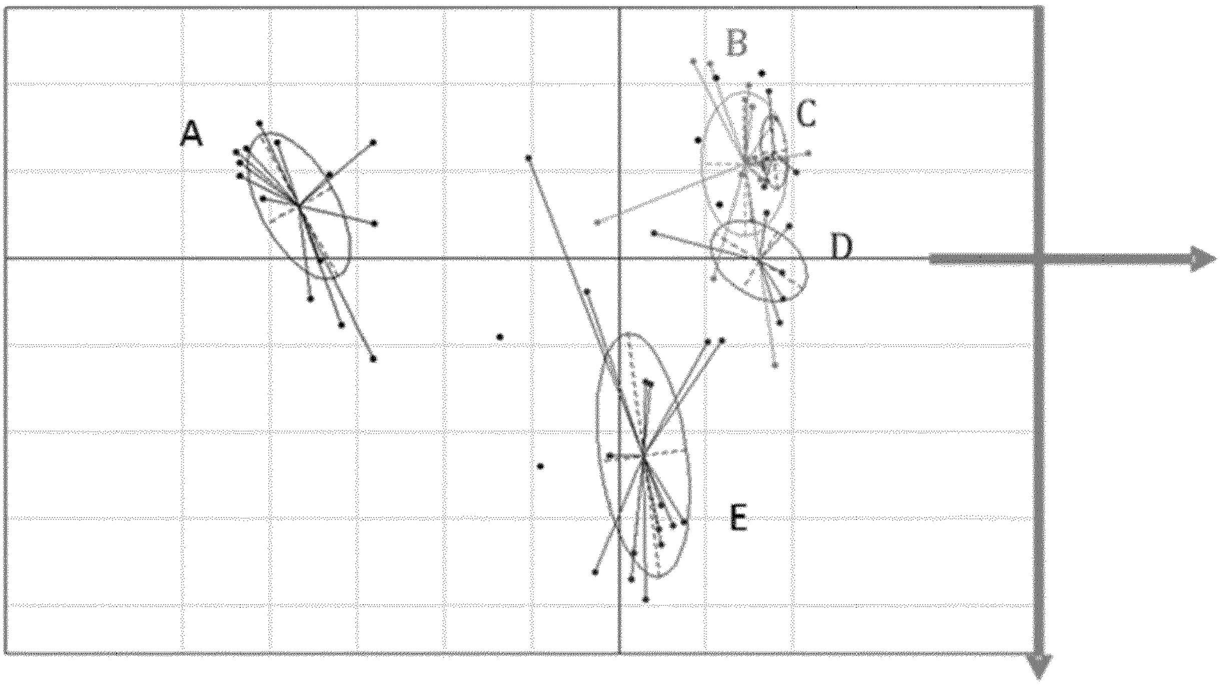


Figure 13

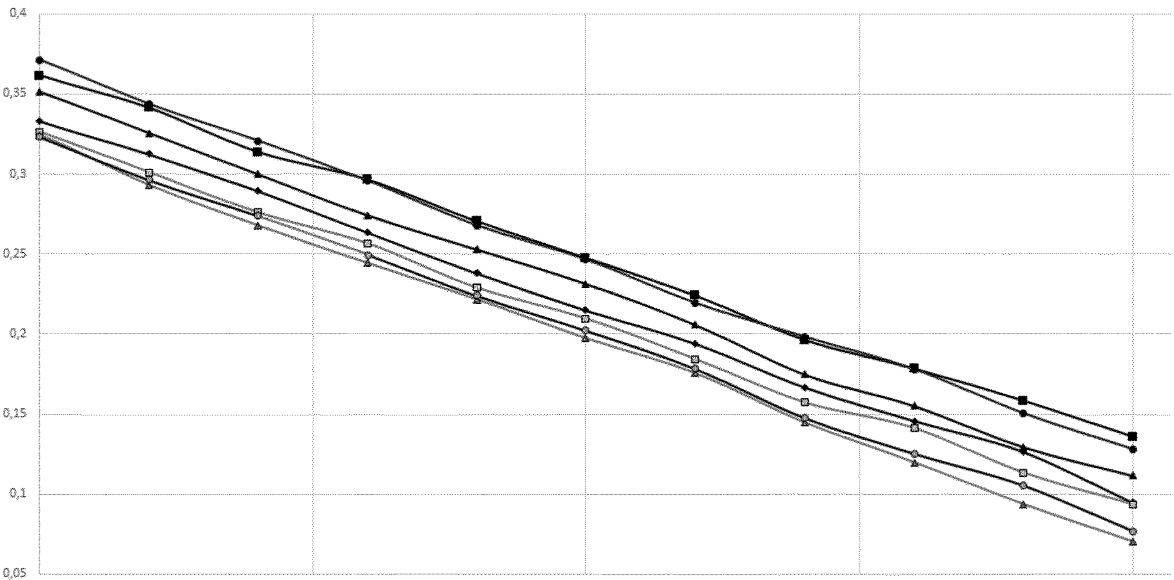


Figure 14

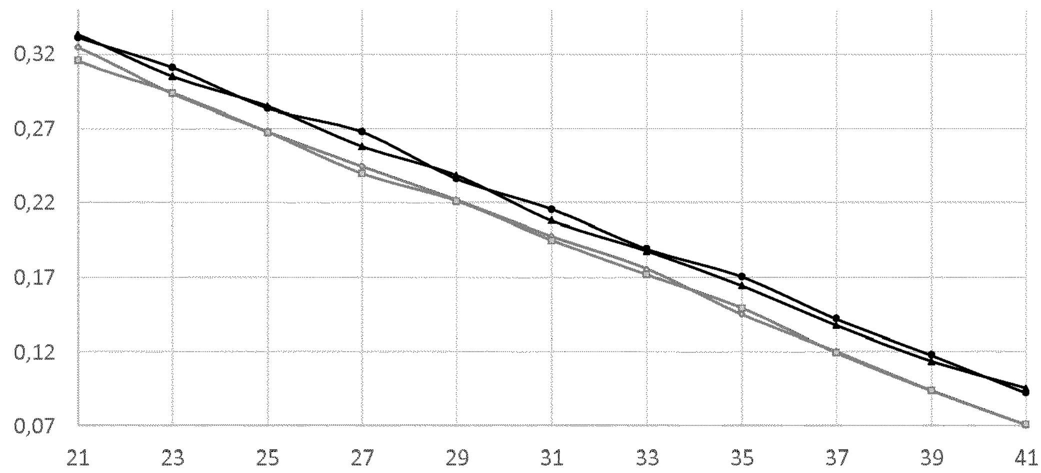


**Figure 15**

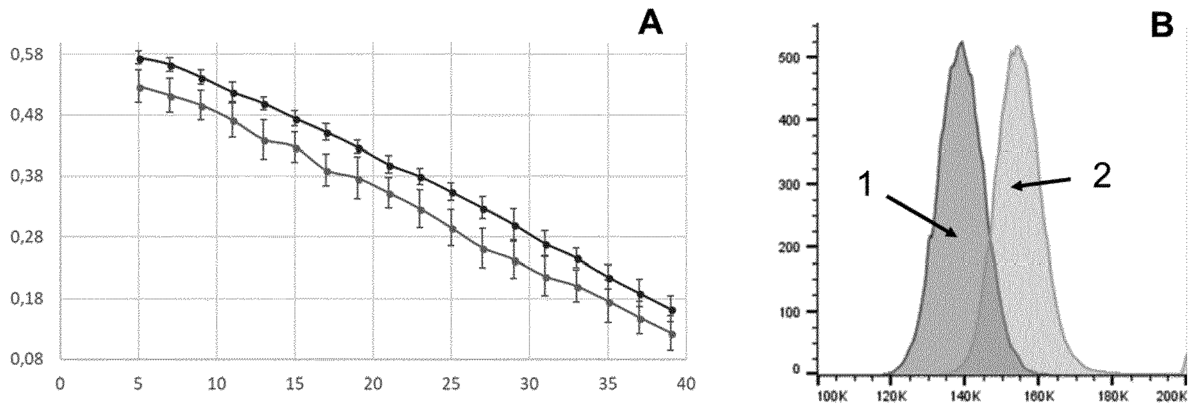


**Figure 16**

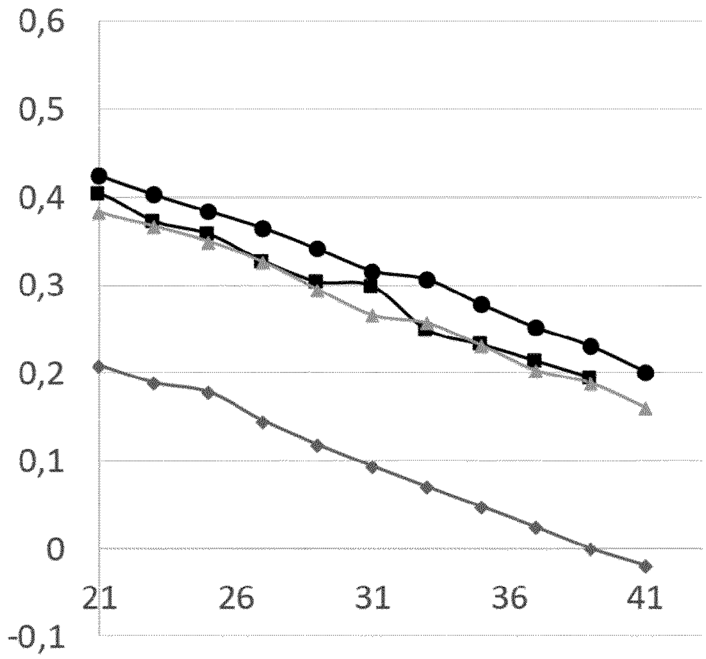


Figure 17

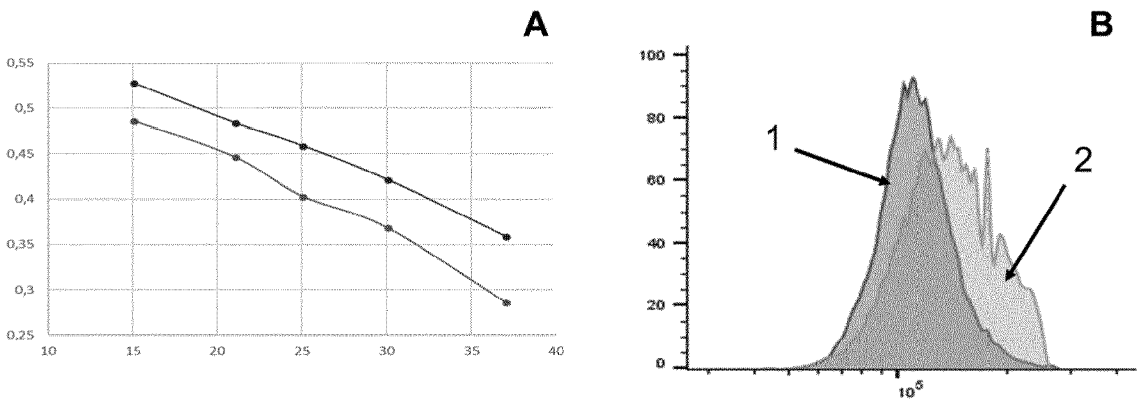


Figure 18

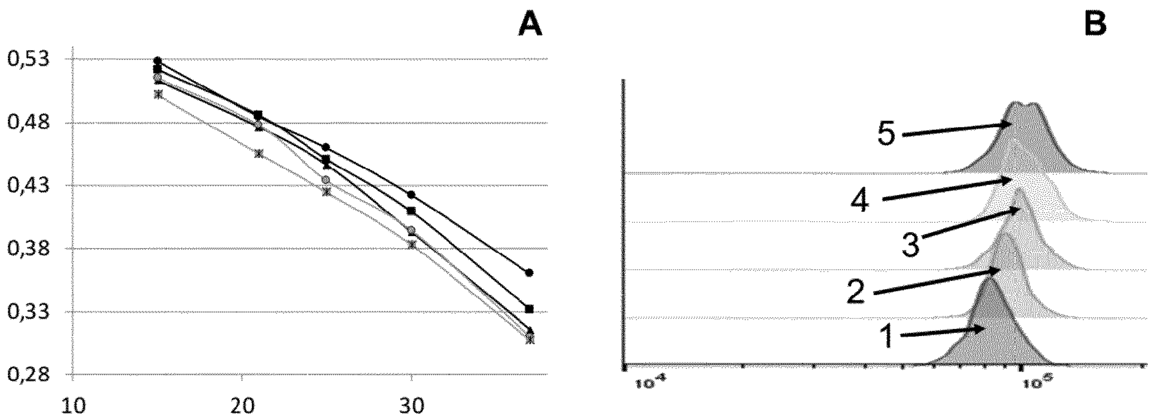


Figure 19

Europäisches Patentamt
European Patent Office
Office européen des brevets

# EUROPEAN SEARCH REPORT

Application Number
EP 19 30 6175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SERGE MAZERES ET AL: "Characterization of M-laurdan, a versatile probe to explore order in lipid membranes", F1000RESEARCH, 25 July 2014 (2014-07-25), XP055679055, DOI: 10.12688/f1000research.4805.1 | 1,2,4-15 | INV. C09B57/00 G01N33/58 A61K49/00 G01N21/64 |
| A | * page 5; table 1 * * page 4; figure 1 * ----- | 3 | |

| TECHNICAL FIELDS SEARCHED (IPC) |
|---|
| C09B G01N A61K |

The present search report has been drawn up for all claims

1

| Place of search | Date of completion of the search | Examiner |
|---|---|---|
| Munich | 24 March 2020 | Mezzato, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PARASASSI et al.** *Biophysical Society,* July 1991, vol. 60, 179-189
- **KIM et al.** *ChemBioChem,* 2007, vol. 8, 553-559
- **CHENIOUR et al.** *RSCAdv.,* 2016, vol. 6, 5547-5557
- **DINIC et al.** *Biochimica et Biophysica Acta,* 2011, vol. 1808, 298-306
- **SEZGIN et al.** *ChemPhysChem,* 2015, vol. 16, 1387-1394
- **AMARO et al.** *J. Phys. D: Appl. Phys.,* 2017, vol. 50, 134004
- **H. M. KIM ; H. J. CHOO ; S. Y. JUNG ; Y. G. KO ; W. H. PARK ; S. J. JEON ; C. H. KIM ; T. JOO ; B. R. CHO.** *ChemBioChem,* 2007, vol. 8, 553-559
- **PARASASSI et al.** *Biophysical Journal,* January 1994, vol. 66, 120-132
- **PARASASSI ; GRATTON.** *Journal of Fluorescence,* 1995, vol. 5 (1
- **STEWART.** *Analytical Biochemistry,* 1980, vol. 104, 10-14
- **BAUMGART, T. et al.** Large-scale fluid/fluid phase separation of proteins and lipids in giant plasma membrane vesicles. *Proc. Natl. Acad. Sci.,* 2007